(19) Europäisches Patentamt
European Patent Office
Office européen des brevets

(11) Publication number : 0 514 133 A1

# (12) EUROPEAN PATENT APPLICATION

(21) Application number : 92304264.2

(22) Date of filing : 12.05.92

(51) Int. Cl.[5] : **C07D 243/14**, C07D 403/12, C07D 405/12, C07D 417/12, A61K 31/55

(30) Priority : 14.05.91 GB 9110438
02.07.91 GB 9114288
25.10.91 GB 9122664
20.01.92 GB 9201104

(43) Date of publication of application :
19.11.92 Bulletin 92/47

(84) Designated Contracting States :
CH DE FR GB IT LI NL

(71) Applicant : MERCK SHARP & DOHME LTD.
Hertford Road
Hoddesdon Hertfordshire EN11 9BU (GB)

(72) Inventor : Chambers, Mark S.
10 Park Avenue Maisonettes, North Bushey
Watford, Hertfordshire (GB)
Inventor : Fletcher, Stephen R.
10 Clipped Hedge, Hatfield Heath
Nr. Bishops Stortford, Hertfordshire (GB)
Inventor : Matassa, Victor G.
The Duck Street Barns
Furneux Pelham, Hertfordshire (GB)

(74) Representative : Barrett-Major, Julie Diane et al
Merck & Co., Inc. European Patent
Department Terlings Park Eastwick Road
Harlow Essex CM20 2QR (GB)

## (54) Benzodiazepine derivatives, compositions containing them and their use in therapy.

(57) Compounds of formula (I), and salts and prodrugs thereof

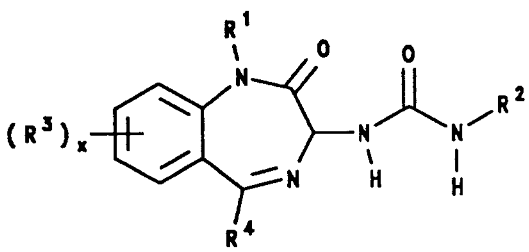

(I)

wherein :

$R^1$ represents optionally substituted $C_{1-6}$alkyl or $C_{3-7}$cycloalkyl ;
$R^2$ represents an optionally substituted phenyl or pyridyl group ;
$R^3$ represents $C_{1-6}$ alkyl or halo ;
$R^4$ represents $C_{3-7}$ cycloalkyl ;
X is 0, 1, 2 or 3 ;

are CCK and/or gastrin antagonists. They and compositions thereof are therefore useful in therapy.

EP 0 514 133 A1

This invention relates to benzodiazepine compounds which are useful as antagonists of cholecystokinin and gastrin.

Cholecystokinins (CCK) and gastrin are structurally related peptides which exist in gastrointestinal tissue and in the central nervous system (see, V. Mutt, Gastrointestinal Hormones, G.B.J. Green, Ed., Raven Press, N.Y., p.169 and G. Nission, ibid. p.127).

Cholecystokinins include CCK-33, a neuropeptide of thirty-three amino acids in its originally isolated form (see, Mutt and Jorpes, Biochem. J. 125, 678 (1971)), its carboxylterminal octapeptide, CCK-8 (also a naturally-occurring neuropeptide and the minimum fully active sequence), and 39- and 12-amino acid forms. Gastrin occurs in 34-, 17- and 14-amino acid forms, with the minimum active sequence being the C-terminal tetrapeptide, Trp-Met-Asp-Phe-$NH_2$, which is the common structural element shared by both CCK and gastrin.

CCKs are believed to be physiological satiety hormones, thereby possibly playing an important role in appetite regulation (G. P. Smith, Eating and Its Disorders, A. J. Stunkard and E. Stellar, Eds, Raven Press, New York, 1984, p. 67), as well as stimulating colonic motility, gall bladder contraction, pancreatic enzyme secretion and inhibiting gastric emptying. They reportedly co-exist with dopamine in certain mid-brain neurons and thus may also play a role in the functioning of dopaminergic systems in the brain, in addition to serving as neurotransmitters in their own right (see A. J. Prange et al., "Peptides in the Central Nervous System", Ann. Repts. Med. Chem 17, 31, 33 [1982] and references cited therein; J. A. Williams, Biomed Res. 3 107 [1982]; and J.E. Morley, Life Sci. 30, 479 [1982]).

The primary role of gastrin, on the other hand, appears to be stimulation of the secretion of water and electrolytes from the stomach and, as such, is involved in control of gastric acid and pepsin secretion. Other physiological effects of gastrin then include increased mucosal blood flow and increased antral motility. Rat studies have shown that gastrin has a positive trophic effect on the gastric mucosa, as evidenced by increased DNA, RNA and protein synthesis.

There are at least two subtypes of cholescystokinin receptors termed CCK-A and CCK-B (T.H. Moran et al., "Two brain cholecystokinin receptors: implications for behavoural actions", Brain Res.; 362, 175-79 [1986]). Both subtypes are found both in the periphery and in the central nervous system.

CCK and gastrin receptor antagonists have been disclosed for preventing and treating CCK-related and/or gastrin related disorders of the gastrointestinal (GI) and central nervous (CNS) systems of animals, especially mammals, and more especially those of humans. Just as there is some overlap in the biological activities of CCK and gastrin, antagonists also tend to have affinity for both CCK-B receptors and gastrin receptors. Other antagonists have activity at the CCK-A subtype.

Selective CCK antagonists are themselves useful in treating CCK-related disorders of appetite regulatory systems of animals as well as in potentiating and prolonging opiate-mediated analgesia [see P. L. Faris et al., Science 226, 1215 (1984)], thus having utility in the treatment of pain. CCK-B and CCK-A antagonists have also been shown to have a direct analgesic effect [M.F. O'Neill et al., Brain Research, 534 287 (1990)]. Selective CCK and gastrin antagonists are useful in the modulation of behaviour mediated by dopaminergic and serotonergic neuronal systems and thus have utility in the treatment of schizophrenia and depression (Rasmussen et. al., 1991, Eur. J. Pharmacol., 209, 135-138; Woodruff et. al., 1991, Neuropeptides, 19, 45-46; Cervo et. al., 1988, Eur. J. Pharmacol., 158, 53-59), as a palliative for gastrointestinal neoplasms, and in the treatment and prevention of gastrin-related disorders of the gastrointestinal system in humans and animals, such as peptic ulcers, Zollinger-Ellison syndrome, antral G cell hyperplasia and other conditions in which reduced gastrin activity is of therapeutic value, see e.g. U.S. Patent 4,820,834. Certain CCK antagonists are useful anxiolytic agents and can be used in the treatment of panic and anxiety disorders.

CCK has been reported to evoke the release of stress hormones such as adrenocorticotrophic hormone, β-endorphin, vasopressin and oxytocin, CCK may function as a mediator of responses to stress and as part of the arousal system. CCK-A receptors are now known to be present in a number of areas of the CNS and may be involved in modulating all of the above.

CCK may be involved in the regulation of stress and its relationship with drug abuse e.g. alleviation of the benzodiazepine withdrawal syndrome (Singh et. al., 1992, Br. J. Pharmacol., 105, 8-10) and neuroadaptive processes.

Since CCK and gastrin also have trophic effects on certain tumours [K. Okyama, Hokkaido J. Med. Sci., 206-216 (1985)], antagonists of CCK and gastrin are useful in treating these tumours [see, R.D. Beauchamp et al, Ann. Surg., 202, 203 (1985)].

In the light of discussion in C. Xu et al., Peptides, 8, 1987, 769-772, CCK antagonists may also be effective in neuroprotection.

CCK receptor antagonists have been found to inhibit the contractile effects of CCK on iris sphincter and ciliary muscles of monkey and human eyes (Eur. J. Pharmacol., 211(2), 183-187; A. Bill et al., Acta Physiol. Scand., 138, 479-485 [1990]), thus having utility in inducing miosis for therapeutic purposes.

A class of benzodiazepine antagonist compounds has been reported which binds selectively to brain CCK (CCK-B and CCK-A) and gastrin receptors [see M. Bock et al.,J. Med Chem., 32, 13-16 (1989)].

European patent application no. 0 167 919 discloses benzodiazepine CCK and gastrin antagonists substituted in the 3-position by, inter alia, a phenyl urea; however, 5-cycloalkyl substitution is not disclosed.

The present invention provides benzodiazepine compounds of formula (I):

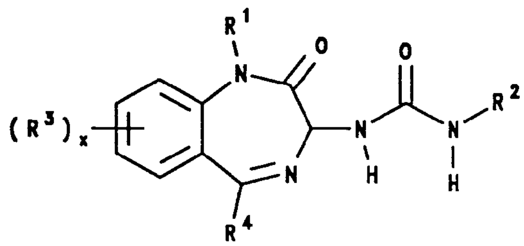

(I)

wherein:

$R^1$ represents $(CH_2)_q$imidazolyl, $(CH_2)_q$tetrazolyl, $(CH_2)_q$triazolyl; $C_{1-6}$alkyl optionally substituted by one or more groups selected from halo, hydroxy and $NR^6R^7$; $C_{3-7}$cycloalkyl; cyclopropylmethyl; $CH_2CO_2R^5$; $CH_2CONR^6R^7$; or $CH_2CH(OH)$-W-$(CH_2)_2NR^6R^7$ where $R^5$ represents $C_{1-4}$alkyl, $R^6$ and $R^7$ each independently represents a hydrogen atom or a $C_{1-4}$alkyl group, or taken together with the nitrogen atom to which they are attached form a pyrrolidinyl, or piperidinyl ring, q is 1, 2 or 3, and W is S or NH;

$R^2$ represents:

(i) a phenyl group optionally substituted by one or more substituents selected from $C_{1-6}$alkyl optionally substituted by hydroxy, $CONR^aR^b$, $CO_2R^a$ or $OCONR^aR^b$; $C_{2-6}$alkenyl optionally substituted by $CO_2R^a$; $C_{2-6}$alkynyl; halo; optionally protected hydroxy; $NHR^8$; $NHPO(OC_{1-4}alkyl)$; $(CH_2)_n$tetrazolyl optionally substituted in the tetrazolyl ring by $C_{1-4}$alkyl; $(CH_2)_n$imidazolyl; CONH-tetrazolyl; CONH-triazolyl; diazolinone; triazolinone optionally substituted by methyl; tetrazolinone; oxathiadiazolone; 5-hydroxy-4-pyrone; $CONH_2$; $CONHCOR^9$; $SO(C_{1-6}alkyl)$; $SO_2(C_{1-6}alkyl)$; $CONHCO_2R^9$; $CONHCONHR^9$; $C(NH_2)NOH$; $COC_{1-4}alkyl$; $CONHSO_2R^9$; $SO_2NH_2$; $NHSO_2NH_2$; $SO_2NHCO_2R^9$; $SO_2NHCONHR^9$; $SO_2NHSO_2R^9$; $SO_2NHPO(OR^aR^b)$; $SO_2NHR^{10}$; cyano; B(OH)2; $CO_2H$; $CH_2OCH_2O(CH_2)_2OCH_3$, where $R^a$ and $R^b$ each independently represent H or $C_{1-6}$alkyl, n is 0, 1 or 2, $R^8$ represents H or $COC_{1-6}alkyl$, $R^9$ represents $C_{1-6}$alkyl, optionally substituted aryl, 2,2-difluorocyclopropane or trifluoromethyl, and $R^{10}$ represents a nitrogen containing heterocycle;

(ii) a group

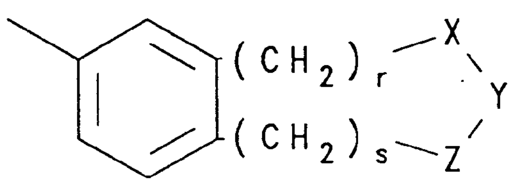

wherein X and Z each independently represent $CH_2$, O, $SO_2$, C=O, NH or N; and

Y represents $CH_2$, C=O, $NR^a$ or N, where $R^a$ is as above defined;

r and s each independently represent 0 or 1;

and the dotted line represents an optional double bond;

provided that: X and Z can only be the same when they are O, and when one of X and Z is O, the other of X and Z must be O;

when X or Z is N, Y is also N and the ring contains one unit of unsaturation;

when X or Z is $SO_2$, Y is NH and the other of X and Z is C=O;

when X or Z is $SO_2$, Y is NH and the other of X and Z is C=O, whichever of r and s is adjacent to $SO_2$ may be 1; otherwise r and s are the same and O;

(iii) a pyridyl group substituted by $C_{1-4}$alkoxy or halo;

$R^3$ represents $C_{1-6}$ alkyl or halo;

$R^4$ represents $C_{3-7}$ cycloalkyl;

x is 0, 1, 2 or 3;

and pharmaceutically acceptable salts and prodrugs thereof.

It will be appreciated that formula (I) is intended to embrace all possible isomers, including optical isomers, and mixtures thereof, including racemates.

The present invention includes within its scope prodrugs of the compounds of formula I above. In general, such prodrugs will be functional derivatives of the compounds of formula I which are readily convertible in vivo into the required compound of formula I. Conventional procedures for the selection and preparation of suitable prodrug derivatives are described, for example, in "Design of Prodrugs", ed. H. Bungaard, Elsevier, 1985.

As used herein, alkyl means linear or branched chain alkyl. Examples of suitable alkyl groups include methyl, ethyl, isopropyl and isobutyl groups.

When $R^1$ represents cycloalkyl, examples include cyclopropyl, cyclopentyl and cyclohexyl groups, preferably, cyclopropyl.

Halo includes fluoro, chloro and bromo. Preferably halo is fluoro or chloro.

Preferably $R^2$ represents a mono- or disubstituted phenyl group.

When $R^2$ represents a monosubstituted phenyl group, the substituent is preferably in the 3- or 4-position of the phenyl ring, more preferably the 3-position.

When $R^2$ represents a disubstituted phenyl group the substituents are preferably in the 3- and 4-positions of the phenyl ring.

When $R^2$ represents the group,

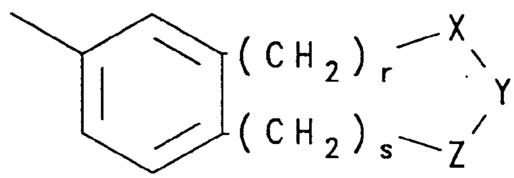

suitable values of $R^2$ include

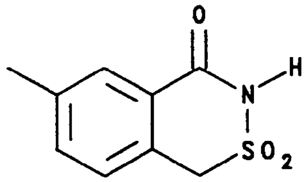

(1)

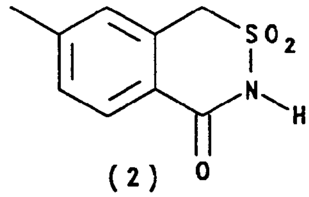

(2)

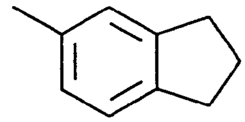

(3)

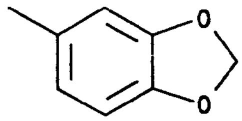

(4)

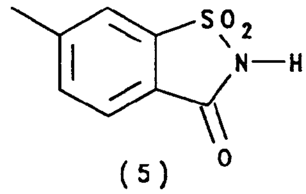

(5)

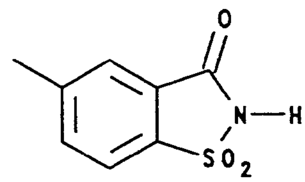

(6)

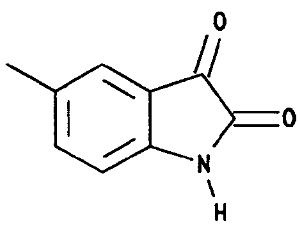

(7)

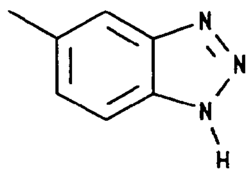

(8)

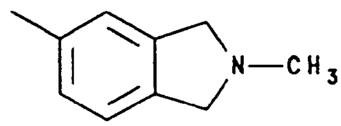

(9) .

Preferred are values for $R^2$ corresponding to the structures numbered (1), (2) and (3) above, especially those numbered (1) and (2).

Suitably $R^4$ represents cyclohexyl, cyclopentyl or cyclobutyl, preferably cyclohexyl or cyclopentyl.

A particular sub-group of compounds according to the invention is represented by compounds of formula (Ia), and salts and prodrugs thereof:

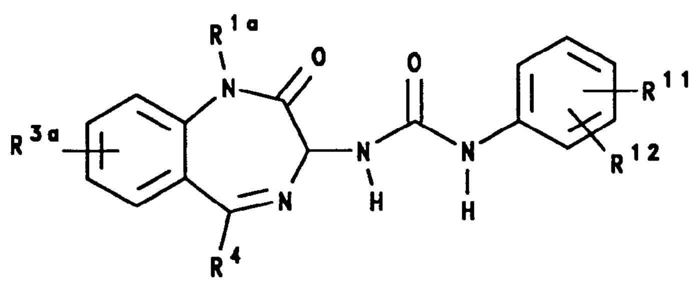

(Ia)

wherein:

$R^{1a}$ represents $C_{1-6}$ alkyl;

$R^{3a}$ represents hydrogen;

$R^4$ represents $C_{3-7}$ cycloalkyl;

$R^{11}$ represents hydrogen or $C_{1-6}$ alkyl;

$R^{12}$ represents $C_{1-6}$ alkyl, halo, $(CH_2)_n$-tetrazolyl, optionally substituted by $C_{1-4}$alkyl, $(CH_2)_n$-imidazolyl, 5-hydroxy-4-pyrone, $SO(C_{1-6}alkyl)$ $CONHSO_2R^{9a}$, $SO_2NHCOR^{9a}$ (where $R^{9a}$ is $C_{1-6}$ alkyl, optionally substituted aryl or trifluoromethyl), $SO_2NHR^{10a}$ (where $R^{10a}$ is a nitrogen containing heterocycle) or a group

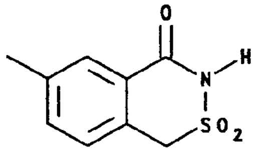

or

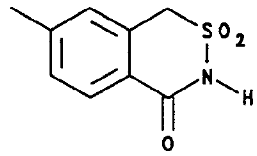

When $R^{9a}$ is optionally substituted aryl, this will preferably be optionally substituted phenyl. Suitable substituents include $C_{1-4}$alkyl, $C_{1-4}$alkoxy, halo and trifluoromethyl.

When $R^{9a}$ is $C_{1-6}$alkyl, it will preferably represent $C_{1-4}$alkyl. Particularly preferred are methyl and isopropyl, especially iso-propyl.

Suitable values for $R^{9a}$ include methyl, ethyl, i-propyl, t-butyl, optionally substituted phenyl and trifluoromethyl. Where $R^{9a}$ is substituted phenyl, preferably the phenyl substituent is $C_{1-4}$alkyl, more preferably methyl.

Preferably $R^{11}$ is H or methyl.

In one preferred group of compounds of formula Ia, $R^{11}$ is H and $R^{12}$ is 3-tetrazol-5-yl.

In a further preferred group of compounds of formula Ia, $R^{12}$ is $CONHSO_2R^{9a}$ or $SO_2NHCOR^{9a}$, more preferably $CONHSO_2R^{9a}$.

When $R^{12}$ is $SO_2NHR^{10a}$, suitable values of $R^{10}$ include, for example, thiazole, thiadiazole and pyrazine.

Preferably m is 1.

Preferably n is zero.

Preferably $R^4$ is cyclobutyl, cyclopentyl or cyclohexyl.

A further subclass of compounds according to the invention is represented by formula (Ib)

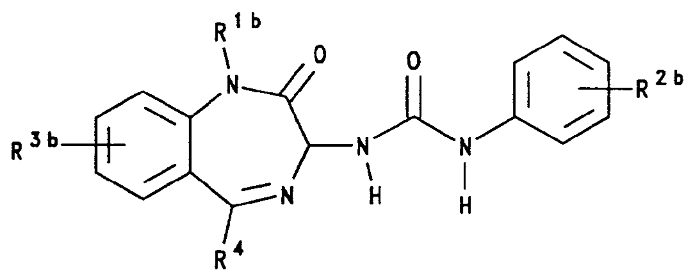

(Ib)

and salts and prodrugs thereof, wherein

$R^{1b}$ represents $C_{1-6}$ alkyl, $C_{3-7}$ cycloalkyl, cyclopropylmethyl, $(CH_2)_m$-imidazolyl (where m is 1 or 2), $CH_2CO_2R^6$ (where $R^6$ is $C_{1-4}$ alkyl) or a group $CH_2CONR^7R^8$ (where $R^7$ and $R^8$ each independently represents a hydrogen atom or a $C_{1-4}$ alkyl group, or $R^7$ and $R^8$ together form a chain $(CH_2)_p$ where p is 4 or 5);

$R^{2b}$ represents hydrogen, $C_{1-6}$ alkyl, halo, $(CH_2)_n$-tetrazolyl optionally substituted by $C_{1-4}$alkyl, $(CH_2)_n$-imidazolyl, $CONHSO_2R^{9b}$, $SO_2NHCOR^{9b}$ (where $R^{9b}$ is $C_{1-6}$ alkyl, optionally substituted aryl or trifluoromethyl), $SO_2NHR^{10b}$ (where $R^{10b}$ is a nitrogen containing heterocycle) or a group $(CH_2)_nCO_2H$, n is zero, 1 or 2;

$R^{3b}$ represents hydrogen, $C_{1-6}$ alkyl or halo;

$R^4$ represents $C_{3-7}$ cycloalkyl.

One subgroup of compounds according to formula (Ib) are those wherein $R^{2b}$ represents hydrogen, $C_{1-6}$ alkyl, halo, $(CH_2)_n$-tetrazolyl, $(CH_2)_n$-imidazolyl, $CONHSO_2R^{9b}$, $SO_2NHCOR^{9b}$ (where $R^{9b}$ is $C_{1-4}$ alkyl, optionally substituted aryl or trifluoromethyl) or a group $(CH_2)_nCO_2H$, n is zero, 1 or 2;

A further subgroup of compounds according to formula (Ib) are those wherein $R^{2b}$ represents $C_{1-6}$ alkyl, halo, $(CH_2)_n$-tetrazolyl, $(CH_2)_n$-imidazolyl or a group $(CH_2)_nCO_2H$, n is zero, 1 or 2.

A yet further subgroup of compounds according to formula (Ib) and the abovementioned subgroups thereof are compounds wherein $R^{1b}$ is $C_{1-6}$alkyl; $R^{3b}$ is hydrogen and $R^4$ is $C_{3-7}$cycloalkyl.

Preferably the salts of the compounds of formula (I) are pharmaceutically acceptable, but non-pharmaceutically acceptable salts may be used for the preparation of pharmaceutically acceptable salts. The pharmaceutically acceptable salts of the compounds of formula (I) include the conventional non-toxic salts or the quaternary ammonium salts of the compounds from formula (I) formed, e.g., from inorganic or organic acids or bases. For example, such conventional non-toxic salts include those derived from inorganic acids such as hydrochloric, hydrobromic, sulphuric, sulphamic, phosphoric, nitric and the like; and the salts prepared from organic acids such as acetic, propionic, succinic, glycolic, steric, lactic, malic, tartaric, citric, ascorbic, palmoic, maleic, hydroxymaleic, phenylacetic, glutamic, benzoic, salicylic, sulphanilic, 2-acetoxy benzoic, fumaric, toluenesulphonic, methanesulphonic, ethane disulphonic, oxalic and isothionic.

The pharmaceutically acceptable salts of the present invention can be synthesized from the compound of formula (I) which contain a basic or acidic moiety by conventional chemical methods. Generally, the salts are prepared by reacting the free base or acid with stoichiometric amounts or with an excess of the desired salt-forming inorganic or organic acid or base in a suitable solvent or various combinations of solvents.

For example, an acid of formula (I) may be reacted with an appropriate amount of a base, such as an alkali or alkaline earth metal hydroxide e.g. sodium, potassium, lithium, calcium, or magnesium, or an organic base such as an amine, e.g. dibenzylethylenediamine, trimethylamine, piperidine, pyrrolidine, benzylamine, and the like, or a quaternary ammonium hydroxide such as tetramethylammonium hydroxide.

The compounds of formula (I) may be prepared by processes analogous to those described in European Patent Specification No. 0284256. For example, according to one general process (A), a compound of formula (I) may be prepared from an intermediate of formula (II)

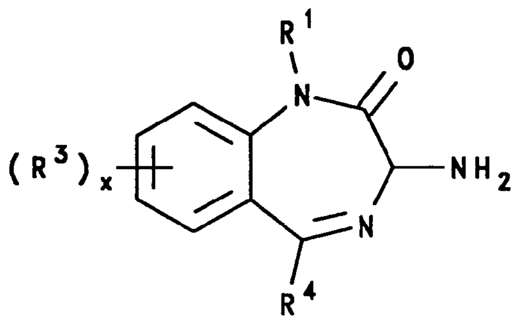

(II)

wherein $R^1$, $R^3$, $R^4$ and x are as defined for formula (I); by reaction with an isocyanate of formula $R^2$-N=C=O wherein $R^2$ is as defined for formula (I).

The reaction is preferably conducted in a suitable organic solvent, such as an ether, for example, tetrahydrofuran, at room temperature.

The isocyanate may be generated in situ from the corresponding amine by treatment with triphosgene.

According to a further general process, (B), compounds of formula (I) may be prepared by reacting a compound of formula (IV)

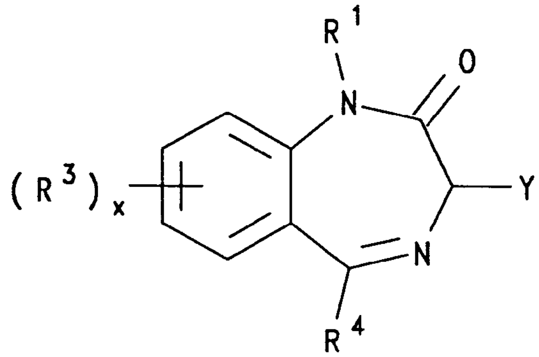

(IV)

wherein $R^1$, $R^3$, $R^4$ and x are as defined for formula (I) and Y represents an activated carbamate, with an amine of formula $R^2NH_2$ in the presence of a base. An "activated carbamate "is a carbamate group which bears a substituent which activates the carbamate function to nucleophilic attack. Suitably Y may represent an appropriately substituted aryl carbamate of formula

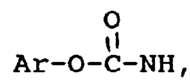

for example

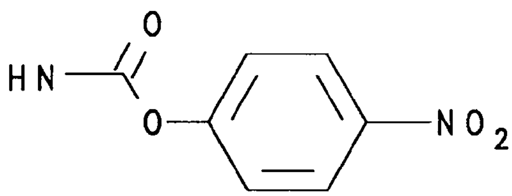

Suitable bases for use in the reaction include tertiary amines, for example, triethylamine.

The reaction is conveniently effected in a suitable organic solvent, for example, dimethylformamide, at ambient or elevated temperature. Preferably the reaction is conducted at approximately 50°C.

Intermediates of formula (II) may be prepared from compounds of formula (VI)

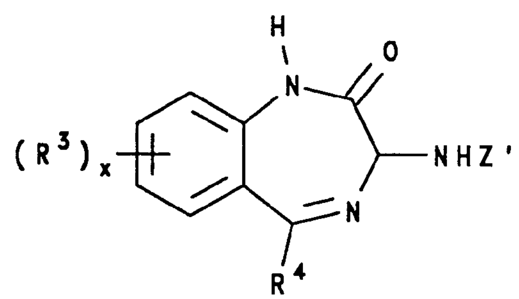

(VI)

wherein $R^3$, $R^4$ and x are as defined for formula (I) and Z′ is a protecting group; by reaction with a reagent suitable to introduce the group $R^1$, for example a halide of formula $R^1Hal$ where Hal represents halo such as bromo or iodo, in the presence of a base, such as an alkali metal hydride or an alkaline earth metal carbonate, for example sodium hydride or caesium carbonate; or a suitable dialkyl acetal of dimethyl formamide in a suitable organic solvent, e.g. toluene, followed by deprotection.

Compounds of formula (VI) may be prepared from compounds of formula (VII)

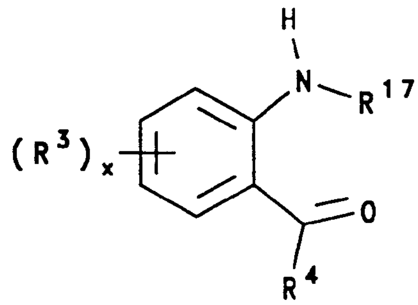

(VII)

wherein $R^3$, $R^4$ and x are as defined for formula (I) and $R^{17}$ is hydrogen, by a reaction sequence comprising:

(i) reaction with a compound of formula (VIII)

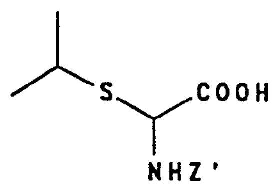

(VIII)

wherein Z′ is as defined above, in the presence of a base, such as a tertiary amine, for example triethylamine or N-methyl morpholine, and a coupling reagent. Any of the coupling reagents commonly used in peptide synthesis are suitable, for example, 1,3-dicyclohexylcarbodiimide (DCC) or isobutyl chloroformate.

(ii) Treatment with gaseous ammonia, preferably in the presence of a mercury containing catalyst, such as mercury(II) chloride. The reaction is conveniently effected in a suitable organic solvent, such as an ether, for example, tetrahydrofuran.

(iii) Treatment with an organic acid, for example acetic or propionic acid, optionally in the presence of an ammonium salt, for example ammonium acetate.

Compounds of formula (VII) wherein $R^{17}$ is hydrogen may be prepared from corresponding compounds of formula (VII) wherein $R^{17}$ is $COCH_3$ by treatment with a mineral acid, for example hydrochloric acid, or by base hydrolysis, for example, using aqueous sodium hydroxide. The reaction is conveniently affected in refluxing methanol.

Alternatively, compounds of formula (VII) wherein $R^{17}$ is hydrogen may be prepared by reaction of a compound of formula (IX)

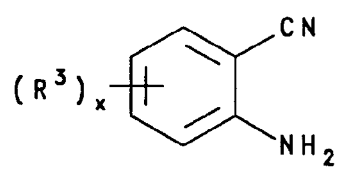

(IX)

wherein $R^3$ and x are as previously defined, with a Grignard reagent of formula $R^4MgHal$ wherein $R^4$ is as previously defined and Hal is halo such as chloro, bromo or iodo.

Compounds of formula (IX) are commercially available or may be prepared from commercially available compounds by conventional methods.

Compounds of formula (VII) wherein $R^{17}$ is $COCH_3$ may be prepared from compounds of formula (X)

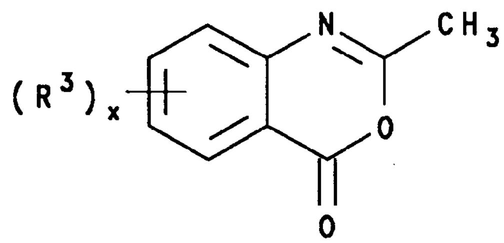

(X)

wherein $R^3$ and x are defined as for formula (I), by reaction with a Grignard reagent of formula $R^4MgHal$ as previously defined.

Compounds of formula (X) may be prepared by known methods, e.g. see D. A. Walsh, Synthesis, 677, (1980).

Intermediates of formula (IV) may be prepared from compounds of formula (II) by reaction with a suitable haloformate of formula

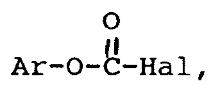

where Hal is as previously defined, preferably chloro, for example

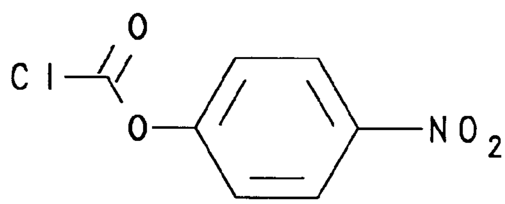

in the presence of a base, such as a tertiary amine, for example, triethylamine.

Amines of formula $R^2NH_2$ are known compounds, or may be prepared from the corresponding nitro compounds of formula $R^2NO_2$ wherein $R^2$ is as defined for formula (I), by reduction.

Suitably the reduction is effected by catalytic hydrogenation, for example, using a noble metal catalyst such as palladium which may be supported, e.g. on carbon. The reaction is conveniently effected in a suitable organic solvent, such as an alcohol, e.g. ethanol.

Compounds of formula $R^2NO_2$ are commercially available or may be prepared by the procedures described in the accompanying Examples or by alternative procedures which will be readily apparent to one skilled in the art.

Intermediates of formula (II), (IV) and (VI) are novel compounds and form a further aspect of the present invention.

Thus, in a further or alternative aspect, the present invention provides an intermediate of formula (XX)

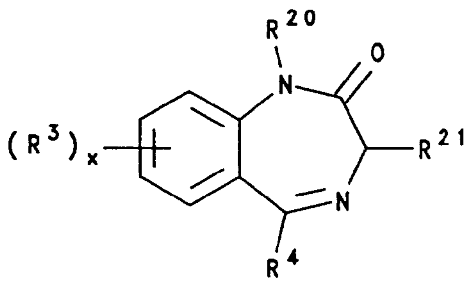

(XX)

wherein $R^3$, $R^4$ and x are as defined for formula (I); $R^{20}$ is hydrogen or $R^1$ as defined for formula (I); and $R^{21}$ is $NH_2$, NHZ′ (where Z′ is a protecting group), or an activated carbamate.

Where the above-described process for the preparation of the compounds according to the invention gives rise to mixtures of stereoisomers these isomers may, if desired, be separated, suitably by conventional techniques such as preparative chromatography.

The novel compounds may be prepared in racemic form, or individual enantiomers may be prepared either by enantiospecific synthesis or by resolution. The novel compounds may, for example, be resolved into their component enantiomers by standard techniques, such as the formation of diastereomeric pairs by salt formation with an optically active acid, such as (-)-di-p-toluoyl-L-tartaric acid and/or (+)-di-p-toluoyl-D-tartaric acid followed by fractional crystallization and regeneration of the free base. The novel compounds may also be resolved by formation of diastereomeric esters or amides, followed by chromatographic separation and removal of the chiral auxiliary. Alternatively, enantiomers of the novel compounds may be separated by HPLC using a chiral column.

Enantiospecific synthesis of compounds of formula (I) may be achieved, for example, by reaction of chiral intermediates of formula (II), which chiral intermediates may be prepared from the corresponding racemate by conventional procedures, for example, as described in J. Org. Chem., 52, 955 and 3232, (1987), with compounds of formula (III).

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene and P.G.M. Wutts, Protective Groups in Organic Synthesis, John Wiley & Sons, 1991. The protecting groups may be removed at a convenient subsequent stage using methods known from the art.

The compounds of formula (I) antagonise CCK and/or gastrin and are useful for the treatment and prevention of disorders including central nervous system disorders wherein CCK and/or gastrin may be involved. Examples of such disease states include gastrointestinal diseases, including gastrointestinal ulcers, such as peptic and duodenal ulcers, irritable bowel syndrome, gastroesophagenal reflux disease or excess pancreatic or gastrin secretion, acute pancreatitis, or motility disorders; central nervous system disorders, including central nervous system disorders caused by CCK interaction with dopamine, serotonin and other monoamine neurotransmitters, such as neuroleptic disorders, tardive dyskinesia, Parkinson's disease, psychosis or Gilles de la Tourette syndrome; depression; schizophrenia; disorders of appetite regulatory systems; Zollinger-Ellison syndrome, antral and cell hyperplasia, or pain.

The compounds of formula (I) are particularly useful in the treatment or prevention of neurological disorders involving anxiety disorders and panic disorders, wherein CCK and/or gastrin is involved. Examples of such disorders include panic disorders, anxiety disorders, panic syndrome, anticipatory anxiety, phobic anxiety, panic anxiety, chronic anxiety and endogenous anxiety.

The compounds of formula (I) are also useful for directly inducing analgesia, opiate or non-opiate mediated, as well as anesthesia or loss of the sensation of pain.

The compounds of formula (I) may further be useful for preventing or treating the withdrawal response produced by chronic treatment or abuse of drugs or alcohol. Such drugs include, but are not limited to benzodiazepines, cocaine, alcohol and nicotine.

The compounds of formula (I) may further by useful in the treatment of stress and its relationship with drug abuse.

The compounds of formula (I) may further be useful in the treatment of oncologic disorders wherein CCK may be involved. Examples of such oncologic disorders include small cell adenocarcinomas and primary tumours of the central nervous system glial and neuronal cells. Examples of such adenocarcinomas and tumours

include, but are not limited to, tumours of the lower oesophagus, stomach, intestine, colon and lung, including small cell lung carcinoma.

The compounds of formula (I) may also be useful as neuroprotective agents, for example, in the treatment and/or prevention of neurodegenerative disorders arising as a consequence of such pathological conditions as stroke, hypoglycaemia, cerebral palsy, transient cerebral ischaemic attack, cerebral ischaemia during cardiac pulmonary surgery or cardiac arrest, perinatal asphyxia, epilepsy, Huntington's chorea, Alzheimer's disease, Amyotrophic Lateral Sclerosis, Parkinson's disease, Olivo-ponto-cerebellar atrophy, anoxia such as from drowning, spinal cord and head injury, and poisoning by neurotoxins, including environmental neurotoxins.

The compounds of formula (I) may further be used to induce miosis for therapeutic purposes after certain types of examination and intraocular surgery. An example of intraocular surgery would include cateract surgery with implantation of an artificial lens. The CCK antagonist compounds of this invention can be used to prevent miosis occuring in association with iritis, ureitis and trauma.

The present invention therefore provides a compound of formula (I) or a salt or prodrug thereof for use in the preparation of a medicament.

The present invention also provides a compound of formula (I) for use in therapy.

In a further or alternative embodiment the present invention provides a method for the treatment or prevention of a physiological disorder involving CCK and/or gastrin which method comprises administration to a patient in need thereof of a CCK and/or gastrin antagonising amount of a compound of formula (I). The present invention also encompasses a pharmaceutical composition comprising a compound of formula (I), or a salt or prodrug thereof and a pharmaceutically acceptable carrier or diluent.

The compounds of formula (I) and their salts and prodrugs, may be administered to animals, preferably to mammals, and most especially to a human subject either alone or, preferably, in combination with pharmaceutically acceptable carriers or diluents, optionally with known adjuvants, such as alum, in a pharmaceutical compostion, according to standard pharmaceutical practice. The compounds can be administered orally, parenterally, including by intravenous, intramuscular, intraperitoneal or subcutaneous administration, or topically.

For oral use of an antagonist of CCK, according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavouring agents may be added.

For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

For topical administration, a compound of formula (I) may be formulated as, for example, a suspension, lotion, cream or ointment.

For topical administration, pharmaceutically acceptable carriers are, for example, water, mixtures of water and water-miscible solvents such as lower alkanols or arylalkanols, vegetable oils, polyalkylene glycols, petroleum based jelly, ethyl cellulose, ethyl oleate, carboxymethylcellulose, polyvinylpyrrolidone, isopropyl myristate and other conventionally-employed non-toxic, pharmaceutically acceptable organic and inorganic carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenyl ethanol, buffering ingredients such as sodium chloride, sodium borate, sodium acetates, gluconate buffers, and other conventional ingredients such as sorbitan monolaurate, triethanolamine, oleate, polyoxyethylene sorbitan monopalmitylate, dioctyl sodium sulfosuccinate, monothioglycerol, thiosorbitol, ethylenediamine tetraacetic acid, and the like.

The present invention further provides a process for the preparation of a pharmaceutical composition containing a compound of formula (I), which process comprises bringing a compound of formula (I) into association with a pharmaceutically acceptable carrier or excipient.

When a compound according to formula (I) is used as an antagonist of CCK or gastrin in a human subject, the daily dosage will normally be determined by the prescibing physician with the dosage generally varying according to the age, weight, and response of the individual patient, as well as the severity of the patient's symptoms. However, in most instances, an effective daily dosage wll be in the range from about 0.005mg/kg to about 100mg/kg of body weight, and preferably, of from 0.05mg/kg to about 50mg/kg, such as from about 0.5mg/kg to about 20mg/kg of body weight, administered in single or divided doses. In some cases, however, it may be

necessary to use dosages outside these limits. For example, animal experiments have indicated that doses as low as 1ng may be effective.

In effective treatment of panic syndrome, panic disorder, anxiety disorder and the like, preferably about 0.05 mg/kg to about 0.5 mg/kg of CCK antagonist may be administered orally (p.o.), administered in single or divided doses per day (b.i.d.). Other routes of administration are also suitable.

For directly inducing analgesia, anaesthesia or loss of pain sensation, the effective dosage preferably ranges from about 100 ng/kg to about 1mg/kg by intraperitoneal administration. Oral administration is an alternative route, as well as others.

In the treatment or irritable bowel syndrome, preferably about 0.1 to 10 mg/kg of CCK antagonist is administered orally (p.o.), administered in single or divided doses per day (b.i.d.). Other routes of administration are also suitable.

The use of a gastrin antagonist as a tumour palliative for gastrointestinal neoplasma with gastrin receptors, as a modulator of central nervous activity, treatment of Zollinger-Ellison syndrome, or in the treatment of peptic ulcer disease, an effective dosage of preferably about 0.1 to about 10 mg/kg administered one-to-four times daily is indicated.

For use as neuroprotective agents the effective dosage preferably ranges from about 0.5mg/kg to about 20mg/kg.

Because these compounds antagonise the function of CCK in animals, they may also be used as feed additives to increase the food intake of animals in daily dosage of preferably about 0.05mg/kg to about 50mg/kg of body weight.

The following examples are provided to assist in a further understanding of the invention. Particular materials employed, species and conditions are intended to be further illustrative of the invention and not limitative of the scope thereof.

EXAMPLE 1 N-[3(*R,S*)-5-Cyclohexyl-2,3-dihydro-1-methyl-2-oxo-1H-1,4-benzodiazepin-3-yl] N'-[3-methylphenyl] urea

Step 1: (2-Acetamidophenyl) cyclohexyl methanone:

Cyclohexylmagnesium bromide (240ml of a 2M solution in ether, 0.48mol) in ether (200ml) was added dropwise to a solution of 2-methyl-4H-3, 1-benzoxazin-4-one (100g, 0.62mol) in ether (1100ml) at -10°C over 2h. The mixture was stirred at this temperature for 2h, then at ambient temperature for 30 min. After cooling to -10°C the suspension was treated with 2M HCl (600ml), keeping the temperature below 0°C. After stirring for 15 min the layers were separated, and the ethereal layer washed sequentially with water (500ml), 5% sodium hydroxide solution (2 x 500ml) and finally water (2 x 500ml). The organic layer was separated, dried ($MgSO_4$), evaporated *in vacuo* and chromatographed on silica gel using petrol:ethyl acetate (2:1) to give (2-acetamidophenyl) cyclohexyl methanone (28g, 24%) as a pale yellow solid. mp 66°C. $^1H$ NMR ($CDCl_3$, 360MHz) δ 1.25-1.89 (10H, m), 2.23 (3H, s), 3.33 (1H, m), 7.13 (1H, d of t, J = 6 and 1Hz), 7.53 (1H, d of t, J = 6 and 1Hz), 7.92 (1H, d, J = 6Hz), 8.76 (1H, d, J = 6Hz), 11.73 (1H, brs).

Step 2: (2-Aminophenyl) cyclohexyl methanone:

A solution of (2-acetamidophenyl) cyclohexyl methanone (0.53g, 2.16mmol) in methanol (5ml) and concentrated hydrochloric acid (15ml) was heated at 80°C for 1 h. After this time the solution was cooled to ambient temperature and the solvents removed *in vacuo*. The residue was dissolved in water (10ml) and basified with 4N sodium hydroxide solution (20ml). The mixture was then extracted into ethyl acetate (4 x 20ml) and the organic layers combined and dried ($MgSO_4$). The solvent was evaporated and the residue chromatographed on silica gel using petrol:ethyl acetate (2:1), to afford the amine (0.40g, 91%) as a white solid. mp 73-75°C. $^1H$ NMR (360MHz, $CDCl_3$) δ 1.23-2.09 (10H, m), 3.27 (1H, m), 6.29 (2H, brs), 6.64 (2H, m), 7.25 (1H, dt, J = 6 and 1Hz), 7.76 (1H, dd, J = 7 and 1Hz).

An alternative procedure could be used for preparation of (2-aminophenyl) cyclohexyl methanone: To a cooled (0°C) and stirred solution of 2-aminobenzonitrile (59.5g, 0.5mol) in anhydrous diethyl ether (210ml) was added dropwise cyclohexylmagnesium chloride (2M in diethyl ether, 700ml) at such a rate as to maintain the temperature below 25°C. After a further 18h stirring at room temperature, the mixture was cooled to -60°C and treated dropwise (CAUTION! highly exothermic reaction) with 5N hydrochloric acid (600ml). The mixture was then allowed to warm to room temperature, diluted with additional 5N hydrochloric acid (500ml) and the ethereal layer was separated. The acidic aqueous solution was basified to pH 4-5 with solid potassium hydroxide and then extracted with ethyl acetate (3 x 700ml). The ethereal and ethyl acetate solutions were combined, washed

with brine (1000ml), dried ($MgSO_4$) and concentrated under vacuum to give the title compound (97g, 94%) as a pale yellow solid.

Step 3: 5-Cyclohexyl-1,3-dihydro-3(*R,S*)-[(benzyloxycarbonyl)amino]-2H- 1,4-benzodiazepin-2-one:

α-(Isopropylthio)-N-(benzyloxybonyl)glycine (30g, 0.11mol) was dissolved in dichloromethane (1000ml) and cooled to 0°C. The stirred solution was then treated with N-methyl morpholine (11.5ml, 0.11mol) followed by isobutyl chloroformate (13.7ml, 0.11mol). The resulting reaction mixture was stirred for a further 15 min at 0°C, then heated to reflux. The refluxing reaction mixture was treated dropwise, over 20 min, with a solution of (2-aminophenyl) cyclohexyl methanone (20.5g, 0.1mol) in dichloromethane (140ml). After addition was complete the reaction was heated at reflux for a further 4h. The mixture was then washed in succession with 10% citric acid solution (2 x 500ml), saturated sodium bicarbonate solution (2 x 500ml) and brine (500ml). The dried ($MgSO_4$) organic phase was evaporated to afford the crude product as a pale orange solid, which was used without further purification.

The crude (isopropylthio)glycinamide was dissolved in anhydrous tetrahydrofuran (800ml) and cooled to 0°C. Ammonia gas was bubbled through the stirred solution for 30 min before adding mercuric chloride (33g, 0.12mol) in one portion. Ammonia was continually bubbled through the solution for a further 5 hours, then the suspended solids were filtered off. The solvent was evaporated *in vacuo* to leave an oil, which was used without further purification.

The crude α-aminoglycinamide was dissolved in glacial acetic acid (500ml) and treated with ammonium acetate (36.2g, 0.47mol). The resulting reaction mixture was stirred at room temperature overnight, before removing the solvent *in vacuo*. The residue was partitioned between ethyl acetate (300ml) and 1N sodium hydroxide solution (300ml). The organic phase was separated, dried ($MgSO_4$) and evaporated. The residue was chromatographed on silica gel, using 2:1 petrol:ethyl acetate as the eluant, to afford 5-cyclohexyl-1,3-dihydro-3(*R,S*)-[(benzyloxycarbonyl)amino]-2H-1,4-benzodiazepin-2-one (25g, 64%) as a white solid. mp 164-166°C. $^1$H NMr (360MHz, $CDCl_3$) δ 1.07-2.04 (10H, m), 2.77 (1H, m), 5.12 (3H, m), 6.44 (1H, d, J = 8Hz), 7.08 (1H, d, J = 8Hz), 7.23-7.36 (6H, m), 7.46 (1H, t, J = 7Hz), 7.59 (1H, d, J = 8Hz), 8.60 (1H, brs).

Step 4: 5-Cyclohexyl-1,3-dihydro-1-methyl-3(*R,S*)-[(benzyloxycarbonyl)amino]-2H- 1,4-benzodiazepin-2-one:

A solution of 5-cyclohexyl-1,3-dihydro-3(*R,S*)-[(benzyloxycarbonyl)amino]-2H-1,4-benzodiazepin-2-one (1.1g, 2.8mmol) in dimethylformamide (13ml), under an atmosphere of nitrogen, was treated with sodium hydride (117mg of a 55-60% dispersion in mineral oil, 2.8mmol) in one portion, at -10°C. After 30 min at -10°C, iodomethane (174µl, 2.8mmol) was added in one portion and the solution allowed to reach 0°C over 1h. The solvent was then removed *in vacuo* and the crude residue partitioned between water (100ml) and dichloromethane (100ml). The organic phase was separated and the aqueous phase extracted with dichloromethane (2 x 100ml). The combined organic layers were washed with brine, dried ($MgSO_4$) and evaporated. The residue was chromatographed on silica gel, using 1:1 petrol:ethyl acetate as the eluant, to afford the title compound (0.75g, 66%) as a white solid. mp 205-207°C. $^1$H NMR (360MHz, $CDCl_3$) δ 1.03-2.04 (10H, m), 2.76 (1H, m), 3.36 (3H, s), 5.10 (3H, m), 6.52 (1H, d, J = 8Hz), 7.25-7.55 (9H, m).

Step 5: N-[3(*R,S*)-5-Cyclohexyl-2,3-dihydro-1-methyl-2-oxo-1H-1,4-benzodiazepin-3-yl] N'-[3-methylphenyl] urea:

5-Cyclohexyl-1,3-dihydro-1-methyl-3(*R,S*)-[(benzyloxycarbonyl)amino]-2H-1,4-benzodiazepin-2-one (0.34g, 0.84mmol) was dissolved in formic acid/methanol (50ml of a 4.5% ($^v/_v$) solution), and added, over 5 min, to a stirred suspension of 10% palladium on carbon (100mg, 30% ($^w/_w$)) in formic acid/methanol (10ml of a 4.5% ($^v/_v$)) solution). After 45 min the catalyst was filtered off and washed sequentially with methanol and acetone. The filtrate was evaporated *in vacuo* and the residue partitioned between ethyl acetate (100ml) and 10% sodium carbonate solution (100ml). The organic phase was separated, dried ($Na_2SO_4$) and evaporated to give a clear oil, which was used without further purification.

A solution of the crude amine (167mg, 0.61mmol) in anhydrous tetrahydrofuran (10ml) was treated with *m*-tolylisocyanate (79µl, 0.61mmol) dropwise over 5 min. After stirring at ambient temperature for 1.5h the solvent was removed under reduced pressure to leave a white solid. The solid was recrystallised from methanol to give the urea (70mg, 28%) as a white solid. mp 207-209°C. $^1$H NMR (360MHz, $CDCl_3$) δ 1.07-2.04 (10H, m), 2.29 (3H, s), 2.79 (1H, m), 3.40 (3H, m), 5.40 (1H, d, J = 8Hz), 6.71 (1H, d, J = 8Hz), 6.84 (2H, m), 7.07-7.30 (5H, m), 7.55 (2H, m).

EXAMPLE 2 N-[3(*R,S*)-5-Cyclohexyl-2,3-dihydro-1-(2-methylpropyl)-2-oxo-1H-1,4-benzodiazepin-3-yl] N'-[3-tetrazol-5-ylphenyl] urea

Step 1: 5-(3-Nitrophenyl)tetrazole:

To a solution of 3-cyanonitrobenzene (20g, 0.13mol) in 1-methyl-2-pyrrolidinone (200ml) was added triethylamine hydrochloride (27.9g, 0.20mol) followed by sodium azide (26.4g, 0.40mol). The mixture was heated at 160°C for 1.5h, then cooled to ambient temperature, poured into ice water (1000ml) and acidified using 5M HCl. The solid which precipitated from the mixture was filtered, washed with water and dried under vacuum at 50°C to afford the title tetrazole (22.1g, 86%) as a beige powder. mp 154-156°C. $^1$H NMR (360MHz, $CDCl_3$) δ 7.59 (1H, t, J = 9Hz), 8.19 (1H, d, J = 8Hz), 8.36 (1H, d, J = 8Hz), 8.86 (1H, s).

Step 2: 5-(3-Aminophenyl)tetrazole, hydrochloride salt:

To a solution of 5-(3-nitrophenyl)tetrazole (22g, 0.12mol) in ethanol (500ml) was added 10% palladium on carbon (1.5g, 7% ($^w/_w$)) in hydrochloric acid (23ml of a 5M solution). The mixture was hydrogenated at 40 psi for 10 min, then the catalyst filtered off and washed with water. The solvents were evaporated *in vacuo* and the brown solid azeotroped with toluene (4 x 100ml). The resulting solid was triturated with hot ethanol to give 5-(3-aminophenyl)tetrazole hydrochloride (16.3g, 71%) as a beige powder. mp 203-205°C. $^1$H NMR (360MHz, $D_2O$) δ 7.63 (1H, d, J = 9Hz), 7.75 (1H, t, J = 8Hz), 8.00 (2H, m).

Step 3: 5-Cyclohexyl-1,3-dihydro-1-(2-methylpropyl)-3(*R,S*)-[(benzyloxycarbonyl)amino]-2H-1,4-benzodiazepin-2-one:

A solution of 5-cyclohexyl-1,3-dihydro-3(*R,S*)-[(benzyloxycarbonyl)amino]-2H-1,4-benzodiazepin-2-one (7.0g, 18mmol) in dimethylformamide (84ml), under an atmosphere of nitrogen, was treated with sodium hydride (0.77g of a 55-60% dispersion in mineral oil, 18mmol) in one portion, at -10°C. After 30 min at -10°C, 1-iodo-2-methylpropane (2.3ml, 19.8mmol) was added in one potion and the solution allowed to reach 0°C over 2h, then stirred at ambient temperature overnight. After this time the solvent was removed under reduced pressure, and the crude residue partitioned between water (500ml) and dichloromethane (500ml). The organic phase was separated and the aqueous phase extracted with dichloromethane (2 x 500ml). The combined organic layers were washed with brine (500ml), dried ($MgSO_4$) and evaporated *in vacuo*. The residue was chromatographed on silica gel, using 2:1 petrol:ethyl acetate as the eluant, to afford the title compound (4.5g, 56%) as a white solid. mp 148-150°C. $^1$H NMR (360MHz, $CDCl_3$) δ 0.73 (3H, d, J = 7Hz), 0.79 (3H, d, J = 7Hz), 1.14-2.09 (11H, m), 2.80 (1H, m), 3.42 (1H, dd, J = 14 and 5Hz), 4.27 (1H, dd, J = 14 and 9Hz), 5.10 (3H, m), 6.55 (1H, d, J = 8Hz), 7.23-7.34 (8H, m), 7.45 (1H, t, J = 8Hz), 7.56 (1H, d, J = 8Hz).

Step 4: N-[3(*R,S*)-5-Cyclohexyl-2,3-dihydro-1-(2-methylpropyl)-2-oxo-1H-1,4-benzodiazepin-3-yl] N'-[3-tetrazol-5-ylphenyl] urea:

5-Cyclohexyl-1,3-dihydro-1-(2-methylpropyl)-3(*R,S*)-[(benzyloxycarbonyl)amino]-2H- 1,4-benzodiazepin-2-one (1.0g, 2.23mmol) was dissolved in formic acid/methanol (130ml of a 4.5% ($^v/_v$) solution), and added over 5 min to a stirred suspension of 10% palladium on carbon (0.36g, 36% ($^w/_w$)) in formic acid/methanol (27ml of a 4.5% ($^v/_v$) solution). After 4h at room temperature the catalyst was filtered off and washed sequentially with methanol and acetone. The filtrate was evaporated *in vacuo* and the solid residue partitioned between ethyl acetate (500ml) and 10% sodium carbonate solution (500ml). The organic phase was dried ($Na_2SO_4$) and evaporated to give a clear oil, which was used without further purification.

To a suspension of 5-(3-aminophenyl)tetrazole hydrochloride (0.29g, 1.45mmol) in tetrahydrofuran (10ml) was added triethylamine (0.4ml, 2.9mmol). The mixture was cooled in an ice bath and triphosgene (0.14g, 0.48mmol) added, followed by triethylamine (0.3ml, 2.2mmol). The ice bath was removed and the mixture stirred at room temperature for 30 min. A solution of the aminobenzodiazepine (0.35g, 1.11mmol), from the above procedure, in tetrahydrofuran (15ml) was added dropwise to the mixture. The reaction mixture was stirred at room temperature for 2h, then diluted with ethyl acetate (30ml) followed by 20% aqueous acetic acid (30ml). After stirring for a further 15 min a white precipitate was filtered off and washed with ethyl acetate. The solid was suspended in methanol (20ml), heated to 50°C, then filtered hot to afford N-[3(*R,S*)-5-cyclohexyl-2,3-dihydro-1-(2-methylpropyl)-2-oxo-1H-1,4-benzodiazepin-3-yl] N'-[3-tetrazol-5-ylphenyl] urea (280mg, 39%) as a white solid. mp 188-190°C. $^1$H NMr (360MHz, $D_6$-DMSO) δ 0.65 (3H, d, J = 7Hz), 0.76 (3H, d, J = 7Hz), 0.99-1.96 (11H, m), 2.97 (1H, m), 3.62 (1H, dd, J = 14 and 5Hz), 4.15 (1H, dd, J = 14 and 9Hz), 5.05 (1H, m), 7.36-7.69

(7H, m), 7.78 (1H, d, J = 8Hz), 8.15 (1H, s), 9.23 ( 1H, s).

EXAMPLE 3 N-[3(*R,S*)-5-Cyclopentyl-2,3-dihydro-1-(2-methylpropyl)-2-oxo-1H-1,4-benzodiazepin-3-yl] N'-[3-tetrazol-5-ylphenyl]urea

The title compound was prepared using the same procedure as that described for Example 2, replacing cyclohexyl with cyclopentyl. The product was purified by separating the organic layer which was dried ($MgSO_4$) and evaporated *in vacuo*. The resulting solid was subjected to preparative thin layer chromatography, eluting with chloroform-methanol-acetic acid (85:10:5). This afforded the title compound (67mg) as a colourless solid. mp 185-187°C. $^1$H NMR (360MHz, $D_6$-DMSO) δ 0.62 (3H, d, J = 7Hz), 0.75 (3H, d, J = 7Hz), 1.26 (1H, m), 1.51-1.99 (8H, m), 3.50 (1H, m), 3.61 (1H, dd, J = 14 and 5Hz), 4.16 (1H, dd, J = 14 and 10Hz), 5.10 (1H, s), 7.38-7.65 (7H, m), 7.78 (1H, d, J = 8Hz), 8.16 (1H, s), 9.22 (1H, s).

EXAMPLE 4 N-[3(*R,S*)-5-Cyclohexyl-2,3-dihydro-1-methyl-2-oxo-1H-1,4-benzodiazepin-3-yl] N'-[3-tetrazol-5-ylphenyl]urea

5-Cyclohexyl-1,3-dihydro-1-methyl-3(*R,S*)-[(benzyloxycarbonyl)amino]-2H-1,4-benzodiazepin-2-one (0.61g, 1.5mmol) was dissolved in formic acid/methanol (80ml of a 4.5% ($^v/_v$) solution), and added, over 5 min, to a stirred suspension of 10% palladium on carbon (180mg, 30% ($^w/_w$)) in formic acid/methanol (18ml of a 4.5% ($^v/_v$) solution). After 1h the catalyst was filtered off and washed sequentially with methanol and acetone. The filtrate was evaporated *in vacuo* and the residue partitioned between ethyl acetate (100ml) and 10% sodium carbonate solution (100ml). The organic phase was separated, dried ($Na_2SO_4$) and evaporated to give a clear oil, which was used without further purification.

To a suspension of 5-(3-aminophenyl)tetrazole hydrochloride (0.39g, 1.95mmol) in tetrahydrofuran (14ml) was added triethylamine (0.54ml, 3.9mmol). The mixture was cooled in an ice bath and triphosgene (0.19g, 0.65mmol) added, followed by triethylamine (0.27ml, 1.95mmol). The ice bath was removed and the mixture stirred at room temperature for 30 min. A solution of the crude aminobenzodiazepine, from the above procedure, in tetrahydrofuran (15ml) was added dropwise to the mixture. The reaction mixture was stirred at room temperature for 2h, then diluted with ethyl acetate (30ml) followed by 20% aqueous acetic acid (30ml). The two phases were separated and the organic phase dried ($MgSO_4$) and evaporated *in vacuo*. The crude residue was triturated in hot methanol (3 x 20ml) to afford the title compound (140mg, 20%) as a colourless solid. mp 203-205°C. $^1$H NMR (360MHz, $D_6$-DMSO) δ 0.92 (1H, m), 1.15-1.59 (7H, m), 1.75 (1H, m), 1.91 (1H, m), 2.93 (1H, m), 3.33 (3H, s), 5.09 (1H, d, J = 5Hz), 7.34-7.66 (7H, m), 7.75 (1H, d, J = 8Hz), 8.11 (1H, s), 9.22 (1H, s).

EXAMPLE 5 N-[3(*R,S*)-5-Cyclopentyl-2,3-dihydro-1-ethyl-2-oxo-1H-1,4-benzodiazepin-3-yl] N'-[3-tetrazol-5-ylphenyl]urea

Step 1: (2-Acetamidophenyl) cyclopentyl methanone:

Replacement of cyclohexylmagnesium bromide in Example 1, Step 1, with cyclopentylmagnesium chloride afforded the title compound as a yellow oil in 39% yield. $^1$H NMR ($CDCl_3$ 250MHz) δ 1.6-1.8 (4H, m), 1.8-2.0 (4H, m), 2.24 (3H, s), 3.75 (1H, p, J = 8Hz), 7.10 (1H, m), 7.55 (1H, m), 8.0 (1H, m), 8.7 (1H, d, J = 8Hz), 11.8 (1H, s).

Step 2: (2-Aminophenyl) cyclopentyl methanone:

A solution of (2-acetamidophenyl) cyclopentyl methanone (4.0g, 0.017mol) in methanolic potassium hydroxide solution (2M, 300ml) was heated at reflux for 12h. After this time the solvent was removed and the residue partitioned between ethyl acetate (100ml) and water (50ml). The organic phase was separated, dried ($MgSO_4$) and evaporated to give a yellow oil, which was purified by column chromatography on silica gel using 3:1 petrol:ethyl acetate as eluant, to afford the product (2.5g, 76%) as an oil. Tlc (silica, EtOAc:Pet ether (60-80°) 1:2), Rf, 0.5. $^1$H NMr (250MHz, $CDCl_3$) δ 1.53-1.80 (4H, m), 1.80-2.0 (4H, m), 3.70 (1H, p, J = 8Hz), 6.0 (2H, brs), 6.70 (2H, m), 7.15-7.3 (1H, m), 7.8 (1H, m).

Step 3: 5-Cyclopentyl-1,3-dihydro-3(*R,S*)-[(benzyloxycarbonyl)amino]-2H-1,4-benzodiazepin-2-one:

Replacement of (2-aminophenyl) cyclohexyl methanone in Example 1, Step 3, with (2-aminophenyl) cyclopentyl methanone afforded the title compound as a colourless solid in 71% yield. Tlc (silica, EtOAc:Pet ether

(60-80°) 1:2), Rf, 0.3. $^1$H NMR (250MHz, $D_6$-DMSO) δ 1.1 (1H, m), 1.40-1.75 (5H, m), 1.75-1.95 (1H, m), 2.1 (1H, m), 3.45 (1H, p, J = 8Hz), 4.9 (1H, m), 5.05 (2H, s), 7.12-7.42 (6H, m), 8.15 (1H, d, J = 8Hz), 10.6 (1H, brs).

Step 4: 5-Cyclopentyl-1,3-dihydro-1-ethyl-3(*R,S*) [(benzyloxycarbonyl)amino]-2H-1,4-benzodiazepin-2-one:

Sodium hydride (60% dispersion, 0.106g, 2.65mmol) was added portionwise to a solution of 5-cyclopentyl-1,3-dihydro-3(*R,S*)-[(benzyloxycarbonyl)amino]-2H-1,4-benzodiazepin-2-one (1g, 2.65mmol) in anhydrous dimethylformamide (30ml) cooled in ice, and the resulting mixture stirred for 1h. Ethyl iodide (0.23ml, 2.8mmol) was then added and the mixture stirred at room temperature for 16h. The solvent was then evaporated and the residue partitioned between dichloromethane (80ml) and water (20ml). The organic phase was separated, dried ($Na_2SO_4$) and evaporated. The residue was purified by column chromatography on silica gel using 2:1 petrol-:ethyl acetate as eluant to afford 0.78g (73% yield) of product as a colourless powder. mp 133-136°C. Tlc (silica, EtOAc:Pet ether (bp 60-80°) 1:2), Rf, 0.5. $^1$H NMR (250MHz, $D_6$-DMSO) δ 0.9 (3H, t, J = 8Hz), 1.1 (1H, m), 1.4-1.9 (6H, m), 2.1 (1H, m), 3.5 (1H, p, J = 7Hz), 3.7 (1H, m), 4.15-4.3 (1H, m), 4.95 (1H, d, J = 8Hz), 5.02 (2H, s), 7.2-7.8 (8H, m), 8.15 (1H, d, J = 8Hz).

Step 5: 3(*R,S*)-Amino-5-cyclopentyl-1,3-dihydro-1-ethyl-2H-1,4-benzodiazepin-2-one:

5-Cyclopentyl-1,3-dihydro-1-ethyl-3(*R,S*)-[(benzyloxycarbonyl)amino]-2H-1,4-benzodiazepin-2-one (0.5g, 1.23mmol) was dissolved in formic acid/methanol (50ml of a 4.5% (v/v) solution), and added, over 5 min, to a stirred suspension of 10% palladium on carbon (200mg, 40% (w/w)) in formic acid/methanol (10ml). After 10 min the catalyst was removed by filtration, washed with methanol and acetone and the filtrate concentrated. The residue was partitioned between ethyl acetate (100ml) and 10% sodium carbonate solution (100 ml). The organic phase was separated, dried ($Na_2SO_4$) and evaporated to give a clear oil which was used without further purification.

Step 6:N-[3(*R,S*)-5-Cyclopentyl-2,3-dihydro-1-ethyl-2-oxo-1H-1,4-benzodiazepin-3-yl] N'-[3-tetrazol-5-ylphenyl] urea:

Triethylamine (0.62ml, 4.5mmol) was added to a suspension of 5-(3-aminophenyl)tetrazole hydrochloride (292mg, 1.5mmol) in tetrahydrofuran (10ml) stirring at room temperature. The mixture was cooled in ice, and triphosgene (0.14g, 0.48mol) added in one portion. The ice bath was removed and the mixture stirred at room temperature for 30 min. A solution of crude 3(*R,S*)-amino-5-cyclopentyl-1,3-dihydro-1-ethyl-2H-1,4-benzodiazepin-2-one (307mg, 1.13mmol) in tetrahydrofuran (30ml) was added dropwise and stirring continued for 30 min. The reaction mixture was then diluted with ethyl acetate (150ml) and aqueous acetic acid (20% aqueous solution 80ml), the organic phase separated, dried ($MgSO_4$) and evaporated. The residue was purified by column chromatography on silica gel using dichloromethane: methanol: acetic acid 94:6:0.6 as eluant, to afford a gummy solid which was triturated with methanol to afford 95mg (16% yield) of the title compound as a colourless powder. mp 210°C (dec.). $^1$H NMR (360MHz, $D_6$-DMSO) δ 0.92 (3H, t, J = 7Hz), 1.05 (1H, m), 1.40-1.86 (6H, m), 2.10 (1H, m), 3.55 (1H, m), 3.62-3.80 (1H, m), 4.25 (1H, m), 5.10 (1H, m), 7.32-7.70 (7H, m), 7.79 (1H, d, J = 7Hz), 8.11 (1H, s), 9.22 (1H, s).

EXAMPLE 6 N-[3(*R,S*)-5-Cyclopentyl-2,3-dihydro-1-propyl-2-oxo- 1H-1,4-benzodiazepin-3-,yl] N'-[3-tetrazol-5-ylphenyl] urea

Carrying out Steps 1-6 of Example 5 replacing ethyl iodide, in Step 4 with n-propyl iodide, afforded the title compound as a colourless powder. mp 220°C (dec.). $^1$H NMR (360MHz, $D_6$-DMSO) δ 0.73 (3H, t, J = 7Hz), 1.08-1.44 (3H, m), 1.46-1.78 (5H, m), 1.79-1.86 (1H, m), 2.05 (1H, m,), 3.45-3.58 (1H, m), 3.63-3.74 (1H, m), 4.25 (1H, m), 5.08 (1H, m), 7.34-7.51 (4H, m), 7.57 (1H, m), 7.62 (2H, m), 7.80 (1H, d, J = 8Hz), 8.17 (1H, s), 9.27 (1H, s).

EXAMPLE 7 Chiral separation of N-[3(*R,S*)-5-cyclohexyl-2,3-dihydro-1-methyl-2-oxo-1H-1,4-benzodiazepin-3-yl] N'-[3-methylphenyl] urea

N-[3(*R,S*)-5-Cyclohexyl-2,3-dihydro-1-methyl-2-oxo-1H-1,4-benzodiazepin-3-yl] N'-[3-methylphenyl] urea (15mg, 0.04mmol) was dissolved in tetrahydrofuran (10mg/ml). 300μl of solution was injected onto a dinitrobenzoylphenyl glycine column (250 x 8.0mm i.d., 5μM) per run, using 15% ethanol in hexane as the mobile

phase. Using a flow rate of 4ml/min and U.V. detection at 280nm, the two enantiomers were efficiently separated. The fractions containing each separate enantiomer were combined and evaporated *in vacuo*.

| | |
|---|---|
| Peak A (7mg): | Retention time 9.8min. mp = 184-186°C. |
| | Purity: A:B = 96:4 |
| Peak B (7mg): | Retention time 13.3 min. mp = 186-188°C. |
| | Purity: B:A = 98:2. |

EXAMPLE 8 N-[3(*R,S*)-5-Cyclopentyl-2,3-dihydro-1-methyl-2-oxo-1H-1,4-benzodiazepin-3-yl] N'-[3-tetrazol-5-ylphenyl] urea

The title compound was prepared using the same procedure as that described for Example 5, replacing ethyl iodide in Step 4 with methyl iodide. The product was purified using preparative thin layer chromatography, eluting with chloroform-methanol-acetic acid (96:6:0.6). This afforded the desired urea (30mg) as a colourless solid. mp 193-195°C. $^1$H NMR (360MHz, $D_6$-DMSO + TFA) δ 1.15 (1H, m), 1.60 (5H, m), 1.86 (1H, m), 2.05 (1H, m), 3.36 (3H, s), 3.52 (1H, m), 5.16 (1H, s), 7.39-7.69 (7H, m), 7.81 (1H, d, J = 7Hz), 8.21 (1H, s), 9.31 (1H, s).

EXAMPLE 9 N-[3(*R,S*)-5-Cyclohexyl-2,3-dihydro-1-methyl-2-oxo-1H-1,4-benzodiazepin-3-yl] N'-[3-ethynylphenyl] urea

5-Cyclohexyl-1,3-dihydro-1-methyl-3(*R,S*)-[(benzyloxycarbonyl)amino]-2H-1,4-benzodiazepin-2-one (0.71g, 1.75 mmol) was dissolved in formic acid/methanol (104ml of a 4.5% ($^v/_v$) solution), and added, over 5 min, to a stirred suspension of 10% palladium on carbon (200mg, 30% ($^w/_w$)) in formic acid/methanol (20ml of a 4.5% ($^v/_v$) solution). After 2h the catalyst was filtered off and washed sequentially with methanol and acetone. The filtrate was evaporated *in vacuo* and the residue partitioned between ethyl acetate (200ml) and 10% sodium carbonate solution (200 ml). The organic phase was separated, dried ($Na_2SO_4$) and evaporated to give a clear oil, which was used without further purification.

A solution of 3-ethynylaminobenzene (0.27g, 2.3mmol) in anhydrous tetrahydrofuran (5ml), cooled in an ice bath, was treated with triphosgene (0.22g, 0.75mmol), followed by triethylamine (0.32ml, 2.3 mmol). The ice bath was removed and the mixture stirred at room temperature for 30 min. A solution of the aminobenzodiazepine, from the above procedure, in tetrahydrofuran (7ml) was added dropwise to the mixture. The reaction mixture was stirred at room temperature for 2h, then diluted with ethyl acetate (30ml) followed by 20% aqueous acetic acid (30ml). The organic layer was separated, dried ($MgSO_4$) and evaporated *in vacuo*. The residue was chromatographed on silica gel, using a gradient elution (2:1 petrol: ethyl acetate followed by methanol). The title compound (0.27g, 37%) was collected as a white solid, after trituration with diethyl ether. mp 198-200°C. $^1$H NMR (360MHz, $D_6$-DMSO) δ 0.90 (1H, m), 1.11 - 1.59 (7H, m), 1.76 (1H, m), 1.89 (1H, m), 2.93 (1H, m), 3.31 (3H, s), 4.09 (1H, s), 5.05 (1H, d, J = 8Hz), 7.00 (1H, d, J = 7Hz), 7.20-7.39 (4H, m), 7.54 (2H, m), 7.63 (1H, dd, J = 8 and 8Hz), 7.74 (1H, d, J = 8Hz), 9.13 (1H, s).

EXAMPLE 10 N-[3(*R,S*)-5-Cyclohexyl-2,3-dihydro-1-methyl-2-oxo-1H-1,4-benzodiazepin-3-yl] N'-[3-carboxyphenyl] urea

Sodium periodate (274mg, 1.3mmol) in water (1.5ml) was added to a vigorously stirred solution of N-[3(*R,S*)-5-cyclohexyl-2,3-dihydro-1-methyl-2-oxo-1H-1,4-benzodiazepin-3-yl] N'-[3-ethynylphenyl] urea (166mg, 0.4 mmol) in acetonitrile (1.8ml) and carbon tetrachloride (1.8ml). Ruthenium trichloride trihydrate (5mg) was then added in one portion, and the mixture stirred at room temperature for 3h. The mixture was then filtered through celite, washed with acetonitrile followed by methanol, and the filtrate evaporated *in vacuo*. The residue was purified by preparative thin layer chromatography, eluting with chloroform-methanol-acetic acid (85:10:5) to give the title compound (55mg, 32%) as a pink solid. mp 199-201°C. $^1$H NMR (360MHz, $D_6$-DMSO) δ 1.10-1.78 (8H, m), 1.80 (1H, m), 1.98 (1H, m), 3.24 (1H, m) 3.53 (3H, m), 5.22 (1H, s), 7.35-7.71 (8H, m), 8.07 (1H, s), 9.36 (1H, m).

EXAMPLE 11 N-[3(*R,S*)-5-Cyclohexyl-2,3-dihydro-1-methyl-2-oxo-1H-1,4-benzodiazepin-3-yl] N'-(3-(isopropylcarbonylaminosulphonyl)phenyl] urea

Step 1: 1-(Isopropylcarbonylaminosulphonyl)-3-nitrobenzene:

To a mixture of isobutyric acid (4.6ml, 0.05 mol), 3-nitrophenyl sulphonamide (10.1g, 0.05 mol) and 4-dimethylaminopyridine (6.1g, 0.05 mol) in anhydrous dichloromethane (400 ml) under an atmosphere of nitrogen was added 1-[3-(dimethylamino)propyl]-3-ethyl carbodiimide hydrochloride (9.6g 0.05 mol). The mixture was stirred at ambient temperature for 20 h. The mixture was extracted with 1M NaOH and the separated aqueous phase was acidified using 5M HCl. The solid which precipitated was collected by filtration, washed with water and dried under vacuum to afford the title compound (8.16g, 60%) as a colourless powder. mp 136-138°C. $^1H$ NMR (360MHz, $D_6$-DMSO) δ 0.95 (6H, d, J = 6.8Hz), 2.48 (1H, septet, J = 6.8Hz), 7.95 (1H, dd, J = 8.1 and 8.1Hz), 8.33 (1H, dd, J = 8.0 and 1.4Hz), 8.54 (1H, dd, J = 8.0, 1.4Hz), 8.60 (1H, dd, J = 1.4 and 1.4Hz), 12.38 (1H, brs).

Step 2: 1-(Isopropylcarbonylaminosulphonyl)-3-aminobenzene:

To a suspension of 1-(isopropylcarbonylaminosulphonyl)-3-nitrobenzene (5g, 18.4 mmol) in ethanol (100 ml) was added 10% palladium on carbon (0.5g, 10% ($^w/_w$)) in water (5ml). The mixture was hydrogenated at 40 psi for 10 min then the catalyst was filtered off and washed with ethanol. The solvents were evaporated *in vacuo* to give the title compound (3.4g, 76%) as a yellow solid. mp 110-112°C. $^1H$ NMR (360MHz, $D_6$-DMSO) δ 0.95 (6H, d, J = 6.9Hz), 2.45 (1H, septet, J = 6.9Hz), 5.63 (2H, br s), 6.79 (1H, ddd, J = 8.0, 2.3 and 0.8Hz), 6.96 (1H, ddd, J = 7.6, 1.7 and 0.8Hz), 7.09 (1H, dd, J = 2.0 and 2.0Hz), 7.20 (1H, dd, J = 7.9 and 7.9Hz), 11.79 (1H, brs).

Step 3: 5-Cyclohexyl-1,3-dihydro-1-methyl-3(*R,S*)-[(4-nitrophenyloxycarbonyl)amino]-2H-1,4-benzodiazepin-2-one:

5-Cyclohexyl-1,3-dihydro-1-methyl-3(*R,S*)-[(benzyloxycarbonyl)amino]-2H-1,4-benzodiazepin-2-one (1.5g, 3.7 mmol) was dissolved in formic acid/methanol (200ml of a 4.5% ($^v/_v$) solution), and added, over 5 min, to a stirred suspension of 10% palladium on carbon (500 mg, 33% ($^w/_w$)) in formic acid/methanol (20 ml of a 4.5% ($^v/_v$) solution). After 1h the catalyst was filtered off and washed sequentially with methanol and acetone. The filtrate was evaporated *in vacuo* and the residue partitioned between ethyl acetate (25ml) and 10% sodium carbonate solution (25ml). The organic phase was separated and the aqueous phase extracted with ethyl acetate (5 x 25ml). The combined organic phases were dried ($Na_2SO_4$) and evaporated *in vacuo* to give a clear oil which was used without further purification.

A solution of the crude amine (1g, 3.7 mmol) in anhydrous tetrahydrofuran (20 ml) under an atmosphere of nitrogen at O°C was treated with triethylamine (0.51ml, 3.7 mmol), followed by a solution of 4-nitrophenyl chloroformate (0.75g, 3.7 mmol) in anhydrous tetrahydrofuran (10ml) dropwise. After stirring at ambient temperature for 20 min, the solid which precipitated from the mixture was filtered and the filtrate was evaporated *in vacuo* to leave a pink solid. The solid was triturated with diethyl ether to give the title compound (1.2g, 75%) as a colourless solid. mp 165-168°C. $^1H$ NMR (360MHz, $CDCl_3$) δ 1.05 (1H, m), 1.18-1.42 (3H, m), 1.55 (1H, m), 1.65 (3H, m), 1.87 (1H, m), 2.05 (1H, m), 2.80 (1H, m), 3.43 (3H, s), 5.18 (1H, d, J = 8.3Hz), 6.90 (1H, d, J = 8.2Hz), 7.30 (4H, m), 7.57 (2H,m), 8.23 (2H, d, J = 7.1Hz).

Step 4: N-[3(*R,S*)-Cyclohexyl-2,3-dihydro-1-methyl-2-oxo-1H-1,4-benzodiazepin-3-yl] N'-[3-(isopropylcarbonylaminosulphonyl) phenyl] urea:

A solution of 5-cyclohexyl-1,3-dihydro-1-methyl-3(*R,S*)-[(4-nitrophenyloxycarbonyl)amino]-2H-1,4-benzodiazepin-2-one (0.3g, 0.69 mmol) in anhydrous dimethylformamide (5ml), under an atmosphere of nitrogen, at ambient temperature was treated with triethylamine (96 μl, 0.69 mmol). After stirring at ambient temperature for 5 min, a solution of 1-(isopropylcarbonylaminosulphonyl)-3-aminobenzene (175mg, 0.72mmol) in anhydrous dimethylformamide (5ml) was added dropwise. The bright yellow solution was heated at 50°C for 6h. The solution was cooled and the solvent evaporated *in vacuo*. The residue was partitioned between ethyl acetate (20ml) and 20% aqueous acetic acid (5ml). The organic phase was separated and the aqueous phase extracted with ethyl acetate (2 x 20ml). The combined organic layers were dried ($Na_2SO_4$) and evaporated *in vacuo*. The residue was triturated with diethyl ether to give a cream solid. This was recrystallised from hot methanol to give the title compound (76mg, 20%) as a colourless solid. mp 180°C (dec.). $^1H$ NMR (360MHz, $D_6$-DMSO) δ 0.92 (6H, d, J = 6.8Hz), 1.10-1.66 (8H,m), 1.78 (1H, m), 1.92 (1H, m), 2.43 (1H, septet, J = 6.8Hz), 2.92 (1H, m), 3.38 (3H, s), 5.06 (1H, d, J = 8.2Hz), 7.37 (6H, m), 7.64 (1H, dd, J =7.7 and 7.7Hz), 7.75 (1H, d, J = 7.9Hz), 8.06 (1H, s), 9.40 (1H, s), 11.94 (1H, brs).

EXAMPLE 12 N-[3(*R,S*)-5-Cyclohexyl-2,3-dihydro-1-methyl-2-oxo-1H-1,4-benzodiazepin-3-yl] N'-[3-(isopropylsulphonylaminocarbonyl)phenyl] urea

Step 1: Isopropylsulphonamide:

Ammonia gas was bubbled through a stirred solution of isopropylsulphonyl chloride (3.9ml, 35mmol) in anhydrous tetrahydrofuran (100ml), cooled to O°C, for 30 min. After allowing to warm to ambient temperature, the mixture was altered and the filtrate evaporated *in vacuo*, to leave a white solid. This was partitioned between ethyl acetate (50ml) and water (50ml). The organic phase was separated and the aqueous phase extracted with ethyl acetate (3 x 50ml). The combined organic layers were dried ($Na_2SO_4$) and evaporated *in vacuo* to afford the title compound (3.5g, 81%) as a colourless solid. mp 51-53°C. $^1H$ NMR (360m Hz, $CDCl_3$) δ 1.92 (6H, d, J = 6.7Hz), 3.22 (1H, septet, J = 6.7Hz), 4.61 (2H, brs).

Step 2: 1-(Isopropylsuphonylaminocarbonyl)-3-nitrobenzene:

The title compound was prepared in the same way as that described in Example 11, Step 1, using isopropylsulphonamide (1.7g, 13.8 mmol), 3-nitrobenzoic acid (2.31g, 13.8 mmol), 4-dimethylaminopyridine (1.69g, 13.8 mmol), 1-[3-(dimethylamino)propyl]-3-ethyl carbodiimide hydrochloride (2.65g, 13.8 mmol) and anhydrous dichloromethane (100ml). The compound (2.74g, 75%) was afforded as a colourless solid. mp 175-177°C. $^1H$ NMR (360MHz, $D_6$-DMSO) δ 1.34 (6H, d, J = 6.9Hz), 3.83 (1H, septet, J = 6.9Hz), 7.83 (1H, dd, J = 8.0 and 8.0Hz), 8.35 (1H, d, J = 8.0Hz), 8.48 (1H, d, J = 8.0Hz), 8.78 (1H, s), 12.40 (1H, brs).

Step 3:1-(Isopropylsuphonylaminocarbonyl)-3-aminobenzene:

In the same way as that described in Example 11, Step 2, using 1-(isopropylsuphonylaminocarbonyl)-3-nitrobenzene (2.5g, 9.2 mmol), 10% palladium on carbon (0.25g, 10% (w/w)) in water (2ml) and ethanol (50ml), the title compound was afforded as a yellow solid. This was recrystallised from ethanol to give a pale yellow crystalline solid (1.7g, 76%). mp 190-193°C. $^1H$ NMR (360MHz, $D_6$-DMSO) δ 1.30 (6H, d, J = 6.9Hz), 3.79 (1H, septet, J = 6.9Hz), 5.36 (2H, brs), 6.79 (1H, dd, J = 7.9 and 1.2Hz), 7.05 (2H, m), 7.13 (1H, dd, J = 7.8 and 7.8Hz).

Step 4: N-[3(*R,S*)-5-Cyclohexyl-2,3-dihydro-1-methyl-2-oxo-1H-1,4-benzodiazepin-3-yl] N'-[3-(isopropylsulphonylaminocarbonyl) phenyl] urea:

The title compound was prepared in the same way as that described in Example 11, Step 4, using 5-cyclohexyl-1,3-dihydro-1-methyl-3(*R,S*)-[(4-nitrophenyloxycarbonyl)amino]-2H-1,4-benzodiazepin-2-one (0.3g, 0.69 mmol), triethylamine (96µl, 0.69 mmol), dimethylformamide (6ml) and 1-(isopropylsulphonylaminocarbonyl)-3-aminobenzene (0.175 mg, 0.72 mmol). After recrystallisation from ethanol the title compound (0.24g, 65%) was afforded as a colourless solid. mp 165°C (dec.). $^1H$ NMR (360MHz, $D_6$-DMSO) δ 0.87-0.98 (1H, m), 1.10-1.64 (7H, m), 1.30 (6H, d, J = 6.9Hz), 1.79 (1H, m), 1.88-1.96 (1H, m), 2.95 (1H, m), 3.33 (3H, s), 3.79 (1H, septet, J = 6.9Hz), 5.07 (1H, d, J = 8.2Hz), 7.37 (3H, m), 7.46 (1H, d, J = 7.8Hz), 7.55 (2H, m), 7.64 (1H, dd, J = 7.1 and 7.1Hz), 7.75 (1H, d, J = 7.9Hz), 7.92 (1H,s), 9.22 (1H,s), 11.93 (1H, brs).

EXAMPLE 13 N-(3(*R,S*)-5-Cyclohexyl-2,3-dihydro-1-methyl-2-oxo-1H-1,4-benzodiazepin-3-yl] N'-[3-(phenylcarbonylaminosulphonyl)phenyl] urea

Step 1: 1-(Phenylcarbonylaminosulphonyl)-3-nitrobenzene:

The title compound was prepared in the same way as that described in Example 11, Step 1, using 3-nitrobenzenesulphonamide (10.1g, 50 mml), benzoic acid (6.1g, 50mmol), 4-dimethylaminopyridine (6.1g, 50mmol), 1-[3-(dimethylamino)propyl]-3-ethyl carbodiimide hydrochloride (9.59g, 50mmol) and anhydrous dichloromethane (400ml). The title compound (13.1g, 86%) was afforded as a colourless solid. mp 181-183°C. $^1H$ NMR (360 MHz, $D_6$-DMSO) δ 7.49 (2H, m), 7.61-7.66 (1H, m), 7.86-7.89 (2H, m), 7.96 (1H, dd, J = 8.0 and 8.0Hz), 8.43 (1H, m), 8.56 (1H, m), 8.71 (1H, m).

Step 2: 1-(Phenylcarbonylaminosulphonyl)-3-aminobenzene:

In the same way as that described in Example 11, Step 2, using 1-(phenylcarbonylaminosulphonyl)-3-nitrobenzene (5.3g, 17.3mmol), 10% palladium on carbon (0.5g, 9% (w/w)) in water (3ml) and ethanol (100ml), the title compound (4.3g, 90%) was afforded as a yellow solid. mp 160-162°C. $^1H$ NMR (360 MHz, $D_6$-DMSO) δ 6.81 (1H, m), 7.07 (1H, m), 7.22 (2H, m), 7.48 (2H, m), 7.60 (1H, dd, J = 7.4 and 7.4Hz), 7.87 (2H, m).

Step 3: N-[3(*R,S*)-5-Cyclohexyl-2,3-dihydro-1-methyl-2-oxo-1H-1,4- benzodiazepin-3-yl] N′-[3-phenylcarbonylaminosulphonyl) phenyl] urea:

The title compound was prepared in the same way as that described in Example 11, Step 4, using 5-cyclohexyl-1,3-dihydro-1-methyl-3(*R,S*)-[(4-nitrophenyloxycarbonyl)amino]-2H-1,4-benzodiazepin-2-one (0.3g, 0.69mmol), triethylamine (96μl, 0.69 mmol), dimethylformamide (6 ml) and 1-(phenylcarbonylaminosulphonyl)-3-aminobenzene (0.21g, 0.76 mmol). After trituration with methanol, the compound (0.16g, 41%) was afforded as a colourless solid. mp 200-202°C. $^1H$ NMR (360 MHz, $D_6$-DMSO) δ 0.91 (1H, m), 1.10-1.40 (3H, m), 1.42-1.69 (4H, m), 1.78 (1H, m), 1.92 (1H, m), 2.94 (1H, m), 3.32 (3H, s), 5.06 (1H, d, J = 8.1Hz), 7.36 (2H, m), 7.45-7.56 (6H, m), 7.62 (2H, m), 7.75 (1H, d, J = 7.9Hz), 7.84 (2H, m), 8.17 (1H, s), 9.42 (1H, s), 12.50 (1H, brs).

EXAMPLE 14 N-[3(*R,S*)-5-Cyclohexyl-2,3-dihydro-1-methyl-2-oxo-1H-1,4-benzodiazepin-3-yl] N′-[3-(phenylsulphonylaminocarbonyl) phenyl] urea

Step 1: 1-(Phenylsulphonylaminocarbonyl)-3-nitrobenzene:

The title compound was prepared in the same way as that described in Example 11, Step 1, using benzenesulphonamide (4.7g, 30mmol), 3-nitrobenzoic acid (5g,30mmol), 4-dimethylamino pyridine (3.66g, 30mmol), 1-[3-(dimethylamino)propyl]-3-ethyl carbodiimide hydrochloride (5.74g, 30mmol), and anhydrous dichloromethane (200ml). The compound (8.05g, 88%) was afforded as a colourless solid. mp 188-190°C. $^1H$ NMR (360MHz, $D_6$-DMSO) δ 7.65 (2H, m), 7.72 (1H, m), 7.79 (1H,dd, J = 8.0 and 8.0Hz), 8.02 (2H, m), 8.28 (1H, dd, J = 8.0 and 1.5Hz), 8.45 (1H, dd, J = 8.0 and 1.5Hz), 8.72 (1H, m).

Step 2: 1-(Phenylsulphonylaminocarbonyl)-3-amino-benzene:

In the same way as that described in Example 11, Step 2, using 1-(phenylsulphonylaminocarbonyl)-3-nitrobenzene (5g, 16mmol), 10% palladium on carbon (0.5g, 10% (w/w)) in water (3ml) and ethanol (100ml), the title compound (3g,67%) was afforded as a pale beige solid after recrystallisation from ethanol. mp 135-138°C. $^1H$ NMR (360MHz, $D_6$-DMSO) δ 6.75-6.78 (1H, m), 6.96-6.98 (2H, m), 7.10 (1H, dd, J = 8.0 and 8.0Hz), 7.60-7.73 (3H, m), 7.97 (2H, m).

Step 3: N-[3(*R,S*)-5-Cyclohexyl-2,3-dihydro-1-methyl-2-oxo-1H-1,4-benzodiazepin-3-yl] N′-[3-(phenylsulphonylaminocarbonyl) phenyl] urea:

The title compound was prepared in the same way as that described in Example 11, Step 4, using 5-cyclohexyl-1,3-dihydro-1-methyl-3(*R,S*)-[(4-nitrophenyloxycarbonyl)amino]-2H-1,4-benzodiazepin-2-one (0.3g, 0.69 mmol), triethylamine (96 μl, 0.69 mmol), dimethylformamide (6ml) and 1-(phenylsulphonylaminocarbonyl)-3-aminobenzene (0.21g, 0.76mmol). After recrystallisation from ethanol the compound (0.23g, 58%) was isolated as a colourless solid. mp 215-217°C. $^1H$ NMR (360MHz, $D_6$-DMSO) δ 0.85-0.98 (1H, m), 1.08-1.39 (3H, m), 1.41-1.66 (4H, m), 1.78 (1H, m), 1.89 (1H, m), 2.95 (1H, m), 3.32 (3H, s), 5.06 (1H, d, J = 8.3Hz), 7.32 (2H, m), 7.39 (2H, m), 7.52 (2H, m), 7.62 (3H, m), 7.72 (2H, m), 7.84 (1H,s), 7.97 (2H, m), 9.17 (1H,s), 12.50 (1H, brs).

EXAMPLE 15 N-[3(*R,S*)-5-Cyclohexyl-2,3-dihydro-1-methyl-2-oxo-1H-1,4-benzodiazepin-3-yl] N′-[3-(methylcarbonylaminosulphonyl)phenyl] urea

Step 1: 1-(Methylcarbonylaminosulphonyl)-3-nitrobenzene:

The title compound was prepared in the same way as that described in Example 11, Step 1, using 3-nitrobenzenesulphonamide (5.0g, 25mmol), acetic acid (1.43 ml, 24mmol), 4-dimethylaminopyridine (4.8g, 25mmol), 1-[3-(dimethylamino)propyl]-3-ethyl carbodiimide hydrochloride (4.8g, 25 mmol) and anhydrous dichloromethane (420 ml). The title compound (5.4g, 92%) was afforded as a colourless solid. mp 187-190°C. $^1H$ NMR (360MHz, $D_6$-DMSO) δ 1.96 (3H, s), 7.95 (1H, dd, J = 8.0 and 8.0Hz), 8.34 (1H, dd, J = 8.0 and 1.6Hz), 8.56 (1H, dd, J = 8.0 and 1.6Hz), 8.62 (1H, dd, J = 1.6 and 1.6Hz), 12.42 (1H, brs).

Step 2: 1-(Methylcarbonylaminosulphonyl)-3-aminobenzene:

In the same way as that described in Example 11, Step 2, using 1-(methylcarbonylaminosulphonyl)-3-nitrobenzene (2.9g, 12 mmol), 10% palladium on carbon (0.4g, 14% (w/w)) in water (3ml) and ethanol (150ml), the title compound (1.8g, 70%) was afforded as a colourless solid. mp 148-150°C. $^1H$ NMR (360MHz, $D_6$-DMSO) δ 1.92 (3H, s), 5.64 (2H, brs), 6.80 (1H, dd, J = 8.0 and 1.7Hz), 6.98 (1H, d, J = 7.6Hz), 7.10 (1H, dd, J = 2.0 and 2.0Hz), 7.21 (1H, dd, J = 7.9 and 7.9Hz), 11.87 (1H, brs).

Step 3: N-[3*(R,S)*-5-Cyclohexyl-2,3-dihydro-1-methyl-2-oxo-1H-1,4-benzodiazepin-3-yl] N'-[3-(methylcarbonylaminosulphonyl) phenyl] urea:

The title compound was prepared in the same way as that described in Example 11, Step 4, using 5-cyclohexyl-1,3-dihydro- 1-methyl-3(*R,S*)-[(4-nitrophenyloxycarbonyl)amino]-2H-1,4-benzodiazepin-2-one (0.5g, 1.2 mmol), triethylamine (0.16ml, 1.2 mmol), dimethylformamide (10 ml) and 1-(methylcarbonylaminosulphonyl)-3-aminobenzene (0.26g, 1.2 mmol). After recrystallisation from methanol, the title compound (0.2g, 34%) was afforded as a colourless solid. mp 195-198°C. $^1H$ NMR (360MHz, $D_6$-DMSO) δ 0.87-0.99 (1H, m), 1.10-1.66 (7H, m), 1.78 (1H, m), 1.94 (1H, m), 1.91 (3H, s), 2.95 (1H, m), 3.30 (3H, s), 5.07 (1H, d, J = 8.3Hz), 7.39 (4H, m), 7.54 (2H, m), 7.64 (1H, dd, J = 7.9 and 7.9Hz), 7.76 (1H, d, J = 7.9Hz), 8.07 (1H, s), 9.42 (1H, s), 11.99 (1H, brs).

EXAMPLE 16 N-(3(*R,S*)-5-Cyclohexyl-2,3-dihydro-1-methyl-2-oxo-1H-1,4-benzodiazepin-3-yl] N'-[3-(methylsulphonylaminocarbonyl)phenyl] urea

Step 1: 1-(Methylsulphonylaminocarbonyl)-3-nitrobenzene:

A solution of methylsulphonamide (5.37g, 57mmol) in anhydrous dichloromethane (100ml), cooled to O°C was treated with triethylamine (7.9ml, 57mmol) followed by a solution of 3-nitrobenzoyl chloride (10g, 54 mmol) in anhydrous dichloromethane (100ml) dropwise. After stirring for 2h at O°C, the reaction mixture was washed with 1M HCl (100ml). The precipitate which formed was collected by filtration and triturated with diethyl ether and was then recrystallised from methanol to afford the title compound (4.3g, 31%) as a colourless crystalline solid. mp 175-178°C. $^1H$ NMR (360MHz, $D_6$-DMSO) δ 3.42 (3H, s), 7.82 (1H, dd, J = 8.0 and 8.0Hz), 8.38 (1H, d, J = 8.0Hz), 8.49 (1H, d, J = 8.0Hz), 8.80 (1H,s).

Step 2: 1-(Methylsulphonylaminocarbonyl)-3-aminobenzene:

In the same way as that described in Example 11, Step 2, using 1-(methylsulphonylaminocarbonyl)-3-nitrobenzene (4g, 16mmol), 10% palladium on carbon (0.5g, 12.5% ($^w/_w$)) in water (5ml) and ethanol (100ml), the title compound (2.9g, 83%) was afforded as a tan powder after trituration with diethyl ether. mp 153-155°C. $^1H$ NMR (360 MHz, $D_6$-DMSO) δ 3.3 (3H, s), 6.79 (1H, d, J = 7.7Hz), 7.05 (1H, d, J = 7.7Hz), 7.08 (1H, d, J = 1.9Hz), 7.13 (1H, dd, J = 7.7 and 7.7 Hz).

Step 3: N-[3*(R,S)*-5-Cyclohexyl-2,3-dihydro-1-methyl-2-oxo-1H-1,4-benzodiazepin-3-yl] N'-[3-(methylsulphonylaminocarbonyl) phenyl] urea:

The title compound was prepared in the same way as that described in Example 11, Step 4, using 5-cyclohexyl-1,3-dihydro-1-methyl-3*(R,S)*-[(4-nitrophenyloxycarbonyl)amino]-2H-1,4-benzodiazepin-2-one (0.5g, 1.2mmol), triethylamine (0.16ml, 1.2mmol), dimethylformamide (10ml) and 1-(methylsulphonylaminocarbonyl)-3-nitrobenzene (0.25g, 1.2mmol). After recrystallisation from ethanol, the compound (0.15g, 26%) was afforded as a pale beige solid. mp 175°C (dec.). $^1H$ NMR (360MHz, $D_6$-DMSO) δ 0.88 (1H, m), 1.10-1.67 (7H, m), 1.72-1.81 (1H, m), 1.91 (1H, m), 2.95 (1H, m), 3.32 (3H, s), 3.34 (3H, s) 5.08 (1H, d, J = 8.2Hz), 7.37 (3H,m), 7.47 (1H, d, J = 7.9Hz), 7.56 (2H, m), 7.64 (1H, dd, J = 7.0 and 7.0Hz), 7.75 (1H, d, J = 7.9Hz), 7.91 (1H, s), 9.21 (1H, s), 12.07 (1H, brs).

EXAMPLE 17 N-[3(*R,S*)-5-Cyclohexyl-2,3-dihydro-1-methyl-2-oxo-1H-1,4-benzodiazepin-3-yl] N'-[3-(trifluoromethylcarbonylaminosulphonyl)phenyl] urea

Step 1: 1-(Trifluoromethylcarbonylaminosulphonyl)-3-nitrobenzene:

A solution of 3-nitrophenylsulphonamide (5g, 25mmol) in anhydrous dimethylformamide (50ml), under at atmosphere of nitrogen, cooled to 0°C, was treated with sodium hydride (1.08g of a 55-60% dispersion in mineral oil, 27.5mmol) in one portion. The mixture was stirred at 0°C for 2h and then trifluoroacetic anhydride (3.8ml, 27.5mmol) was added dropwise. After stirring at 0°C for a further 1h, the solvent was evaporated *in vacuo*. The residue was partitioned between dichloromethane (50ml) and water (50ml). The organic phase was separated and the aqueous phase extracted with dichloromethane (5 x 50ml). The combined organic layers were evaporated *in vacuo* and azeotroped with toluene (2 x 50ml). The residue was chromatographed on silica gel eluting with dichloromethane:methanol:acetic acid (85:10:5). The impure mixture was purified further by chromatography on silica gel, eluting with ethyl acetate:petrol (1:1) followed by ethyl acetate, to afford the title compound (2.7g, 37%) as a colourless solid. mp 241°C (dec.). $^1$H NMR (360 MHz, $D_6$-DMSO) δ 7.78 (1H, dd, J = 7.9 and 7.9Hz), 8.20 (1H, d, J = 7.5Hz), 8.35 (1H, dd, J = 8.3 and 2.3Hz), 8.54 (1H, dd, J = 2.3 and 2.3Hz).

Step 2: 1-(Trifluoromethylcarbonylaminosulphonyl)-3-amino benzene:

In the same way as that described in Example 11, Step 2, using 1-(trifluoromethylcarbonylaminosulphonyl)-3-nitrobenzene (2.7g, 9.1mmol), 10% palladium on carbon (0.25g, 9% ($^w/_w$)) in water (3ml) and ethanol (70ml), the title compound (1.7g, 70%) was afforded as a colourless solid. mp 106-108°C. $^1$H NMR (360MHz, $D_6$-DMSO) δ 6.59 (1H, m), 6.88 (1H, m), 7.02 (2H, m).

Step 3: N-[3(*R,S*)-5-Cyclohexyl-2,3-dihydro-1-methyl-2-oxo-1H-1,4-benzodiazepin-3-yl]N'-[3-(trifluoromethylcarbonylaminosulphonyl)phenyl] urea:

The title compound was prepared in the same way as that described in Example 11, Step 4, using 5-cyclohexyl-1,3-dihydro-1-methyl-3(*R,S*)-[(4-nitrophenyloxycarbonyl)amino]-2H-1,4-benzodiazepin-2-one (0.5g, 1.2mmol), triethylamine (0.16ml, 1.2mmol), dimethylformamide (10ml) and 1-(trifluoromethyl carbonylaminosulphonyl)-3-aminobenzene (0.32g, 1.2mmol). The product was purified by chromatography on silica gel eluting with dichloromethane-methanol (9:1). This afforded the title compound (60mg, 9%) as a pale beige solid. mp 190°C (dec.). $^1$H NMR (360 MHz, $D_6$-DMSO) δ 0.86-1.00 (1H, m), 1.10-1.67 (7H,m), 1.76 (1H, m), 1.89-1.98 (1H, m), 2.95 (1H, m), 3.33 (3H, s) 5.08 (1H, m), 7.28 (3H, m), 7.38 (1H, m), 7.52 (2H, m), 7.64 (1H, m), 7.77 (2H, m), 9.21 (1H,s).

EXAMPLE 18 N-[3(*R,S*)-5-Cyclohexyl-2,3-dihydro-1-methyl-2-oxo-1H-1,4-benzodiazepin-3-,yl] N'-[3-(trifluoromethylsulphonylaminocarbonyl)phenyl] urea

Step 1: Trifluoromethylsulphonamide:

The title compound was prepared using the same procedure as described in Example 12, Step 1, replacing isopropylsulphonyl chloride with trifluoromethylsulphonyl chloride. The compound was purified by trituration with hexane to give the product (8.5g, 96%) as a colourless solid. mp 116-119°C.

Step 2: 1-(Trifluoromethylsulphonylaminocarbonyl)-3-nitrobenzene:

A solution of trifluoromethylsulphonamide (2.1g, 14.1mmol) in anhydrous dichloromethane (50ml), cooled to 0°C was treated with triethylamine (2.0ml, 14.1mmol) followed by a solution of 3-nitrobenzoyl chloride (2.5g, 13.4 mmol) in anhydrous dichloromethane (50ml) dropwise. After stirring at 0°C for 1h, the mixture was stirred at ambient temperature for 2h. The mixture was extracted using 1M NaOH (100ml), and the aqueous phase was acidified using 5M HCl. The aqueous phase was then extracted using dichloromethane (4 x 100ml), followed by ethyl acetate (2 x 100ml). The combined organic layers were dried ($Na_2SO_4$) and evaporated *in vacuo* to leave a yellow solid. This was recrystallised from diethyl ether:hexane (1:1) to afford the title compound (1.5g, 36%) as a pale yellow solid. mp 80-83°C. $^1$H NMR (360 MHz, $D_6$-DMSO) δ 7.73 (1H, dd, J = 8.0 and 8.0Hz), 8.35 (2H, m), 8.56 (1H, brs), 8.68 (1H, m).

Step 3: 1 (Trifluoromethylsulphonylaminocarbonyl)-3-aminobenzene:

In the same way as that described in Example 11, Step 2, using 1-(trifluoromethylsulphonylaminocarbonyl)-3-nitrobenzene (1.5g, 5.6mmol), 10% palladium on carbon (0.2g, 13% ($^w/_w$)) in water (3ml) and ethanol (40ml), the title compound was afforded as a cream solid. This was triturated with diethyl ether to give a colourless solid (0.91g, 68%). mp 255-257°C. $^1$H NMR (360MHz, $D_6$-DMSO) δ 7.43 (1H, m), 7.53 (1H, dd, J = 7.9 and 7.9Hz), 7.94 (2H, m).

Step 4: N-[3*(R,S)*-5-Cyclohexyl-2,3-dihydro-1-methyl-2-oxo-1H-1,4-benzodiazepin-3-yl] N′-[3-(trifluoro methylsulphonylaminocarbonyl) phenyl] urea:

The title compound was prepared in the same way as that described in Example 11, Step 4, using 5-cyclohexyl-1,3-dihydro-1-methyl-3*(R,S)*-[(4-nitrophenyloxycarbonyl)amino]-2H-1,4-benzodiazepin-2-one (0.5g, 1.2 mmol), triethylamine (0.16ml, 1.2mmol) dimethylformamide (10ml) and 1-(trifluoromethylsulphonylaminocarbonyl)-3-aminobenzene (0.32g, 1.2mmol). After recrystallisation from ethanol the compound (0.17g, 26%) was afforded as a colourless solid. mp 175°C (dec.). $^1$H NMR (360MHz, $D_6$-DMSO) δ 0.88-1.03 (1H, m) 1.08-1.41 (3H, m), 1.42-1.66 (4H, m) 1.78 (1H, m), 1.93 (1H, m), 2.96 (1H, m), 3.33 (3H, s), 5.10 (1H, brs), 7.21 (1H, dd, J = 7.9 and 7.9Hz), 7.19-7.31 (1H, m), 7.39 (1H, dd, J = 7.2Hz), 7.47 (1H, d, J = 7.8Hz), 7.57 (2H, m), 7.65 (1H, dd, J = 7.1 and 7.1Hz), 7.78 (2H, m), 9.11 (1H, s).

EXAMPLE 19 N-(3*(R,S)*-5-Cyclohexyl-2,3-dihydro-1-(2-methylpropyl)-2-oxo-1H-1,4-benzodiazepin-3-yl] N′-[3-(methylsulphonylaminocarbonyl)phenyl] urea

Step 1: 5-Cyclohexyl-1,3-dihydro-1-(2-methylpropyl)-3*(R,S)*-[(4-nitrophenyloxycarbonyl)amino]-2H-1,4-benzodiazepin-2-one:

5-Cyclohexyl-1,3-dihydro-1-(2-methylpropyl)-3(*R,S*)-[(benzyloxycarbonyl)amino]-2H-1,4-benzodiazepin-2-one (1.0g, 2.24 mmol) was dissolved in formic acid/methanol (100ml of a 4.5% ($^v/_v$) solution), and added over 5 min to a stirred suspension of 10% palladium on carbon (0.3g, 30% ($^w/_w$)) in formic acid/methanol (10ml of a 4.5% ($^v/_v$) solution). After 1.5h at ambient temperature, the catalyst was filtered off and washed sequentially with methanol and acetone. The filtrate was evaporated *in vacuo* and the residue partitioned between ethyl acetate (25ml) and 10% sodium carbonate solution (25ml). The organic phase was separated and the aqueous phase extracted with ethyl acetate (2 x 25ml). The combined organic phases were dried ($Na_2SO_4$) and evaporated *in vacuo* to give a yellow gum which was used without further purification.

A solution of the crude amine (0.7g, 2.24mmol) in anhydrous tetrahydrofuran (15ml) under an atmosphere of nitrogen, at 0°C, was treated with triethylamine (0.31ml, 2.24mmol), followed by a solution of the 4-nitrophenyl chloroformate (0.45g, 2.24mmol) in anhydrous tetrahydrofuran (10ml) dropwise. After stirring at ambient temperature for 20 min, the solid which precipitated from the mixture was filtered and the filtrate was evaporated *in vacuo* to leave a pale yellow solid. The solid was triturated with diethyl ether to give the title compound (0.88g, 82%) as a colourless solid. mp 163-165°C. $^1$H NMR (360 MHz, $CDCl_3$) δ 0.76 (3H, d, J = 6.7Hz), 0.82 (3H, d, J = 6.7Hz), 1.12-1.44 (5H, m), 1.53-1.79 (4H, m) 1.85-1.95 (1H, m), 2.05-2.14 (1H, m), 2.85 (1H, m), 3.47 (1H, dd J = 13.8 and 4.3Hz), 4.32 (1H, dd, J = 13.8 and 9.3Hz), 5.13 (1H, d, J = 8.1Hz), 6.93 (1H, d, J = 8.1Hz), 7.26-7.39 (4H, m), 7.52 (1H, m), 7.60 (1H, d, J = 6.5Hz), 8.22 (2H, m).

Step 2: N-[3*(R,S)*-5-Cyclohexyl-2,3-dihydro-1-(2-methylpropyl)-2-oxo-1H-1,4-benzodiazepin-3-yl] N′-[3-(methylsulphonylaminocarbonyl) phenyl] urea:

The title compound was prepared from 5-cyclohexyl-1,3-dihydro-1-(2-methylpropyl)-3(*R,S*)-[(4-nitrophenyloxycarbonyl)amino]-2H-1,4-benzodiazepin-2-one (0.3g, 0.63 mmol), triethylamine (87μl, 0.63mmol), dimethylformamide (6ml) and 1-(methylsulphonylaminocarbonyl)-3-aminobenzene (0.15g, 0.69mmol) [Example 16, Step 2] using the procedure described in Example 11, Step 4. The product (0.17g, 49%) was afforded as a pale cream solid after trituration with methanol. mp 250-252°C. $^1$H NMR (360MHz, $D_6$-DMSO) δ 0.64 (3H, d, J = 6.6Hz), 0.76 (3H, d, J = 6.7Hz), 0.92-1.70 (9H, m), 1.74-1.82 (1H, m), 1.98 (1H, m), 2.90-3.30 (1H, m), 3.33 (3H, m), 3.62 (1H, dd, J = 14.0 and 4.7Hz), 4.14 (1H, dd, J = 13.9 and. 9.4Hz), 5.04 (1H, m), 7.37 (3H, m), 7.47 (1H, m), 7.60 (3H, m), 7.78 (1H, m), 7.90 (1H, s), 9.18 (1H, s), 12.06 (1H, brs).

EXAMPLE 20 N-[3(*R,S*)-5-Cyclopentyl-2,3-dihydro-1-ethyl-2-oxo-1H-1,4-benzodiazepin-3-yl] N′-[3-(methylcarbonylaminosulphonyl) phenyl] urea

Step 1: 5-Cyclopentyl-1,3-dihydro-1-ethyl-3(*R,S*)-[(4-nitrophenyloxycarbonyl)amino]-2H-1,4-benzodiazepin-2-one:

A solution of crude 3(*R,S*)-amino-5-cyclopentyl-1,3-dihydro-1-ethyl-2H-1, 4-benzodiazepin-2-one [Example 5, Step 5] (0.23g, 0.92 mmol) in dry tetrahydrofuran (15 ml) under an atmosphere of nitrogen, at 0°C, was treated with triethylamine (0.11 ml, 0.92 mmol) followed by a solution of 4-nitrophenyl chloroformate (184mg, 0.92 mmol) in tetrahydrofuran (10 ml). After stirring at ambient temperature for 30 min the solid which precipitated was removed by filtration and the filtrate concentrated *in vacuo* to afford the title compound (0.32g, 79%) as a colourless solid. mp 136-138°C. $^1$H NMR (360 MHz, $CDCl_3$) δ 0.9 (3H, t, J = 7Hz), 1.0-2.2 (8H,m), 3.50 (1H, p, J = 7 Hz), 3.70 (1H, m), 4.18-4.28 (1H, m), 5.14 (1H, d, J = 8 Hz), 7.30-8.20 (8H, m), 9.12 (1H, d, J = 8 Hz).

Step 2: N-[3(*R,S*)-5-Cyclopentyl-2,3-dihydro-1-ethyl-2-oxo-1H-1,4-benzodiazepin-3-yl] N′-[3-(methylcarbonylaminosulphonyl) phenyl] urea:

This compound was prepared from 5-cyclopentyl-1,3-dihydro-1-ethyl-3(*R,S*)-[(4-nitrophenyloxycarbonyl)amino]-2H-1,4-benzodiazepin-2-one (141mg, 0.32mmol) and 1-(methylcarbonylaminosulphonyl)-3-aminobenzene [Example 15, Step 2] (68mg, 0.32mmol) using the procedure described in Example 11, Step 4, to afford the product (50mg, 30%) as a colourless powder. mp 202-204°C. $^1$H NMR (250 MHz, $D_6$-DMSO) δ 0.9 (3H, t, J = 7 Hz), 1.00-1.20 (1H, m), 1.40-1.90 (6H, m), 1.91 (3H, s), 2.00-2.20 (1H, m), 3.45-3.62 (1H, m) 3.64-3.84 (1H, m), 4.16-4.36 (1H, m), 5.05 (1H, d, J = 8 Hz), 7.30-7.90 (8H, m), 8.06 (1H, s), 9.41 (1H, s), 12.01 (1H, s).

EXAMPLE 21 N-[3(*R,S*)-5-Cyclohexyl-2,3-dihydro-1-methyl-2-oxo-1H-1,4-benzodiazepin-3-yl] N′-[3-(1,3,4-thiadiazol-2-ylaminosulphonyl)phenyl]urea

Step 1: 1-(1,3,4-Thiadiazol-2-ylaminosulphonyl)-3-nitrobenzene:

To a stirred suspension of 2-amino-1,3,4-thiadiazole (1.5g, 15mmol) in anhydrous pyridine (6ml) at 0°C, under nitrogen, was added 3-nitrobenzenesulphonyl chloride (3.5g, 16mmol) portionwise. The mixture turned yellow and set solid. The mixture was then heated at 120°C for 1h before aqueous sodium hydroxide (20% ($^w$/w), 3.3ml) was added cautiously. Heating was continued for a further 15 min then the solution allowed to cool to ambient temperature. The mixture was evaporated *in vacuo* and the residue evaporated with water (2 x 50ml) followed by toluene (2 x 50ml). The residue was then taken up in water and the resultant brown solid collected by filtration. This was then recrystallised from glacial acetic acid. The title compound (1.4g, 37%) was isolated as a yellow solid, which contained one mole of acetic acid. mp 189-192°C. $^1$H NMR (250MHz, D6-DMSO) δ 7.88 (1H, dd, J = 8 and 8Hz), 8.23 (1H, dd, J = 8 and 1Hz), 8.45 (2H, m), 8.83 (1H, s).

Step 2: 1-(1,3,4-Thiadiazol-2-ylaminosulphonyl)-3-aminobenzene:

A suspension of 1-(1,3,4-thiadiazol)-2-ylaminosulphonyl)-3-nitrobenzene (1.3g, 4.6mmol) in ethanol (50ml)/water (5ml)/5N hydrochloric acid (10ml) was hydrogenated for 4h at 40 psi, using a palladium on carbon catalyst (0.5g, 38% ($^w/_w$)). After this time the catalyst was filtered off and the filtrate evaporated *in vacuo*. After azeotroping with toluene (20ml) the resultant solid was dissolved in water (30ml) and the solution adjusted to pH 5 using 1M sodium hydroxide solution. The mixture was extracted with ethyl acetate (2 x 20ml) and the organic layers combined and dried ($Na_2SO_4$). The filtrate was evaporated *in vacuo* and the residue azeotroped with toluene (20ml) then triturated with anhydrous ether. The title compound (408mg, 35%) was isolated as a beige solid. mp 155-158°C. $^1$H NMR (360MHz, D6-DMSO) δ 6.72 (1H, dd, J = 8 and 2Hz), 6.87 (1H, d, J = 8Hz), 6.98 (1H, dd, J = 2 and 2Hz), 7.15 (1H, dd, J = 8 and 8Hz), 8.76 (1H, s).

Step 3: N-[3(*R,S*)-5-Cyclohexyl-2,3-dihydro-1-methyl-2-oxo-1H-1,4-benzodiazepin-3-yl] N′-[3-(1,3,4-thiadiazol-2-ylaminosulphonyl)phenyl]urea:

To a stirred solution of 5-cyclohexyl-1,3-dihydro-1-methyl-3(*R,S*)-[(4-nitrophenyloxycarbonyl)-amino]-2H-1,4-benzodiazepin-2-one [Example 11, Step 3], (200mg, 0.46mmol) in anhydrous dimethylformamide (3ml), under nitrogen, was added triethylamine (63μl, 0.46mmol) dropwise over 5 min. After stirring at room temperature

for a further 5 min a solution of 1-( 1,3,4-thiadiazol-2-ylaminosulphonyl)-3-aminobenzene (117mg, 0.46mmol) in dimethylformamide (3ml) was added dropwise over 5 min. The solution was then heated at 60°C for 5h, then the mixture allowed to cool to ambient temperature. The solvent was removed *in vacuo* and the residue partitioned between ethyl acetate (20ml) and 20% aqueous acetic acid (20ml). The organic phase was separated, dried ($MgSO_4$) and evaporated *in vacuo*. The residue was azeotroped with toluene (2 x 20ml), then triturated in toluene (20ml) and the solid filtered off. The solid was triturated in hot methanol, filtered and stirred in anhydrous ether overnight. The title compound (63mg, 24%) was isolated as a pale pink solid. mp 233-235°C. $^1H$ NMR (360MHz, D6-DMSO) δ 0.91 (1H, m), 1.14-1.88 (9H, m), 2.93 (1H, m), 3.32 (3H, s), 5.05 (1H, d, J = 8Hz), 7.30-7.40 (5H, m), 7.55 (1H, d, J = 8Hz), 7.64 (1H, dd, J = 7Hz), 7.75 (1H, d, J = 8Hz), 8.01 (1H, s), 8.76 (1H, s), 9.33 (1H, s).

EXAMPLE 22 N-[3(*R,S*)-5-Cyclohexyl-2,3-dihydro-1-methyl-2-oxo-1H-1,4-benzodiazepin-3-yl] N′-[3-(2-pyrazinylaminosulphonyl)phenyl]urea

Step 1: 1-(2-Pyrazinylaminosulphonyl)-3-nitrobenzene:

To a stirred solution of 2-aminopyrazine (4.1g, 0.043mol) in anhydrous pyridine (17ml) at 0°C, under nitrogen, was added 3-nitrobenzenesulphonyl chloride (10g, 0.045mol) portionwise. The mixture was warmed to room temperature and stirred for 3h. After this time aqueous sodium hydroxide (18% ($^w/_w$), 10ml) was added cautiously. Stirring was continued for a further 15 min then the mixture was evaporated *in vacuo*. The residue was then taken up in water (100ml) and evaporated once more. The solid was taken up in water (100ml) and filtered off. This material was chromatographed on silica gel, using dichloromethane:methanol (95:5) as the eluant, to afford the sulphonamide (1.15g, 10%) as a brown solid. $^1H$ NMR (360MHz, D6-DMSO) δ 7.91 (1H, dd, J = 8 and 8Hz), 8.24 (1H, d, J = 2Hz), 8.27 (1H, d, J = 2Hz), 8.37 (2H, m), 8.46 (1H, dd, J = 8 and 2Hz), 8.72 (1H, s), 12.00 (1H, brs). MS (CI, $NH_3$) 281 (M+1).

Step 2: N-[3(*R,S*)-5-Cyclohexyl-2,3-dihydro-1-methyl-2-oxo-1H-1,4-benzodiazepin-3-yl] N′-[3-(2-pyrazinylaminosulphonyl)phenyl]urea:

1-(2-Pyrazinylaminosulphonyl)-3-nitrobenzene (1.15g, 4.1mmol) was added portionwise to a hot suspension of iron powder (4g) in ethanol (15ml)/5N hydrochloric acid (1ml) under nitrogen. The mixture was heated at reflux for 3h then the mixture filtered whilst hot. On cooling a precipitate separated which was collected by filtration and triturated with ether. The solid (142mg, 14%) was assumed to be the desired aniline and was used without further purification.

To a stirred solution of 5-cyclohexyl-1,3-dihydro-1-methyl-3(*R,S*)-[(4-nitrophenyloxycarbonyl)amino]-2H-1,4-benzodiazepin-2-one (335mg, 0.77mmol) [Example 11, Step 3] in anhydrous tetrahydrofuran (4ml), under nitrogen, was added triethylamine (106μl, 0.77mmol) dropwise over 5 min. After stirring at room temperature for a further 5 min a solution of the aniline (194mg, 0.77mmol) (prepared as described above) in anhydrous tetrahydrofuran (4ml) was added dropwise over 5 min. The mixture was headed at reflux for 12h, then cooled to ambient temperature and evaporated *in vacuo*. The residue was partitioned between ethyl acetate (20ml) and 20% aqueous acetic acid (20ml). The undissolved solid was filtered off and washed with anhydrous ether. On standing more solid precipitated from the filtrate and was collected by filtration. The solids were combined and subjected to preparative thin layer chromatography, using dichloromethane:methanol:acetic acid 94:6:0.4 as the eluant. The title compound (60mg, 14%) was isolated as a beige solid. mp 255-257°C (dec.). $^1H$ NMR (360MHz, D6-DMSO + TFA) δ 0.97 (1H, m), 1.15-1.91 (9H, m), 2.96 (1H, m), 3.34 (3H, s), 5.1 (1H, brs), 7.38-7.49 (5H, m), 7.56 (1H, d, J = 8Hz), 7.65 (1H, d, J = 7Hz), 7.78 (1H, d, J = 7Hz), 8.15 (1H, s), 8.21 (2H, m), 8.36 (1H, s), 9.42 (1H, s).

EXAMPLE 23 N-[3(*R,S*)-5-Cyclohexyl-2,3-dihydro-1-methyl-2-oxo-1H-1,4-benzodiazepin-3-yl] N′-[3-(ethylsulphonylaminocarbonyl)phenyl]urea

Step 1: Ethylsulphonamide:

The title compound was prepared using the same procedure as described in Example 12, Step 1, replacing isopropylsulphonyl chloride with ethylsulphonyl chloride. The compound was purified by trituration with hexane to give the product (6.17g, 73%) as a colourless solid. mp 53-56°C. $^1H$ NMR (360MHz, $D_6$-DMSO) δ 1.22 (3H, t, J = 7.4Hz), 2.95 (2H, q, J = 7.4Hz), 6.69 (2H, brs).

Step 2: 1-(Ethylsulphonylaminocarbonyl)-3-nitrobenzene:

The title compound was prepared in the same way as that described in Example 11, Step 1, using ethyl sulphonamide (3g, 27.5mmol), 3-nitrobenzoic acid (4.6g, 27.5mmol), 4-dimethylaminopyridine (3.36g, 27.5mmol), 1-[3-(dimethylamino)propyl]-3-ethyl carbodiimide hydrochloride (5.28g, 27.5mmol) and anhydrous dichloromethane (200ml). The title compound (6g, 84%) was afforded as a colourless solid. mp 178-181°C. $^1$H NMR (360MHz, $D_6$-DMSO) δ 1.28 (3H, t, J = 7.4Hz), 3.54 (2H, q, J = 7.4Hz), 7.83 (1H, dd, J = 8.0 and 8.0Hz), 8.33-8.38 (1H, m), 8.47-8.50 (1H, m), 8.75-8.90 (1H, m), 12.40 ( 1H, brs).

Step 3: 1-(Ethylsulphonylaminocarbonyl)-3-aminobenzene:

In the same way as that described in Example 11, Step 2, using 1-(ethylsulphonylaminocarbonyl)-3-nitrobenzene (3g, 11.6mmol), 10% palladium on carbon (0.3g, 10% ($^w/_w$)) in water (3ml) and ethanol (100ml), the title compound (1.95g, 74%) was afforded as a pale beige solid after recrystallisation from ethanol. mp 129-132°C. $^1$H NMR (360MHz, $D_6$-DMSO) δ 1.24 (3H, t, J = 7.4Hz), 3.47 (2H, q, J = 7.4Hz), 5.40 (2H, brs), 6.77-6.83 (1H, m), 7.02-7.08 (2H, m), 7.13 (1H, dd, J = 7.8 and 7.7Hz).

Step 4: N-[3(*R,S*)-5-Cyclohexyl-2,3-dihydro-1-methyl-2-oxo-1H-1,4-benzodiazepin-3-yl] N'-[3-(ethylsulphonylaminocarbonyl)phenyl]urea:

The title compound was prepared in the same way as that described in Example 11, Step 4, using 5-cyclohexyl-1,3-dihydro-1-methyl-3(*R,S*)-[(4-nitrophenyloxycarbonyl)amino]-2H-1,4-benzodiazepin-2-one (0.25g, 0.57mmol), triethylamine (80μl, 0.57mmol), dimethylformamide (5ml) and 1-(ethylsulphonylaminocarbonyl)-3-nitrobenzene (0.14g, 0.63mmol). After trituration with hot methanol, the compound (125mg, 42%) was afforded as a colourless solid. mp 210°C (dec.). $^1$H NMR (360MHz, $D_6$-DMSO) δ 0.86-0.98 (1H, m), 1.10-1.25 (2H, m), 1.23 (3H, t, J = 7.4Hz), 1.26-1.64 (5H, m), 1.72-1.80 (1H, m), 1.87-1.96 (1H, m), 2.88-2.99 (1H, m), 3.33 (3H, s), 3.48 (2H, q, J = 7.4Hz), 5.08 (1H, d, J = 8.3Hz), 7.31-7.42 (3H, m), 7.47 (1H, d, J = 7.9Hz), 7.52-7.58 (2H, m), 7.67 (1H, dd, J = 7.9 and 7.8Hz), 8.76-8.80 (1H, m), 7.92 (1H, dd, J = 1.8 and 1.8Hz), 9.21 (1H, s), 11.98 (1H, brs).

EXAMPLE 24 Chiral separation of N-[3(*R,S*)-5-cyclohexyl-2,3-dihydro-1-methyl-2-oxo-1H-1,4-benzodiazepin-3-yl] N'-[3-(isopropylsulphonylaminocarbonyl)phenyl]urea

N-[3(*R,S*)-5-Cyclohexyl-2,3-dihydro-1-methyl-2-oxo-1H-1,4-benzodiazepin-3-yl] N'-[3-(isopropylsulphonyl aminocarbonyl)phenyl]urea (110mg, 0.20mmol) [Example 12], was dissolved in tetrahydrofuran (40mg/ml). 150μl of solution was injected onto a dinitrobenzoyl leucine column (250 x 8.0mm i.d., 5μm) per run, using 2% methanol in dichloromethane plus 0.5% acetic acid as the mobile phase. Using a flow rate of 4ml/min and UV detection at 300nm, the two enantiomers were efficiently separated. The fractions containing each separate enantiomer were combined and evaporated *in vacuo*.

| | |
|---|---|
| Peak A (45mg): | Retention time 6 min. mp 190°C (dec.). |
| | Purity: A:B > 99:1 |
| Peak B (40mg): | Retention time 13 min. mp 195°C (dec.). |
| | Purity: A:B 97:3. |

EXAMPLE 25 N-[3(*R,S*)-5-Cyclohexyl-2,3-dihydro-1-propyl-2-oxo-1H-1,4-benzodiazepin-3-yl] N'-(3-(methylsulphonylaminocarbonyl)phenyl]urea

Step 1: 5-Cyclohexyl-1,3-dihydro-1-propyl-3(*R,S*)-[(benzyloxycarbonyl)amino]-2H-1,4-benzodiazepin-2-one:

A solution of 5-cyclohexyl-1,3-dihydro-3(*R,S*)-[(benzyloxycarbonyl)amino]-2H-1,4-benzodiazepin-2-one (2g, 5.1mmol) in anhydrous dimethylformamide (15ml), under an atmosphere of nitrogen, was treated with sodium hydride (0.22g of a 55-60% dispersion in mineral oil, 5.1mmol) in one portion, at 0°C. After 45 min at 0°C, 1-iodopropane (0.55ml, 5.6mmol) was added in one portion and the solution allowed to reach ambient temperature and stirred overnight. After this time the solvent was removed under reduced pressure, and the crude residue partitioned between water (25ml) and dichloromethane (25ml). The organic phase was separated and the aqueous phase extracted with dichloromethane (3 x 25ml). The combined organic layers were washed with brine (50ml), dried ($MgSO_4$) and evaporated *in vacuo*. The residue was triturated with diethyl ether to give the title compound (1.74g, 79%) as a colourless solid. mp 160-163°C. $^1$H NMR (360MHz, $CDCl_3$) δ 0.82 (3H, t, J

= 10.5Hz), 0.94-1.48 (5H, m), 1.50-1.76 (5H, m), 1.79-1.90 (1H, m), 1.96-2.08 (1H, m), 2.70-2.84 (1H, m), 3.46-3.59 (1H, m), 4.22-4.35 (1H, m), 5.06-5.16 (3H, m), 5.07 (1H, d, J = 12.0Hz), 7.21-7.40 (7H, m), 7.44-7.59 (2H, m).

Step 2: 5-Cyclohexyl-1,3-dihydro-1-propyl-3(*R,S*)-[(4-nitrophenyloxycarbonyl)amino]-2H-1,4-benzodiazepin-2-one:

5-Cyclohexyl-1,3-dihydro-1-propyl-3(*R,S*)-[(benzyloxycarbonyl)amino]-2H-1,4-benzodiazepin-2-one (1.6g, 3.7mmol) was dissolved in formic acid/methanol (130ml of a 4.5% ($^v/_v$) solution), and added over 5 min to a stirred suspension of 10% palladium on carbon (0.4g, 25% ($^w/_w$)) in formic acid/methanol (20ml of a 4.5% ($^v/_v$) solution). After 1h at ambient temperature, the catalyst was filtered off and washed sequentially with methanol and acetone. The filtrate was evaporated *in vacuo* and the residue partitioned between ethyl acetate (25ml) and 10% sodium carbonate solution (25ml). The organic phase was separated and the aqueous phase extracted with ethyl acetate (3 x 25ml). The combined organic phases were dried ($Na_2SO_4$) and evaporated *in vacuo* to give a yellow gum which was used without further purification.

A solution of the crude amine (1.1g, 3.7mmol) in anhydrous tetrahydrofuran (15ml) under an atmosphere of nitrogen, at 0°C, was treated with triethylamine (0.51ml, 3.7mmol), followed by a solution of 4-nitrophenyl chloroformate (0.74g, 3.7mmol) in anhydrous tetrahydrofuran (15ml) dropwise. After stirring at ambient temperature for 15 min, the solid which precipitated from the mixture was removed by filtration, and the filtrate was evaporated *in vacuo* to leave an orange solid. The solid was triturated with diethyl ether to give the title compound (1.3g, 76%) as a colourless solid. mp 152-155°C. $^1H$ NMR (360MHz, $CDCl_3$) δ 0.85 (3H, t, J = 7.4Hz), 1.02-1.50 (6H, m), 1.56-1.76 (4H, m), 1.84-1.93 (1H, m), 2.00-2.08 (1H, m), 2.76-2.87 (1H, m), 3.53-3.63 (1H, m), 4.26-4.36 (1H, m), 5.14 (1H, d, J = 8.0Hz), 6.93 (1H, d, J = 8.3Hz), 7.22-7.40 (4H, m), 7.50-7.62 (2H, m), 8.18-8.26 (2H, m).

Step 3: N-[3(*R,S*)-5-Cyclohexyl-2,3-dihydro-1-propyl-2-oxo-1H-1,4-benzodiazepin-3-yl] N'-[3-(methylsulphonylaminocarbonyl)phenyl]urea:

The title compound was prepared from 5-cyclohexyl-1,3-dihydro-1-propyl-3(*R,S*)-[(4-nitrophenyloxycarbonyl)amino]-2H-1,4-benzodiazepin-2-one (0.3g, 0.65mmol), triethylamine (90μl, 0.65mmol), dimethylformamide (6ml) and 1-(methylsulphonylaminocarbonyl)-3-aminobenzene (0.15g, 7.1mmol) [Example 16, Step 2] using the procedure described in Example 11, Step 4. The product (0.16g, 46%) was afforded as a cream solid after trituration with hot methanol. mp 205°C (dec.). $^1H$ NMR (360MHz, $D_6$-DMSO) δ 0.73 (3H, t, J = 7.3Hz), 0.86-1.00 (1H, m), 1.08-1.42 (5H, m), 1.43-1.54 (2H, m), 1.57-1.67 (2H, m), 1.74-1.83 (1H, m), 1.87-1.96 (1H, m), 2.91-3.01 (1H, m), 3.33 (3H, s), 3.62-3.72 (1H, m), 4.16-4.26 (1H, m), 5.04 (1H, d, J = 7.8Hz), 7.34-7.43 (3H, m), 7.47 (1H, d, 7.8Hz), 7.56-7.68 (3H, m), 7.77 (1H, d, J = 7.7Hz), 7.91 (1H, dd, J = 1.9 and 1.9Hz), 9.20 (1H, s), 12.04 (1H, brs).

EXAMPLE 26 N-[3(*R,S*)-5-Cyclohexyl-2,3-dihydro-1-methyl-2-oxo-1H-1,4-benzodiazepin-3-yl] N'-[3-(thiazol-2-ylaminosulphonyl)phenyl]urea

Step 1: 1-(Thiazol-2-ylaminosulphonyl)-3-nitrobenzene:

To a solution of 2-aminothiazole (1g, 10.5mmol) in dry pyridine (3ml) was added, portionwise, 3-nitrobenzenesulphonyl chloride (2.32g, 10.5mmol), maintaining the internal temperature below 55°C. After addition was complete, the mixture was heated at 100°C for 75 min. 4M Sodium hydroxide solution (2.75ml, 11mmol) was then added dropwise and heating was continued for a further 5 min. After cooling, water (25ml) was added and the precipitate was collected by filtration to afford the product (2.48g, 87%) as a brown solid. mp 195°C (dec.). $^1H$ NMR (360MHz, $D_6$-DMSO) δ 6.91 (1H, d, J = 4.6Hz), 7.31 (1H, d, J = 4.5Hz), 7.86 (1H, dd, J = 8.0 and 8.1Hz), 8.20-8.26 (1H, m), 8.40-8.45 (1H, m), 8.46-8.49 (1H, m), 13.00 (1H, brs).

Step 2: 1-(Thiazol-2-ylaminosulphonyl)-3-aminobenzene:

To a suspension of 1-(thiazol-2-ylaminosulphonyl)-3-nitrobenzene (0.5g, 1.8mmol) in ethanol (50ml) was added 10% palladium on carbon (0.25g, 50% ($^w/_w$)) in water (2ml) and 5M hydrochloric acid (1ml, 5mmol). The mixture was hydrogenated at 45 psi for 3h. The catalyst was filtered off and washed with ethanol. The solvents were evaporated *in vacuo* and the residue chromatographed on silica gel using 10% methanol in dichloromethane as the eluant to afford the product (0.34g, 76%) as a pale yellow solid. mp 187-190°C. $^1H$ NMR (360MHz,

$D_6$-DMSO) δ 5.49 (2H, brs), 6.66-6.73 (1H, m), 6.80 (1H, d, J = 4.6Hz), 6.85-6.90 (1H, m), 6.98-7.02 (1H, m), 7.11 (1H, dd, J = 7.9 and 7.9Hz), 7.22 (1H, d, J = 4.7Hz), 12.59 (1H, brs).

Step 3: N-[3(*R,S*)-5-Cyclohexyl-2,3-dihydro-1-methyl-2-oxo-1H-1,4-benzodiazepin-3-yl] N'-[3-(thiazol-2-ylaminosulphonyl)phenyl]urea:

The title compound was prepared from 5-cyclohexyl-1,3-dihydro-1-methyl-3(*R,S*)-[(4-nitrophenyloxycarbonyl)amino]-2H-1,4-benzodiazepin-2-one (0.3g, 0.69mmol), triethylamine (96μl, 0.69mmol), dimethylformamide (6ml) and 1-(thiazol-2-ylaminosulphonyl)-3-aminobenzene (0.19g, 0.76mmol) using the procedure described in Example 11, Step 4. The product (125mg, 33%) was afforded as a cream solid after trituration with methanol. mp 230°C (dec.). $^1$H NMR (360MHz, $D_6$-DMSO) δ 0.84-0.96 (1H, m), 1.08-1.65 (7H, m), 1.71-1.81 (1H, m), 1.86-1.94 (1H, m), 2.88-2.96 (1H, m), 3.32 (3H, s), 5.06 (1H, d, J = 8.2Hz), 6.80 (1H, d, J = 4.6Hz), 7.22 (1H, d, J = 4.6Hz), 7.28-7.42 (5H, m), 7.55 (1H, d, J = 7.4Hz), 7.64 (1H, dd, J = 7.1 and 7.0Hz), 7.75 (1H, d, J = 7.9Hz), 7.98 (1H, s), 9.30 (1H, s), 12.63 (1H, brs).

EXAMPLE 27 (-)-N-[3(*R*)-5-Cyclohexyl-2,3-dihydro-1-methyl-2-oxo-1H-1,4-benzodiazepin-3-yl] N'-[3-(phenylsulphonylaminocarbonyl)phenyl]urea

Step 1: 3(*R,S*)-Amino-5-cyclohexyl-1,3-dihydro-1-methyl-2H-1,4-benzodiazepin-2-one:

A mixture of 5-cyclohexyl-1,3-dihydro-1-methyl-3(*R,S*)-[(benzyloxycarbonyl)amino]-2H-1,4-benzodiazepin-2-one (3.0g, 7.4mmol) and hydrobromic acid (45% in acetic acid, 6.2ml) was stirred for 1h at room temperature under an atmosphere of nitrogen. The mixture was then diluted with cold anhydrous diethyl ether (40ml) and stirred at 0°C for 45 min. The white precipitate was collected by filtration, washed with cold diethyl ether (4 x 30ml) and then dissolved in a mixture of water (30ml) and aq. sodium hydroxide (2M, 15ml). The basic aqueous phase was extracted with ethyl acetate (3 x 70ml) and the combined organic layers were washed with brine (30ml), dried ($Na_2SO_4$) and concentrated. The residue was chromatographed on silica gel, using 94:6, dichloromethane:methanol as the eluant, to afford the title compound (1.6g, 80%) as a pale pink solid. mp 133-136°C. $^1$H NMR (360MHz, $CDCl_3$) δ 1.02-1.40 (4H, m), 1.47-1.56 (1H, m), 1.61-1.74 (3H, m), 1.84-1.91 (1H, m), 1.96-2.06 (1H, m), 2.17 (2H, brs), 2.70-2.80 (1H, m), 3.39 (3H, s), 4.29 (1H, s), 7.20-7.27 (2H, m), 7.44-7.54 (2H, m).

Step 2: 3(*R,S*)-[2(*R*)-(*tert*-Butyloxycarbonyl)amino-3-phenylpropionylamino]-5-cyclohexyl-1,3-dihydro-1-methyl-2H-1,4-benzodiazepin-2-one:

To a solution of 3(*R,S*)-amino-5-cyclohexyl-1,3-dihydro-1-methyl-2H-1,4-benzodiazepin-2-one (4g, 14.8mmol) in anhydrous dimethylformamide (35ml), under an atmosphere of nitrogen, was added in succession Boc-D-phenyl-alanine (4.11g, 15.4mmol), 1-hydroxybenzotriazole trihydrate (2.09g, 15.4mmol) and 1-ethyl-3-[3-(dimethylamino) propyl]carbodiimide hydrochloride (2.97g, 15.4mmol). Triethylamine (2.16ml, 15.4mmol) was then added and the resulting suspension was stirred at ambient temperature for 20 min. The solvent was removed under reduced pressure and the residue was partitioned between ethyl acetate (50ml) and 10% citric acid solution (50ml). The organic phase was separated and the aqueous phase extracted with ethyl acetate (3 x 50ml). The combined organic phases were washed with 10% sodium hydroxide solution (50ml), water (50ml) and brine (50ml), dried ($MgSO_4$) and evaporated *in vacuo*. The residue was chromatographed on silica gel, using 1:1 petrol:ethyl acetate as the eluant, to afford the product (7.26, 95%) as a pale yellow solid. mp 95-98°C. $^1$H NMR (360MHz, $CDCl_3$) δ 0.99-1.11 (1H, m), 1.16-1.72 (7H, m), 1.40 (9H, s), 1.83-1.92 (1H, m), 1.98-2.06 (1H, m), 2.73-2.83 (1H, m), 3.10-3.24) (2H, m), 3.38 (3H, s), 4.53 (1H, brs), 4.98 (1H, brs), 5.28-5.34 (2H, m), 7.19-7.32 (7H, m), 7.49-7.58 (2H, m).

Step 3: (+)-3(*R*)-(2(*R*)-Amino-3-phenylpropionylamino)-5-cyclohexyl-1,3-dihydro-1-methyl-2H-1,4-benzodiazepin-2-one:

3(*R,S*)-[2(*R*)-(*tert*-Butyloxycarbonyl)amino-3-phenylpropionylamino]-5-cyclohexyl-1,3-dihydro- 1-methyl-2H-1,4-benzodiazepin-2-one (4.7g, 9.1mmol ) was dissolved in ethyl acetate (20ml) and cooled to 0°C. This solution was then saturated with hydrogen chloride gas. After 1.5h, the resulting precipitate (which was shown to be the undesired diastereoisomer, $R_f$= 0.04 ethyl acetate), was removed by filtration and the filtrate evaporated. The solid residue was partitioned between ethyl acetate (25ml) and 10% sodium carbonate solution (20ml). The organic phase was separated and the aqueous extracted with ethyl acetate (2 x 25ml). The combined organic phases were dried ($Na_2SO_4$) and evaporated *in vacuo*. The residue was chromatographed on silica gel using a gradient elution of 0-20% methanol in ethyl acetate to afford the title compound (1.66g, 44%, $R_f$ = 0.13 ethyl acetate) as a pale yellow solid. mp 100-103°C. $^1$H NMR (360MHz, $CDCl_3$) δ 1.00-1.39 (4H, m), 1.50-1.72 (4H, m), 1.84-1.92 (1H, m), 2.00-2.07 (1H, m), 2.72-2.84 (2H, m), 3.28 (1H, dd, J = 13.8 and 4.0Hz), 3.40 (3H, s), 3.69 (1H, dd, J = 9.8 and 4.1Hz), 5.36 (1H, d, J = 8.3Hz), 7.21-7.36 (7H, m), 7.47-7.58 (2H, m), 8.66 (1H, d, J = 8.3Hz). $[\alpha]_D^{23}$ +32.7° (c = 0.58, $CH_3OH$).

The undesired diastereoisomer (Rf 0.04, ethyl acetate) could be epimerised to 3(*R,S*)-(2(*R*)-amino-3-phenylpropionylamino)-5-cyclohexyl-1,3-dihydro- 1-methyl-2H-1,4-benzodiazepin-2-one using the following procedure:

The undesired diastereoisomer (Rf 0.04, ethyl acetate) (18.6g, 0.044mol) was dissolved in anhydrous ether (200ml), and potassium-*tert*-butoxide (0.68g, 6.1mmol) was added. The mixture was stirred at room temperature for 1h, then more potassium-*tert*-butoxide (0.68g, 6.1mmol) was added and the mixture heated at reflux for 5h. The mixture was then cooled to ambient temperature, the solvent removed under vacuum, and the residue partitioned between ethyl acetate (200ml) and water (200ml). The organic layer was separated, dried ($MgSO_4$), filtered and evaporated in vacuo to afford the epimerised material.

Step 4: (+)-N-[1(*R*)-2-[(3(*R*)-5-Cyclohexyl-2,3-dihydro-1-methyl-2-oxo-1H-1,4-benzodiazepin-3-yl)amino]-2-oxo-1-(phenylmethyl)ethyl] N'-phenyl thiourea:

A solution of (+)-3(*R*)-(2(*R*)-amino-3-phenylpropionylamino)-5-cyclohexyl-1,3-dihydro-1-methyl-2H-1,4-benzodiazepin-2-one (1.6g, 3.83mmol) in anhydrous dichloromethane (10ml) was treated with phenyl isothiocyanate (0.5ml, 4.21mmol), and then heated on the steam bath for 30 min. The solvent was evaporated *in vacuo* and the residue was chromatographed on silica gel with 1:1, ethyl acetate:petrol as the eluant, to afford the product (2.1g, 100%) as a pale yellow solid. mp 129-132°C. $^1$H NMR (360MHz, $CDCl_3$) δ 0.95-1.07 (1H, m), 1.15-1.37 (3H, m), 1.45-1.69 (4H, m), 1.81-1.88 (1H, m), 1.93-2.00 (1H, m), 2.70-2.80 (1H, m), 3.24-3.41 (2H, m), 3.38 (3H, s), 5.23 (1H, d, J = 7.3Hz), 5.31-5.40 (1H, m), 6.67 (1H, 7.0Hz), 6.87-7.02 (2H, m), 7.20-7.35 (9H, m), 7.46-7.52 (2H, m), 7.65 (1H, s). $[\alpha]^{25}_D$ +27.3° (c = 0.31, $CH_2Cl_2$).

Step 5: (-)-N-[3(*R*)-5-Cyclohexyl-2,3-dihydro-1-methyl-2-oxo-1H-1,4-benzodiazepin-3-yl] N'-[3-(phenylsulphonylaminocarbonyl)phenyl]urea:

(+)-N-[1(*R*)-2-[(3(*R*)-5-Cyclohexyl-2,3-dihydro- 1-methyl-2-oxo-1H-1,4-benzodiazepin-3-yl)amino]-2-oxo-1-(phenylmethyl)ethyl] N'-phenyl thiourea (1g, 1.8mmol) was dissolved in trifluoroacetic acid (10ml) and heated to 55°C for 15 min. The trifluoroacetic acid was removed under reduced pressure and the residue azeotroped with dichloromethane (2 x 10ml) and toluene (2 x 10ml). The residue was chromatographed on silica gel using 90:10:0.1:0.1, dichloromethane:methanol:acetic acid:water as the eluant to afford an orange gum. This was dissolved in ethyl acetate (40ml), cooled to 0°C, and treated with 10% sodium carbonate solution (3ml). After stirring for 1 min, the organic layer was separated and the aqueous re-extracted with ethyl acetate (2 x 20ml). The combined organics were dried ($Na_2SO_4$) and evaporated *in vacuo* to afford an orange solid. This was assumed to be the amine and was used without further purification.

1-(Phenylsulphonylaminocarbonyl)-3-aminobenzene (297mg, 1.08mmol) was dissolved in anhydrous tetrahydrofuran (25ml) and cooled to 0°C under an atmosphere of nitrogen. Triphosgene (106mg, 0.36mmol) was added in one portion and the mixture was stirred for 2 min. The mixture was then treated with triethylamine (0.45ml, 3.23mmol) in portions of 129, 129, 64, 64 and 64μl over a period of 5 min. The mixture was allowed to warm to 15°C over a period of 10 min and was then re-cooled to 0°C. The amine (0.2g, 0.74mmol, prepared as described above) was dissolved in anhydrous tetrahydrofuran (5ml) and added to the reaction dropwise. The mixture was stirred at 0°C for 5 min and then stirred at ambient temperature for 40 min. The precipitated solid was removed by filtration and washed with tetrahydrofuran. The filtrate was evaporated and partitioned between ethyl acetate (200ml) and 10% citric acid solution (40ml). The organic phase was separated and washed with more 10% citric acid solution (40ml) and brine (40ml), and dried ($Na_2SO_4$). The solvent was evaporated *in vacuo* and the residue was chromatographed on silica gel using a gradient elution of 5-10% methanol in dichloromethane to afford the product (0.42g, 99%) as a colourless solid. This was dissolved in tetrahydrofuran (40mg/ml) and 150μl of solution was injected onto a dinitrobenzoyl leucine column (250 x 8.0mm id., 5μm) per run, using 10% methanol in dichloromethane plus 0.4% acetic acid as the mobile phase. Using a flow rate of 4ml/min and UV detection at 300nm, the fractions containing the single enantiomer were combined and evaporated *in vacuo* to afford the product (0.23g, 55%) as a colourless solid with > 99.5% ee. mp 180°C (dec.). $^1$H NMR (360MHz, $D_6$-DMSO) δ 0.85-0.98 (1H, m), 1.08-1.39 (3H, m), 1.41-1.66 (4H, m), 1.75-1.82 (1H, m), 1.87-1.93 (1H, m), 2.90-2.98 (1H, m), 3.32 (3H, s), 5.06 (1H, d, J = 8.3Hz), 7.29-7.35 (2H, m), 7.37-7.41 (2H, m),

7.49-7.56 (2H, m), 7.60-7.64 (3H, m), 7.68-7.76 (2H, m), 7.84 (1H, s), 7.96-7.99 (2H, m), 9.17 (1H, s), 12.50 (1H, brs). $[\alpha]^{25}_{D}$ -7.9° (c = 0.61, $CH_3OH$).

EXAMPLE 28 N-[3(*R,S*)-5-Cyclohexyl-2,3-dihydro-1-methyl-2-oxo-1H-1,4-benzodiazepin-3-yl] N'-[3-(2-methyltetrazol-5-yl)phenyl]urea

Step 1: 1-Methyl-5-(3-nitrophenyl)tetrazole and 2-methyl-5-(3-nitrophenyl)tetrazole:

Sodium hydroxide (1.22g, 0.030mol) in water (20ml) was added to a stirred solution of 5-(3-nitrophenyl)tetrazole (5.28g, 0.028mol) in ethanol (60ml). Iodomethane (1.9ml, 0.030mol) was added and the reaction mixture was stirred at room temperature for 7h. Further iodomethane (1.9ml, 0.030mol) was added and the reaction mixture was stirred for a further 18 h. The mixture was evaporated to dryness and the residue partitioned between ethyl acetate (100ml) and water (50ml). The organic layer was separated and the aqueous re-extracted with ethyl acetate (2 x 100ml). The combined organic layers were dried ($Na_2SO_4$) then evaporated to give a brown solid which was purified by column chromatography on silica gel using dichloromethane:methanol (10:1) to first afford 2-methyl-5-(3-nitrophenyl)tetrazole (3.85g, 67%) as a cream solid. mp 105°C. $R_f$ 0,78 in dichloromethane:diethyl ether (5:1) on silica plates. $^1H$ NMR (360MHz, $CDCl_3$) δ 4.45 (3H, s), 7.70 (1H, dd, J = 8 and 8Hz), 8.33 (1H, ddd, J = 8, 2 and 2Hz), 8.49 (1H, brd, J = 8Hz), 8.99 (1H, dd, J = 2 and 2Hz). Found: C, 47.12; H, 3.49; N, 34.05. $C_8H_7N_5O_2$ requires C, 46.83; H, 3.44; N, 34.13%.

The second product to elute was 1-methyl-5-(3-nitrophenyl)tetrazole (345mg, 6%) as a cream solid, mp 143-144°C. $R_f$ 0.60 in dichloromethane:diethyl ether (5:1) on silica plates. $^1H$ NMR (360MHz, $CDCl_3$) δ 4.28 (3H, s), 7.82 (1H, dd, J = 8 and 8Hz), 8.18 (1H, ddd, J = 8, 2 and 2Hz), 8.47 (1H, brd, J = 8Hz), 8.64 (1H, dd, J = 2 and 2Hz). Found: C, 46.96; H, 3.40; N, 34.00. $C_8H_7N_5O_2$ requires C, 46.83; H, 3.44; N, 34.13%.

Step 2: 5-(3-Aminophenyl)-2-methyltetrazole:

2-Methyl-5-(3-nitrophenyl)tetrazole (2.30g, 0.0112mol) was hydrogenated at 20 psi in ethanol (50ml) using 10% palladium on carbon (230mg) for 15 min. The mixture was filtered then evaporated to dryness *in vacuo* to give the title compound (1.55g, 79%) as a colourless solid. mp 97°C. $R_f$ 0.40 in dichloromethane:methanol (5:1) on silica plates. $^1H$ NMR (360MHz, $CDCl_3$) δ 3.80 (2H, brs), 4.38 (3H, s), 6.78 (1H, ddd, J = 8, 2 and 2Hz), 7.26 (1H, dd, J = 8 and 8Hz), 7.47 (1H, dd, J = 2 and 2Hz), 7.51 (1H, dd, J = 8 and 2Hz).

Step 3: N-[3(*R,S*)-5-Cyclohexyl-2,3-dihydro- 1-methyl-2-oxo-1H-1,4-benzodiazepin-3-yl] N'-[3-(2-methyltetrazol-5-yl)phenyl]urea:

To a stirred solution of 5-cyclohexyl-1,3-dihydro-1-methyl-3(*R,S*)[(4-nitrophenyloxycarbonyl)amino]-2H-1,4-benzodiazepin-2-one (150mg, 0.34mmol) [Example 11, Step 3], in anhydrous dimethylformamide (3ml) was added triethylamine (48μl, 0.34mmol). After 5 min a solution of 5-(3-aminophenyl)-2-methyltetrazole (65mg, 0.37mmol) in anhydrous dimethylformamide (3ml) was added, and the solution heated at 50°C for 2h. After this time the solution was cooled to ambient temperature and evaporated *in vacuo*. The residue was dissolved in ethyl acetate (5ml) and on standing a solid precipitated. This was recrystallised from ethyl acetate to afford the urea (65mg, 40%) as a colourless solid. mp 195-196°C. $^1H$ NMR (360MHz, $CDCl_3$) δ 1.05-2.06 (10H, m), 2.79 (1H, m), 3.45 (3H, s), 4.36 (3H, s), 5.42 (1H, d, J = 8Hz), 6.75 (1H, d, J = 8Hz), 7.15-7.60 (7H, m), 7.77 (1H, d, J = 8Hz), 8.06 (1H, s).

EXAMPLE 29 N-(3(*R,S*)-5-Cyclopentyl-2,3-dihydro-1-ethyl-2-oxo-1H-1,4-benzodiazepin-3-yl] N'-(3-(methylsulphonylaminocarbonyl)phenyl] urea

The title compound was prepared from 5-cyclopentyl-1,3-dihydro-1-ethyl-3(*R,S*)-[(4-nitrophenyloxycarbonyl)amino]-2H-1,4-benzodiazepin-2-one and 1-(methylsulphonylaminocarbonyl) 3-aminobenzene [Example 16, Step 2] using the procedure described in Example 11, Step 4, to afford the product as a colourless powder. mp 210-212°C, $^1H$ NMR (360MHz, D6-DMSO) δ 0.93 (3H, t, J = 7Hz), 1.00-1.20 (1H, m), 1.40-1.90 (6H, m), 2.00-2.20 (1H, m), 3.34 (3H, s), 3.40-3.60 (1H, m), 3.62-3.80 (1H, m), 4.20-4.40 (1H, m), 5.00-5.10 (1H, m), 7.30-7.85 (8H, m), 7.93 (1H, s), 9.21 (1H, s), 12.06 (1H, brs).

EXAMPLE 30 N-[3(*R,S*)-5-Cyclopentyl-2,3-dihydro-1-ethyl-2-oxo-1H-1,4-benzodiazepin-3-yl] N'-[3-(isopropylsulphonylaminocarbonyl)phenyl] urea

The title compound was prepared from 5-cyclopentyl-1,3-dihydro- 1-ethyl-3(*R,S*)-[(4-nitrophenyloxycarbonyl)amino]-2H-1,4-benzodiazepin-2-one and 1-(isopropylsulphonylaminocarbonyl)-3-aminobenzene [Example 12, Step 3] using the procedure described in Example 11, Step 4, to afford the product as a colourless powder. mp 160-162°C. $^1$H NMR (360MHz, D6-DMSO) δ 0.92 (3H, t, J = 8Hz), 1.00-1.20 (1H, m), 1.30 (6H, d, J = 7Hz), 1.40-1.80 (6H, m), 2.00-2.20 (1H, m), 3.40-3.60 (1H, m), 3.62-3.85 (2H, m), 4.10-4.30 (1H, m), 5.05 (1H, d, J = 7Hz), 7.30-7.80 (8H, m), 7.92 (1H, s), 9.20 (1H, s), 11.91 (1H, brs).

EXAMPLE 31 N-[3(*R,S*)-5-Cyclohexyl-2,3-dihydro-1-methyl-2-oxo-1H-1,4-benzodiazepin-3-yl] N'-[3-(1,1- dimethylethylsulphonylaminocarbonyl)phenyl] urea

Step 1: 1,1-Dimethylethylsulphinic acid:

Sulphur dioxide gas was bubbled through a solution of 1,1-dimethylethylmagnesium chloride (0.4mol) in diethyl ether (400ml) for 2h, maintaining the internal temperature at 5°C. The mixture was then poured into cold (5°C) saturated ammonium chloride solution (200ml) and stirred vigorously at this temperature for 15 min. The mixture was filtered and the ethereal layer separated and dried ($MgSO_4$). The solvent was removed *in vacuo* to afford the title compound (15.7g, 32%) as a colourless solid. $^1$H NMR (360MHz, $CDCl_3$) δ 1.21 (9H, s). MS (CI, $NH_3$) 121 (M-1).

Step 2: 1,1-Dimethylethylsulphinyl chloride:

Thionyl chloride (18.8ml, 0.26mol) was added dropwise to 1,1-dimethylethylsulphinic acid (15.7g, 0.13mol) over a period of 20 min, at ambient temperature, under an atmosphere of nitrogen. The solution was stirred for a further 2h then the excess thionyl chloride was removed *in vacuo* and the residue azeotroped with diethyl ether (2 x 200ml). The residue was distilled under reduced pressure to afford the title compound (12.92g, 72%) as a straw yellow oil. b.p. 75°C at 6mmHg. $^1$H NMR (360MHz, $CDCl_3$) δ 1.41 (9H, s).

Step 3: 1,1-Dimethylethylsulphinamide:

To a suspension of 1,1-dimethylethylsulphinyl chloride (2.5g, 17.7mmol) in water (10ml) was added aqueous ammonia solution (30%, 100ml) dropwise. This mixture was heated to 80°C for 10 min, cooled and the solvent evaporated *in vacuo*. The residue was azeotroped with toluene (2 x 25ml), then triturated with diethyl ether (100ml), and the resultant precipitate removed by filtration. The filtrate was evaporated *in vacuo* and the residue was chromatographed on silica gel using a gradient elution of 50% to 100% of ethyl acetate in petrol followed by 10% methanol in ethyl acetate, to afford the title compound (1.42g, 65%) as a colourless solid. mp 107-109°C. $^1$H NMR (360MHz, $CDCl_3$) δ 1.23 (9H, s), 3.67 (2H, brs).

Step 4: 1,1-Dimethylethylsulphonamide:

To a refluxing solution of 1,1-dimethylethylsulphinamide (0.5g, 4.1mmol) in anhydrous acetone (25ml) was added dropwise a saturated solution of potassium permanganate in acetone (40ml) over a period of 1h, until a purple colour persisted. The mixture was refluxed for a further 30 min and the precipitate was removed by hot filtration. The precipitate was washed well with further acetone and the filtrate evaporated *in vacuo*. The resultant yellow solid was triturated with petrol and the precipitate was collected by filtration to give the title compound (0.32g, 57%) as a colourless solid. mp 160-162°C. $^1$H NMR (360MHz, D6-DMSO) δ 1.27 (9H, s), 6.58 (2H, brs).

Step 5: 1-(1,1-Dimethylethylsulphonylaminocarbonyl)-3-nitrobenzene:

To a mixture of 3-nitrobenzoic acid (0.37g, 2.2mmol), 1,1-dimethylethylsulphonamide (0.3g, 2.2mmol) and 4-dimethylaminopyridine (0.27g, 2.2mmol) in anhydrous dichloromethane (20ml), under an atmosphere of nitrogen, was added 1-[3-(dimethylamino)propyl]-3-ethyl carbodiimide hydrochloride (0.42g, 2.2mmol). The mixture was stirred at ambient temperature for 22h. The mixture was extracted with 1M NaOH (50ml) and the separated aqueous phase acidified using 5M HCl. This was extracted with dichloromethane (5 x 20ml), then the combined organic extracts were dried ($Na_2SO_4$) and evaporated to dryness. The residue was chromatographed

on silica gel using 1% methanol in dichloromethane as the eluant. The title compound (0.53g, 85%) was isolated as a colourless solid. mp 220°C. $^1$H NMR (360MHz, D6-DMSO) δ 1.27 (9H, s), 6.59 (1H, brs), 7.65 (1H, t, J = 7.9Hz), 8.24-8.30 (1H, m), 8.34-8.38 (1H, m), 8.68-8.72 (1H, m).

Step 6: 1-(1,1-Dimethylethylsulphonylaminocarbonyl)-3-aminobenzene:

In the same way as that described in Example 11, Step 2, using 1-(1,1-dimethylethylsulphonylaminocarbonyl)-3-nitrobenzene (0.36g, 1.26mmol), 10% palladium on carbon (0.1g, 28% (w/w)) in water (2ml) and ethanol (25ml), the title compound (0.32g, 99%) was afforded as a colourless solid. mp 150°C (dec.). $^1$H NMR (360MHz, D6-DMSO) δ 1.30 (9H, s), 4.99 (2H, brs), 6.56-6.63 (1H, m), 6.97 (1H, t, J = 7.7Hz), 7.13-7.25 (2H, m).

Step 7: N-[3(*R,S*)-5-Cyclohexyl-2,3-dihydro-1-methyl-2-oxo-1H-1,4-benzodiazepin-3-yl] N'-[3-(1,1-dimethylethylsulphonylaminocarbonyl)phenyl] urea:

The title compound was prepared in the same way as that described in Example 11, Step 4, using 5-cyclohexyl-1,3-dihydro-1-methyl-3(*R,S*)-[(4-nitrophenyloxycarbonyl)amino]-2H-1,4-benzodiazepin-2-one (0.3g, 0.69mmol), triethylamine (96μl, 0.69mmol), dimethylformamide (6ml) and 1-(1,1-dimethylethylsulphonylaminocarbonyl)-3-aminobenzene (0.19g, 0.76mmol). The product (176mg, 46%) was afforded as a colourless solid after recrystallisation from ethanol. mp 180°C (dec.). $^1$H NMR (360MHz, D6-DMSO) δ 0.84-0.98 (1H, m), 1.38 (9H, s), 1.08-1.65 (7H, m), 1.71-1.81 (1H, m), 1.86-1.94 (1H, m), 2.88-2.98 (1H, m), 3.32 (3H, s), 5.08 (1H, d, J = 8.3Hz), 7.29-7.42 (4H, m), 7.54 (1H, s), 7.56 (1H, s), 7.64 (1H, t, J = 7.7Hz), 7.75 (1H, d, J = 7.9Hz), 7.84 (1H, s), 9.19 (1H, s), 11.51 (1H, brs).

EXAMPLE 32 N-(3(*R,S*)-5-Cyclohexyl-2,3-dihydro-1-methyl-2-oxo-1H-1,4-benzodiazepin-3-yl] N'-(3-(1,1-dimethylethylcarbonylaminosulphonyl)phenyl] urea

Step 1: 1-(1,1-Dimethylethylcarbonylaminosulphonyl)-3-nitrobenzene:

To a mixture of trimethylacetic acid (5.55g, 54mmol), 3-nitrobenzenesulphonamide (11g, 54mmol) and 4-dimethylaminopyridine (6.65g, 54mmol) in anhydrous dichloromethane (400ml), under an atmosphere of nitrogen, was added 1-[3-(dimethylamino)propyl]-3-ethyl carbodiimide hydrochloride (10.43g, 54mmol). The mixture was stirred at ambient temperature for 24h. The mixture was extracted with 1M NaOH (50ml) and the separated aqueous phase was mixed with dichloromethane (200ml) and acidified with 5M HCl. The two layers were separated and the aqueous phase was further extracted with dichloromethane (2 x 100ml). The combined organic phases were dried ($Na_2SO_4$) and evaporated to dryness. The resultant solid was recrystallised from methanol to afford the title compound (9g, 58%) as a colourless solid. mp 178-181°C. $^1$H NMR (360MHz, D6-DMSO) δ 1.07 (9H, s), 7.95 (1H, t, J = 8.0Hz), 8.30-8.34 (1H, m), 8.52-8.56 (1H, m), 8.57-8.60 (1H, m), 12.02 (1H, brs).

Step 2: 1-(1,1-Dimethylethylcarbonylaminosulphonyl)-3-aminobenzene:

In the same way as that described in Example 11, Step 2, using 1-(1,1-dimethylethylcarbonylaminosulphonyl)-3-nitrobenzene (5g, 17.5mmol), 10% palladium on carbon (0.5g, 10% (w/w)) in water (4ml) and ethanol (100ml), the title compound (3.36g, 75%) was afforded as a pale yellow solid after recrystallisation from ethanol. mp 147- 150°C. $^1$H NMR (360MHz, $D_6$-DMSO) δ 1.07 (9H, s), 5.62 (2H, brs), 6.78 (1H, dd, J = 8.0 and 2.0Hz), 6.94 (1H, d, J = 8.0Hz), 7.08 (1H, t, J = 2.0Hz), 7.19 (1H, t, J = 7.9Hz), 11.47 (1H, brs).

Step 3: N-[3(*R,S*)-5-Cyclohexyl-2,3-dihydro-1-methyl-2-oxo-1H-1,4-benzodiazepin-3-yl] N'-[3-(1,1-dimethylethylcarbonylaminosulphonyl)phenyl] urea:

The title compound was prepared in the same way as that described in Example 11, Step 4, using 5-cyclohexyl-1,3-dihydro-1-methyl-3(*R,S*)-[(4-nitrophenyloxycarbonyl)amino]-2H-1,4-benzodiazepin-2-one (0.3g, 0.69mmol), triethylamine (96μl, 0.69mmol), dimethylformamide (6ml) and 1-(1,1-dimethylethylcarbonylaminosulphonyl)-3-aminobenzene (194mg, 0.76mmol). After trituration with hot methanol, the title compound (0.15g, 39%) was afforded as a colourless solid. mp 235°C (dec.). $^1$H NMR (360MHz, D6-DMSO) δ 0.84-0.98 (1H, m), 1.04 (9H, s), 1.06-1.40 (4H, m), 1.41-1.68 (3H, m), 1.73-1.82 (1H, m), 1.88-1.96 (1H, m), 2.89-3.00 (1H, m), 3.33 (3H, s), 5.06 (1H, d, J = 8.3Hz), 7.32-7.50 (4H, m), 7.52-7.58 (2H, m), 7.64 (1H, t, J = 7.0Hz), 7.75 (1H,

d, J = 7.9Hz), 8.03 (1H, s), 9.39 (1H, s), 11.62 (1H, brs).

EXAMPLE 33 N-[3(*R*)-5-Cyclohexyl-2,3-dihydro-1-methyl-2-oxo-1H-1,4-benzodiazepin-3-yl] N′-[3-(2-methylphenylsulphonylaminocarbonyl)phenyl] urea

Step 1: 1-(2-Methylphenylsulphonylaminocarbonyl)-3-nitrobenzene:

To a mixture of 3-nitrobenzoic acid (5g, 30mmol), 2-methylphenylsulphonamide (5.13g, 30mmol) and 4-dimethylaminopyridine (3.65g, 30mmol) in anhydrous dichloromethane (200ml), under an atmosphere of nitrogen, was added 1-[3-(dimethylamino)propyl]-3-ethyl carbodiimide hydrochloride (5.74g, 30mmol). The mixture was stirred at ambient temperature for 4h. The resultant precipitate was collected by filtration and partitioned between dichloromethane (1 x 500ml, 2 x 100ml) and 1M HCl (250ml). The combined organic phases were dried ($MgSO_4$) and evaporated to dryness to afford the title compound (6.4g, 67%) as a colourless solid. mp 197-199°C. $^1$H NMR (360MHz, D6-DMSO) δ 2.63 (3H, s), 7.40-7.50 (2H, m), 7.61 (1H, td, J = 10.7 and 2.1Hz), 7.79 (1H, t, J = 11.4Hz), 8.07 (1H, dd, J = 11.4 and 2.0Hz), 8.27-8.31 (1H, m), 8.44-8.48 (1H, m), 8.74-8.78 (1H, m).

Step 2: 1-(2-Methylphenylsulphonylaminocarbonyl)-3-aminobenzene:

In the same way as that described in Example 11, Step 2, using 1-(2-methylphenylsulphonylaminocarbonyl)-3-nitrobenzene (3g, 9.4mmol), 10% palladium on carbon (0.3g, 10% (w/w)) in water (2ml) and ethanol (60ml), the title compound was afforded as a yellow solid. This was recrystallised from ethanol to give a pale yellow crystalline solid (2.21g, 81%). mp 150-152°C. $^1$H NMR (360MHz, D6-DMSO) δ 2.61(3H, s), 6.78 (1H, d, J = 7.8Hz), 6.97-7.03 (2H, m), 7.12 (1H, t, J = 7.8Hz), 7.39-7.47 (2H, m), 7.58 (1H, t, J = 7.5Hz), 8.02 (1H, d, J = 7.9Hz).

Step 3: (+)-3(*R*)-Amino-5-cyclohexyl-1,3-dihydro-1-methyl-2H-1,4-benzodiazepin-2-one:

N-[1(*R*)-2-[(3(*R*)-5-Cyclohexyl-2,3-dihydro- 1-methyl-2-oxo-1H-1,4-benzodiazepin-3-yl)amino]-2-oxo-1-(phenylmethyl )ethyl] N′-phenyl thiourea (4.5g, 8. 1mmol) [Example 27, Step 4] was dissolved in trifluoroacetic acid (25ml) and stirred at ambient temperature for 30 min. The trifluoroacetic acid was removed under reduced pressure and the residue azeotroped with dichloromethane (2 x 20ml) and toluene (2 x 20ml). The residue was chromatographed on silica gel using 90:10:0.1:0.1, dichloromethane:methanol:acetic acid:water as the eluant, to afford an orange gum. This was dissolved in ethyl acetate (150ml), cooled to 0°C, and treated with 10% sodium carbonate solution (15ml). After diluting with water (25ml) and stirring for 1 min, the organic layer was separated and the aqueous re-extracted with ethyl acetate (2 x 50ml). The combined organics were dried ($Na_2SO_4$) and evaporated *in vacuo* to afford the title compound (1.56g, 71%) as a pink solid with 99% e.e. mp 133-136°C. $^1$H NMR (360MHz, $CDCl_3$) δ 1.01-1.39 (4H, m), 1.50-1.54 (1H, m), 1.60-1.70 (3H, m), 1.84-1.92 (1H, m), 1.96-2.04 (1H, m), 2.36 (2H, brs), 2.70-2.80 (1H, m), 3.41 (3H, s), 4.32 (1H, s), 7.22-7.28 (2H, m), 7.46-7.58 (2H, m). $[\alpha]_D$ +33.2° (c = 0.66, $CH_3OH$).

Step 4: N-[3(*R*)-5-Cyclohexyl-2,3-dihydro-1-methyl-2-oxo-1H-1,4-benzodiazepin-3-yl] N′-[3-(2-methylphenylsulphonylaminocarbonyl)phenyl] urea:

1-(2-Methylphenylsulphonylaminocarbonyl )phenyl]-3-aminobenzene (234mg, 0.81mmol) was dissolved in anhydrous tetrahydrofuran (20ml) and cooled to 0°C under an atmosphere of nitrogen. Triphosgene (0.08g, 0.27mmol) was added in one portion and the mixture was stirred for 2 min. The mixture was then treated with triethylamine (0.34ml, 2.4mmol) in portions of 98, 98, 49, 49 and 49μl over a period of 5 min. The mixture was allowed to warm to 12°C over a period of 10 min and was then re-cooled to 0°C. 3(*R*)-Amino-5-cyclohexyl-1,3-dihydro-1-methyl-2H-1,4-benzodiazepin-2-one (0.15g, 0.55mmol) was dissolved in anhydrous tetrahydrofuran (5ml) and added to the reaction dropwise. The mixture was stirred at 0°C for 5 min and then stirred at ambient temperature for 15 min. The precipitated solid was removed by filtration and washed with tetrahydrofuran. The filtrate was evaporated and partitioned between ethyl acetate (150ml) and 10% citric acid solution (30ml), brine (30ml) and then dried ($Na_2SO_4$). The solvent was evaporated *in vacuo* and the residue chromatographed on silica gel using a gradient elution of 5-10% methanol in dichloromethane. The resultant solid was recrystallised from ethanol to afford the title compound (0.17g, 52%) as a colourless solid with > 99% e.e. mp 175°C (dec.). $^1$H NMR (360MHz, D6-DMSO) δ 0.84-0.97 (1H, m), 1.06-1.66 (7H, m), 1.72-1.82 (1H, m), 1.86-1.96 (1H, m), 2.59 (3H, s), 2.88-2.98 (1H, m), 3.32 (3H, s), 5.06 (1H, d, J = 8.3Hz), 7.28-7.46 (6H, m), 7.50-7.57 (3H, m),

7.63 (1H, t, J = 8.4Hz), 7.75 (1H, d, J = 6.5Hz), 7.81 (1H, s), 7.99 (1H, d, J = 7.7Hz), 9.15 (1H, s), 12.60 (1H, brs). $[\alpha]_D$ -9.3° (c = 0.57, $CH_3OH$).

EXAMPLE 34 N-[3(*R*)-5-Cyclohexyl-2,3-dihydro-1-methyl-2-oxo-1H-1,4-benzodiazepin-3-yl] N'-[4-methyl-3-(methylsulphonylaminocarbonyl)phenyl] urea

Step 1: 2-Methyl-1-(methylsulphonylaminocarbonyl)-5-nitrobenzene:

The title compound was prepared in the same way as that described in Example 11, Step 1, using 2-methyl-5-nitrobenzoic acid (5g, 27.6mmol), methyl sulphonamide (2.63g, 27.6mmol), 4-dimethylaminopyridine (3.37g, 27.6mmol), 1-[3-(dimethylamino)propyl]-3-ethyl carbodiimide hydrochloride (5.29g, 27.6mmol) and anhydrous dichloromethane (200ml). The title compound (5.67g, 79%) was afforded as a colourless solid. mp 180°C (dec.). $^1$H NMR (360MHz, D6-DMSO) δ 2.50 (3H, s), 3.42 (3H, s), 7.61 (1H, d, J = 8.5Hz), 8.28 (1H, dd, J = 8.4 and 2.5Hz), 8.36 (1H, d, J = 2.5Hz), 12.46 (1H, brs).

Step 2: 2-Methyl-1-(methylsulphonylaminocarbonyl)-5-aminobenzene:

In the same way as that described in Example 11, Step 2, using 2-methyl-1-(methylsulphonylaminocarbonyl)-5-nitrobenzene (3g, 11.6mmol), 10% palladium on carbon (0.3g, 10% (w/w)) in water (2ml) and ethanol (100ml), the title compound (2.1g, 79%) was afforded as a pale yellow crystalline solid. mp 174-176°C. $^1$H NMR (360MHz, D6-DMSO) δ 2.17 (3H, s), 3.32 (3H, s), 6.61 (1H, dd, J = 8.1 and 2.5Hz), 6.67 (1H, d, J = 2.4Hz), 6.92 (1H, d, J = 8.2Hz).

Step 3: N-(3(*R*)-5-Cyclohexyl-2,3-dihydro-1-methyl-2-oxo-1H-1,4-benzodiazepin-3-yl] N'-[4-methyl-3-(methylsulphonylaminocarbonyl)phenyl] urea:

The title compound was prepared in the same way as that described in Example 33, Step 4, using 2-methyl-1-(methylsulphonylaminocarbonyl)-5-aminobenzene (185mg, 0.81mmol), triphosgene (80mg, 0.27mmol), triethylamine(0.34ml, 2.42mmol), 3(*R*)-amino-5-cyclohexyl-1,3-dihydro-1-methyl-2H-1,4-benzodiazepin-2-one (0.15g, 0.56mmol) and tetrahydrofuran (25ml). After recrystallisation from methanol, the title compound (135mg, 46%) was afforded as a colourless solid with > 99% e.e. mp 175°C (dec.). $^1$H NMR (360MHz, D6-DMSO) δ 1.00-1.41 (4H, m), 1.51-1.71 (4H, m), 1.80-1.86 (1H, m), 1.96-2.04 (1H, m), 2.35 (3H, s), 2.79-2.88 (1H, m), 3.31 (3H, s), 3.40 (3H, s), 5.26 (1H, d, J = 7.5Hz), 7.08 (1H, d, J = 8.3Hz), 7.30-7.42 (4H, m), 7.54-7.65 (3H, m), 8.94 (1H, s), 11.86 (1H, s). $[\alpha]_D$ -7.7° (c = 0.44, $CH_3OH$).

EXAMPLE 35 N-[3(*S*)-5-Cyclohexyl-2,3-dihydro-1-methyl-2-oxo-1H-1,4-benzodiazepin-3-yl] N'-[3-(phenylsulphonylaminocarbonyl)phenyl] urea

Step 1: (-)-3(*S*)-(2-(*R*)-Amino-3-phenylpropionylamino)-5-cyclohexyl-1,3-dihydro-1-methyl-2H-1,4-benzodiazepin-2-one:

3(*R,S*)-[2(*R*)-(tert-Butyloxycarbonyl)amino-3-phenylpropionylamino]-5-cyclohexyl-1,3-dihydro-1-methyl-2 H-1,4-benzodiazepin-2-one (12.7g, 24.5mmol) [Example 27, Step 2] was dissolved in ethyl acetate (20ml) and cooled to 0°C. This solution was then saturated with hydrogen chloride gas. After 1.5h the resulting precipitate was collected by filtration. After recrystallisation twice from ethanol, the precipitate was partitioned between ethyl acetate (50ml) and 10% sodium carbonate solution (50ml). The organic phase was separated and the aqueous extracted with further ethyl acetate (2 x 50ml). The combined organic phases were dried ($Na_2SO_4$) and evaporated *in vacuo* to afford the title compound (2g, 20%) as a pale yellow solid. mp 75-78°C. $^1$H NMR (360MHz, $CDCl_3$) δ 1.03-1.41 (4H, m), 1.48-1.72 (4H, m), 1.82-1.92 (1H, m), 1.97-2.06 (1H, m), 2.70-2.84 (2H, m), 3.28-3.42 (4H, m), 3.76-3.82 (1H, m), 5.36 (1H, d, J = 8.2Hz), 7.21-7.33 (7H, m), 7.50 (1H, t, J = 8.4Hz), 7.56 (1H, d, J = 8.1Hz), 8.67-8.72 (1H, m). $[\alpha]_D$ -13.5° (c=0.63, $CH_3OH$).

Step 2: N-[1(*R*)-2[(3(*S*)-5-Cyclohexyl-2,3-dihydro-1-methyl-2-oxo-1H-1,4-benzodiazepin-3-yl)amino]-2-oxo-1-(phenylmethyl)ethyl] N'-phenyl thiourea:

In the same way as that described in Example 27, Step 4, using (-)-3*(S)-(2(R)*-amino-3-phenylpropionylamino)-5-cyclohexyl-1,3-dihydro-1-methyl-2H-1,4-benzodiazepin-2-one (1.5g, 3.6mmol), phenyl isothiocyanate (0.47ml, 4.0mmol) and anhydrous dichloromethane (10ml), the title compound (1.95g, 98%) was afforded

as a colourless solid. mp 134-137°C. $^1$H NMR (360MHz, $CDCl_3$) δ 1.00-1.12 (1H, m), 1.16-1.44 (3H, m), 1.48-1.78 (4H, m), 1.84-1.96 (1H, m), 2.00-2.08 (1H, m), 2.72-2.82 (1H, m), 3.29-3.44 (2H, m), 3.37 (3H, s), 5.26 (1H, d, J = 7.6Hz), 5.28-5.36 (1H, m), 6.79 (1H, d, J = 7.8Hz), 6.98-7.04 (2H, m), 7.15-7.42 (9H, m), 7.43-7.56 (2H, m), 7.62 (1H, s). $[\alpha]_D^{23}$ -15.7° (c-=0.70, $CH_2Cl_2$).

Step 3: 3(*S*)-Amino-5-cyclohexyl-1,3-dihydro-1-methyl-2H-1,4-benzodiazepin-2-one:

In the same way as that described in Example 33, Step 3, using N-[1(*R*)-2-[(3(*S*)-5-cyclohexyl-2,3-dihydro-1-methyl-2-oxo-1H-1,4-benzodiazepin-3-yl)amino]-2-oxo-1-(phenylmethyl)ethyl] N'-phenyl thiourea (1.8g, 3.3mmol) and trifluoroacetic acid (10ml), the title compound (0.8g, 91%) was afforded as a pink solid with 98.4° e.e. mp 130-133°C. $^1$H NMR (360MHz, $CDCl_3$) δ 1.01-1.39 (4H, m), 1.50-1.54 (1H, m), 1.60-1.72 (3H, m), 1.84-1.96 (1H, m), 1.97-2.02 (1H, m), 2.70 (2H, brs), 2.70-2.79 (1H, m), 3.39 (3H, s), 4.33 (1H, s), 7.22-7.28 (2H, m), 7.46-7.54 (2H, m). $[\alpha]_D$ -33.2° (c = 0.70, $CH_3OH$).

Step 4: N-[3(*S*)-5-Cyclohexyl-2,3-dihydro-1-methyl-2-oxo-1H-1,4-benzodiazepin-3-yl] N'-[3-, (phenylsulphonylaminocarbonyl)phenyl] urea:

The title compound was prepared in the same way as that described in Example 33, Step 4, using 1-(phenylsulphonylaminocarbonyl)-3-aminobenzene (0.30g, 1.08mmol) [Example 14, Step 2]), triphosgene (107mg, 0.36mmol), triethylamine (0.45ml, 3.23mmol), 3(*S*)-amino-5-cyclohexyl-1,3-dihydro-1-methyl-2H-1,4-benzodiazepin-2-one (0.2g, 0.74mmol) and tetrahydrofuran (30ml). After trituration with diethyl ether, the title compound (248mg, 59%) was afforded as a colourless solid with > 99% e.e. mp 180°C (dec.). $^1$H NMR (360MHz, D6-DMSO) δ 0.82-0.98 (1H, m), 1.08-1.65 (7H, m), 1.75-1.83 (1H, m), 1.88-1.96 (1H, m), 2.90-3.00 (1H, m), 3.31 (3H, s), 5.06 (1H, d, J = 8.3Hz), 7.30-7.36 (2H, m), 7.37-7.41 (2H, m), 7.49-7.56 (2H, m), 7.60-7.65 (3H, m), 7.68-7.76 (2H, m), 7.84 (1H, s), 7.96-7.99 (2H, m), 9.17 (1H, s), 12.46 (1H, brs). $[\alpha]_D$ +9.4° (c = 0.37, $CH_3OH$).

EXAMPLE 36 N-[3(*R,S*)-5-Cyclopentyl-2,3-dihydro-1-propyl-2-oxo-1H-1,4-benzodiazepin-3-yl] N'-[3-(isopropylsulphonylaminocarbonyl)phenyl] urea

Step 1: 5-Cyclopentyl-1,3-dihydro-1-propyl-3(*R,S*)-[(benzyloxycarbonyl)amino]-2H-1,4-benzodiazepin-2-one:

To a solution of 5-cyclopentyl-1,3-dihydro-3(*R,S*)-[(benzyloxycarbonyl)amino]-2H-1,4-benzodiazepin-2-one [Example 5, Step 3] (1.48g, 3.93mmol) in anhydrous dimethylformamide (40ml), cooled in an ice bath, was added sodium hydride (60% dispersion in oil, 173mg, 4.33mmol) and the resulting mixture stirred under nitrogen for 1h. Iodopropane (0.46ml, 4.72mmol) was then added and the mixture stirred at room temperature for 3h. The solvent was evaporated and the residue partitioned between dichloromethane (100ml) and water (100ml). The aqueous layer was re-extracted with more dichloromethane (100ml), the organic extracts were combined, dried ($MgSO_4$) and evaporated. The residue was then triturated with ether to afford the title compound (1.36g, 83%) as a white solid. mp 138-140°C. $^1$H NMR (360MHz, $CDCl_3$) δ 0.80 (3H, t, J = 7.4Hz), 1.26-1.79 (8H, m), 1.94 (1H, m), 2.12 (1H, m), 3.30 (1H, m), 3.55 (1H, m), 4.29 (1H, m), 5.10 (2H, m), 5.13 (1H, d), 6.52 (1H, d, J = 8.1Hz), 7.23-7.34 (5H, m), 7.48 (1H, t of d, J = 8.0 and 1.4Hz), 7.56 (1H, dd, J = 7.8 and 1.4Hz).

Step 2: 3(*R,S*)-Amino-5-cyclopentyl-1,3-dihydro-1-propyl-2H-1,4-benzodiazepin-2-one:

To 5-cyclopentyl-1,3-dihydro-1-propyl-3(*R,S*)-[(benzyloxycarbonyl)amino]-2H-1,4-benzodiazepin-2-one (507mg, 1.21mmol) was added 45% hydrobromic acid in acetic acid (1ml) and the mixture stirred at room temperature for 2h. Anhydrous ether (10ml) was then added and the mixture was stirred for 1.5h before removing the solvent by pipette. The resulting solid was washed with more ether, filtered off and washed again with ether, before partitioning between dichloromethane (30ml ) and 2N sodium hydroxide solution (30ml). The aqueous layer was re-extracted with more dichloromethane (2 x 30ml), the organic extracts combined, dried ($Na_2SO_4$) and evaporated to leave the title compound (333mg, 96%) as a colourless oil. $^1$H NMR (360MHz, $CDCl_3$) δ 0.81 (3H, t, J = 7.4Hz), 1.25-1.94 (9H, m), 2.16 (1H, m), 3.29 (1H, m), 3.56 (1H, m), 4.28 (1H, s), 4.30 (1H, m), 7.24 (1H, t, J = 7.3Hz), 7.31 (1H, d, J = 7.9Hz), 7.46 (1H, t of d, J = 7.8 and 1.5Hz), 7.53 (1H, dd, J = 7.8 and 1.4Hz).

Step 3: 5-Cyclopentyl-1,3-dihydro-1-propyl-3(*R,S*)-[(4-nitrophenyloxycarbonyl)amino]-2H-1,4-benzodiazepin-2-one:

The title compound was prepared from 3(*R,S*)-amino-5-cyclopentyl-1,3-dihydro-1-propyl-2H-1,4-benzodiazepin-2-one (321mg, 11.2mmol) and 4-nitrophenyl chloroformate (229mg, 11.4mmol) using the procedure described in Example 20, Step 1. The crude product was triturated with ether (10ml) to give the title compound (396mg, 78%) as a white solid. $^1H$ NMR (360MHz, $CDCl_3$) 0.83 (3H, t, J = 7.4Hz), 1.33-1.82 (8H, m), 1.97 (1H, m), 2.15 (1H, m), 3.34 (1H, m), 3.60 (1H, m), 4.32 (1H, m), 5.16 (1H, d, J = 8.3Hz), 6.89 (1H, d, J = 8.4Hz), 7.30-7.37 (4H, m), 7.52 (1H, t of d, J = 7.8 and 1.6Hz), 7.59 (1H, dd, J = 7.8 and 1.5Hz), 8.22 (2H, d, J = 9.2Hz).

Step 4: N-[3(*R,S*)-5-Cyclopentyl-2,3-dihydro-1-propyl-2-oxo-1H-1,4-benzodiazepin-3-yl] N′-(3-(isopropylsulphonylaminocarbonyl)phenyl] urea:

The title compound was prepared from 5-cyclopentyl-1,3-dihydro-1-propyl-3(*R,S*)-[(4-nitrophenyloxycarbonyl)amino]-2H-1,4-benzodiazepin-2-one (254mg, 0.56mmol) and 1-(isopropylsulphonylaminocarbonyl)-3-aminobenzene [Example 12, Step 3] (137mg, 0.56mmol) using the procedure described in Example 11, Step 4, to afford the title compound (162mg, 52%) as a white solid. mp 159-162°C (MeOH). $^1H$ NMR (360MHz, D6-DMSO) δ 0.72 (3H, t, J = 7.3Hz), 1.10-1.31 (9H, m), 1.49-1.63 (4H, m), 1.71 (1H, m), 1.82 (1H, m), 2.04 (1H, m), 3.51 (1H, m), 3.68 (1H, m), 3.79 (1H, m), 4.22 (1H, m), 5.06 (1H, d, J = 8.3Hz), 7.33-7.41 (3H, m), 7.46 (1H, d, J = 7.8Hz), 7.55 (1H, d, J = 7.9Hz), 7.63-7.64 (2H, m), 7.79 (1H, d, J = 7.8Hz), 7.92 (1H, s), 9.19 (1H, s), 11.93 (1H, s).

EXAMPLE 37 N-[3(*R,S*)-5-Cyclopentyl-2,3-dihydro-1-propyl-2-oxo-1H-1,4-benzodiazepin-3-yl] N′-[3-(1,1-dimethylethylsulphonylaminocarbonyl)phenyl] urea

This compound was prepared from 5-cyclopentyl-1,3-dihydro-1-propyl-3(*R,S*)-[(4-nitrophenyloxycarbonyl)amino]-2H-1,4-benzodiazepin-2-one [Example 36, Step 3] (136mg, 0.31mmol) and 1-(1,1-dimethylethylsulphonylaminocarbonyl)-3-aminobenzene [Example 31, Step 6] (79mg, 0.31mmol) using the procedure described in Example 11, Step 4, with a heating time of 18h to afford the title compound (64mg, 37%) as a white solid. mp 160-166°C (MeOH/$H_2O$). $^1H$ NMR (360MHz, D6-DMSO) δ 0.72 (3H, t, J = 7.3Hz), 1.16-1.38 (3H, m), 1.38 (9H, s), 1.50-1.61 (4H, m), 1.70 (1H, m), 1.82 (1H, m), 2.03 (1H, m), 3.51 (1H, m), 3.68 (1H, m), 4.20 (1H, m), 5.06 (1H, d, J = 8.4Hz), 7.34-7.40 (4H, m), 7.54 (1H, d, J = 8.1Hz), 7.63 (2H, m), 7.78 (1H, d, J = 8.2Hz), 7.84 (1H, s), 9.18 (1H, s), 11.51 (1H, s).

EXAMPLE 38 N-(3(*R,S*)-5-Cyclopentyl-2,3-dihydro-1-methyl-2-oxo-1H-1,4-benzodiazepin-3-yl] N′-[3-(isopropylsulphonylaminocarbonyl)phenyl] urea

A solution of 5-cyclopentyl-1,3-dihydro-1-methyl-3(*R,S*)-[(4-nitrophenyloxycarbonyl)amino]-2H-1,4-benzodiazepin-2-one (155mg, 0.37mmol) in anhydrous dimethylformamide (4ml), under an atmosphere of nitrogen, at ambient temperature was treated with triethylamine (60μl, 0.43mmol). After stirring at ambient temperature for 5 min, 1-(isopropylsulphonylaminocarbonyl)-3-aminobenzene was added in one portion. The yellow solution was heated at 60°C for 2h. The solution was cooled and the solvent evaporated *in vacuo*. The residue was partitioned between ethyl acetate (20ml) and 20% aqueous acetic acid (5ml). The organic phase was collected and the aqueous phase extracted with ethyl acetate (2 x 20ml). The combined organic layers were dried ($MgSO_4$) and evaporated *in vacuo*. The residue was azeotroped with toluene (20ml) and triturated with diethyl ether to give a cream solid. This was recrystallised from methanol to give the title compound (87mg, 45%) as a white solid. mp 169-170°C (dec.). $^1H$ NMR (360MHz, $CDCl_3$) δ 1.20-2.20 (9H, m), 1.43 (6H, dd, J = 6.8 and 1Hz), 3.37 (1H, m), 3.48 (3H, s), 3.93 (1H, septet, J = 6.8Hz), 5.44 (1H, d, J = 7.5Hz), 7.10-7.70 (8H, m), 8.27 (1H, s).

EXAMPLE 39 N-(3(*R,S*)-5-Cyclopentyl-2,3-dihydro-1-methyl-2-oxo-1H-1,4-benzodiazepin-3-yl] N′-[3-(1,1-dimethylethylsulphonylaminocarbonyl)phenyl] urea

Step 1: 5-Cyclopentyl-1,3-dihydro-1-methyl-3(*R,S*)-[(benzyloxycarbonyl)amino]-2H-1,4-benzodiazepine-2-one:

To a solution of 5-cyclopentyl-1,3-dihydro-3(*R,S*)-[(benzyloxycarbonyl)amino]-2H- 1,4-benzodiazepin-2-one (1.47g, 3.91mmol) in anhydrous dimethylformamide (40ml) under nitrogen, cooled in an ice bath, was added sodium hydride (55% dispersion in oil, 178mg, 4.08mmol) in portions and the resulting mixture stirred for 1h. Iodomethane (0. 26ml, 4.1mmol) was then added and the mixture stirred for 30 min. The solvent was evaporated and the residue partitioned between dichloromethane (20ml) and water (20ml). The aqueous layer was re-extracted with dichloromethane (20ml), the organic extracts were combined, dried ($MgSO_4$), and evaporated. The residue was triturated with diethyl ether to afford the title compound (1.19g, 78%) as a white solid. $^1H$ NMR (360MHz, $CDCl_3$) δ 1.26 (1H, m), 1.46-2.00 (6H, m), 2.00-2.20 (1H, m), 3.30 (1H, m), 3.40 (3H, s), 5.08 (2H, d, J = 1.4Hz), 5.15 (1H, d, J = 8Hz), 6.50 (1H, d, J = 8Hz), 7.24-7.62 (9H, m).

Step 2: 3(*R,S*)-Amino-5-cyclopentyl-1,3-dihydro-1-methyl-2H-1,4-benzodiazepin-2-one:

To 5-cyclopentyl-1,3-dihydro-1-methyl-3(*R,S*)-[(benzyloxycarbonyl)amino]-2H-1,4-benzodiazepin-2-one (475mg, 1.21mmol) was added 45% hydrobromic acid in acetic acid (1ml) and the mixture stirred at room temperature for 2h. Anhydrous diethyl ether (50ml) was added and the resultant cream coloured suspension stirred at 0°C for 1h. On settling, the solvent was decanted off, and the solid triturated in anhydrous diethyl ether. The resultant solid was collected by filtration and washed with more diethyl ether. The solid was partitioned between 10% $Na_2CO_3$ solution (25ml) and ethyl acetate (20ml), the aqueous layer was further extracted with ethyl acetate (4 x 10ml) and the organic extracts combined, dried ($MgSO_4$), and evaporated to give the title compound (247mg, 79%) as a colourless oil.

Step 3: N-[3-(*R,S*)-5-Cyclopentyl-2,3-dihydro-1-methyl-2-oxo-1H-1,4-benzodiazepin-3-yl] N′-[3-(1,1-dimethylethylsulphonylaminocarbonyl)phenyl] urea:

To 1-(1,1-dimethylethylsulphonylaminocarbonyl)-3-aminobenzene (290mg, 0.86mmol) stirring in tetrahydrofuran (20ml), under nitrogen at 0°C, was added triphosgene (78mg, 0.26mmol) in one portion. After 10 min triethylamine (230mg, 2.27mmol) was added dropwise as a solution in tetrahydrofuran (1ml) over 5 min, and the resulting mixture stirred at room temperature for 20 min. On re-cooling the mixture to 0°C, 3(*R,S*)-amino-5-cyclopentyl-1,3-dihydro-1-methyl-2H-1,4-benzodiazepin-2-one (176mg, 0.68mmol) was added as a solution in tetrahydrofuran (9ml) *via* cannula and the mixture stirred for 4h at room temperature. The reaction mixture was filtered, the solid washed with tetrahydrofuran (2 x 20ml) and the filtrate evaporated. The residue was partitioned between ethyl acetate (100ml) and 10% citric acid solution (20ml) and the organic layer retained. The aqueous layer was extracted further with ethyl acetate (2 x 50ml), the organic extracts were combined, dried ($MgSO_4$), concentrated, and the residue subjected to chromatography on silica gel (5:95 - methanol: dichloromethane). The resulting solid was recrystallised twice from methanol-water to give a white solid (64mg, 14%). mp 196-197°C (dec.). $^1H$ NMR (360MHz, D6-DMSO) 1.06-1.12 (1H, m), 1.39 (9H, s), 1.49-1.65 (5H, m), 1.79-1.82 (1H, m), 2.00-2.03 (1H, m), 3.33 (3H, s), 3.49 (1H, m), 5.08 (1H, d, J = 7.6Hz), 7.35-7.42 (4H, m), 7.53-7.66 (3H, m), 7.76-7.79 (1H, m), 7.85 (1H, s), 9.19 (1H, s), 11.51 (1H, s). MS (CI, $NH_3$) 556 ($M+NH_4^+$).

EXAMPLE 40 N-[3(*R,S*)-5-Cyclopentyl-2,3-dihydro-1-methyl-2-oxo-1H-1,4-benzodiazepin-3-yl] N′-[3-(phenylsulphonylaminocarbonyl)phenyl] urea

To 1-(phenylsulphonylaminocarbonyl)-3-aminobenzene (185mg, 0.67mmol), stirring in anhydrous tetrahydrofuran (25ml) under nitrogen, at 0°C, was added triphosgene (65mg, 0.22mmol) in one portion. After 5 min triethylamine (0.26ml, 1.87mmol) was added slowly over 3 min and the cooling bath removed. The mixture was stirred at room temperature for 20 min, cooled to 0°C and 3(*R,S*)-amino-5-cyclopentyl-1,3-dihydro-1-methyl-2H-1,4-benzodiazepin-2-one (150mg, 0.58mmol) in anhydrous tetrahydrofuran (5ml) added dropwise over 5 min. The reaction mixture was stirred at 0°C for 10 min and at room temperature for 1h, before filtering to remove the white suspension. The solid was washed with tetrahydrofuran (2 x 10ml) and the combined filtrates evaporated to give an oily residue. On partitioning between ethyl acetate (100ml) and 10% citric acid solution (20ml) the organic layer was retained and the aqueous layer re-extracted with ethyl acetate (2 x 20ml). The organic layers were combined, dried ($MgSO_4$), and evaporated to give a cream coloured solid. Chromatography on silica gel, using a gradient elution (5:95 - methanol:dichloromethane then 10:90 methanol:dichloromethane) gave a cream coloured solid which was recrystallised from methanol-water to give the title compound (40mg, 12%) as a white solid. mp 187°C (dec.). $^1H$ NMR (360MHz, D6-DMSO) 1.06 (1H, m), 1.50-1.66 (5H, m), 1.77 (1H, m), 1.81 (1H, m), 3.33 (3H, s), 3.48 (1H, m), 5.07 (1H, d, J = 7.7Hz), 7.30-7.41 (4H, m), 7.49-7.69 (6H, m), 7.76-7.78 (1H, m), 7.85 (1H, s), 7.95-7.98 (2H, m), 9.15 (1H, s), 12.48 (1H, brs). MS (CI, $NH_3$) 559 ($M^+$).

EXAMPLE 41 N-[3(*R,S*)-5-Cyclobutyl-2,3-dihydro-1-methyl-2-oxo-1H-1,4-benzodiazepin-3-yl] N′-[3-methylphenyl] urea

Step 1: 2-Aminophenyl cyclobutyl methanone:

Over a period of 1h a solution of cyclobutyl bromide (13g, 0.1mol) in diethyl ether (150ml) was added dropwise to a slurry of magnesium turnings (2.5g, 0.11mol) and a crystal of iodine in diethyl ether (20ml) at reflux. The mixture was stirred for a further hour whereupon the Grignard solution was cannulated into a pressure equalising dropping funnel, attached to a three-necked round-bottomed flask, which was under an atmosphere of nitrogen.

A solution of 2-aminobenzonitrile (3.78g, 32mmol) at 0°C in diethyl ether (50ml) was treated dropwise with the Grignard reagent prepared above, over a period of 15 min. Once the addition was complete, the mixture was warmed to room temperature and stirred for 16h under nitrogen. The solution was cooled to 0°C, quenched with 5N hydrochloric acid (20ml), and basified using solid sodium hydroxide (4g). The aqueous solution was extracted with ethyl acetate (2 x 100ml) and the combined organic layers were dried ($Na_2SO_4$) and evaporated. The residue was chromatographed on silica gel using 2:1 petrol:ethyl acetate as the eluant. This gave a yellow oil which was then azeotroped with toluene (2 x 80ml) to give the title compound (4g, 71%) as a pale yellow solid. mp 55°C. $^1H$ NMR (250MHz, $CDCl_3$) δ 1.72-2.48 (6H, m), 3.80-4.00 (1H, m), 6.23 (2H, brs), 6.50-6.61 (2H, m), 7.11-7.22 (1H, m), 7.45-7.54 (1H, m).

Step 2: 5-Cyclobutyl-1,3-dihydro-3(*R,S*)-[(benzyloxycarbonyl)amino]-2H-1,4-benzodiazepin-2-one:

A solution of α-isopropylthio-N-benzyloxycarbonyl glycine (8.4g, 29.7mmol) in anhydrous dichloromethane (200ml) was cooled to 0°C. N-methylmorpholine (3.3ml, 29.7mmol) was added over 2 min followed by isobutyl chloroformate (3.9ml, 29.7mmol). This mixture was stirred for 15 min at 0°C whereupon the mixture was heated to reflux. 2-Aminophenyl cyclobutyl methanone (4g, 22.9mmol) in anhydrous dichloromethane (20ml) was added dropwise at reflux to the reaction mixture over 10 min and the mixture stirred at reflux for a further 1.5 h. The reaction mixture was washed with 1N citric acid (100ml), water (100ml), saturated sodium bicarbonate solution ( 100ml) and brine (100ml). The organic phase was dried ($Na_2SO_4$), evaporated and azeotroped with toluene (2 x 100ml) to give a yellow oil. Trituration with 7:1 petrol:ethyl acetate afforded the product (8g, 80%) as a colourless solid. This material was used without further purification.

A solution of anhydrous tetrahydrofuran (300ml) was cooled to 0°C and saturated with ammonia gas. To this solution was added the glycinamide (8g, 18mmol) prepared above, followed by mercuric chloride (7.4g, 27mmol). The mixture was stirred at 0°C for 1.5 h with continuous bubbling of ammonia gas. The mixture was filtered through "hyflo" and the filtrate evaporated to afford the desired amine as a colourless waxy solid. The material was used without further purification.

The amine (6.9g, 18mmol) prepared above was dissolved in acetic acid (250ml) and treated with ammonium acetate (6.5g, 84.6mmol). This mixture was stirred at room temperature for 16h under nitrogen. The solvent was evaporated and the residue partitioned between ethyl acetate (250ml) and 10% sodium hydroxide solution (100ml). The organic layer was separated, dried ($Na_2SO_4$) and evaporated to give a yellow solid. Trituration with diethyl ether afforded the title compound (3.8g, 50%) as a colourless solid. mp 200-202°C. TLC (silica, petrol:ethyl acetate 2:1). Rf = 0.3. $^1H$ NMR (250MHz, $CDCl_3$) δ 1.60-2.80 (6H, m), 3.70 (1H, m), 5.12 (2H, m), 5.22 (1H, d, J = 8Hz), 6.50 (1H, d, J = 8Hz), 7.02-7.53 (9H, m), 9.44 (1H, s).

Step 3: 5-Cyclobutyl-1,3-dihydro-3(*R,S*)-[(benzyloxycarbonyl)amino]-1-methyl-2H-1,4-benzodiazepin-3-one:

5-Cyclobutyl-1,3-dihydro-3(*R,S*)-[(benzyloxycarbonyl)amino]-2H-1,4-benzodiazepin-2-one (1g, 2.75mmol) in anhydrous toluene (70ml) was heated to reflux. A solution of dimethylformamide dimethyl acetal (1.75ml, 13.7mmol) in anhydrous toluene (10ml) was added dropwise and the mixture was heated at reflux for a further 3h. The solvent was evaporated and the residue triturated with diethyl ether to afford the title compound (0.75g, 72%) as a colourless solid. mp 210-211°C. $^1H$ NMR (250MHz, $CDCl_3$) δ 1.68-2.06 (4H, m), 2.20-2.60 (2H, m), 3.41 (3H, s), 3.60-3.80 (1H, m), 5.00-5.30 (3H, m), 6.51 (1H, d, J = 14Hz), 7.14-7.54 (9H, m).

Step 4: 3(*R,S*)-Amino-5-cyclobutyl-1,3-dihydro-1-methyl-2H-1,4-benzodiazepin-2-one:

5-Cyclobutyl-1,3-dihydro-3(*R,S*)-[(benzyloxycarbonyl)amino]-1-methyl-2H-1,4-benzodiazepin-3-one (400mg, 1.06mmol) was treated with a solution of 45% hydrogen bromide in acetic acid (10ml), and stirred for 20 min at room temperature. The mixture was then added dropwise onto cold (0°C) diethyl ether (50ml). A white

solid was precipitated and filtered off. The solid was treated with 10% sodium hydroxide solution (50ml), then extracted with ethyl acetate (80ml). The organic layer was separated, dried ($Na_2SO_4$) and evaporated to give a yellow foam. This material was then used without further purification.

Step 5: N-[3(*R,S*)-5-Cyclobutyl-2,3-dihydro-1-methyl-2-oxo-1H-1,4-benzodiazepin-3-yl] N'-[3-methylphenyl] urea:

A solution of 3(*R,S*)-amino-5-cyclobutyl-1,3-dihydro-1-methyl-2H-1,4-benzodiazepin-2-one (50mg, 0.21mmol) in anhydrous tetrahydrofuran (5ml) under nitrogen was treated with 3-methylphenylisocyanate (27μl, 0.21mmol). The mixture was stirred for 20 min at room temperature whereupon a solid precipitated out of solution. The solid was collected by filtration and triturated with diethyl ether to afford the title compound (50mg, 64%) as a colourless powder. mp 135-137°C. $^1H$ NMR (250MHz, D6-DMSO) δ 1.60-2.00 (4H, m), 2.28 (3H, s), 2.30-2.45 (2H, m), 3.36 (3H, s), 3.80-4.00 (1H, m), 5.00-5.10 (1H, m), 6.60-7.70 (8H, m), 8.55 (1H, s), 8.90 (1H, s).

EXAMPLE 42 N-[3(*R,S*)-5-Cyclohexyl-2,3-dihydro-1-methyl-2-oxo-1H-1,4-benzodiazepin-3-yl] N'-[3(*R,S*)-(methylsulphinyl)phenyl] urea

Step 1: N-*tert*-Butyloxycarbonyl-3-(methylmercapto)aniline:

To a solution of 3-(methylmercapto)aniline (5ml, 40.5mmol) in anhydrous dichloromethane (100ml) was added di-*tert*-butyl dicarbonate (8.86g, 40.5mmol) and the resulting dark brown mixture stirred at room temperature for 64h under a nitrogen atmosphere. Diethyl ether (200ml) was then added and the organic phase was washed with 1N hydrochloric added (1 x 25ml), water (1 x 25ml), brine (1 x 40ml), dried ($MgSO_4$) and concentrated. Flash chromatography of the residue on silica gel (hexane: diethyl ether, 90:10) gave the title compound (8.45g, 87%) as a white solid. $^1H$ NMR (250MHz, $CDCl_3$) δ 1.52 (9H, s), 2.47 (3H, s), 6.45 (1H, brs), 6.92 (1H, ddd, J = 7.8, 1.9 and 1.2Hz), 7.06 (1H, ddd, J = 7.8, 1.9 and 1.2Hz), 7.18 (1H, t, J = 7.8Hz), 7.36 (1H, t, J = 1.9Hz). MS (CI, $NH_3$) 239 ($M^+$).

Step 2: N-*tert*-Butyloxycarbonyl-3(*R,S*)-(methylsulphinyl)aniline:

To a solution of N-*tert*-butyloxycarbonyl-3-(methylmercapto)aniline (3.7g, 15.5mmol) and n-tetrabutylammonium bromide (1g, 3.1mmol) in dichloromethane (500ml) was added a solution of ammonium cesium (IV) nitrate (17.8g, 32.5mmol) in water (90ml). The resulting two-phase system was vigorously stirred at room temperature for 3h before the organic phase was decanted off, washed with water (2 x 400ml), dried ($Na_2SO_4$) and concentrated. Flash chromatography of the remaining brown oil on silica gel, using ethyl acetate as the eluant afforded the title compound (550mg, 14%) as a brown-red glass. $^1H$ NMR (250MHz, $CDCl_3$) δ 1.54 (9H, s), 2.73 (3H, s), 6.79 (1H, brs), 7.27-7.32 (1H, m), 7.40-7.51 (2H, m), 7.72 (1H, brs). MS (CI, $NH_3$) 255 ($M^+$).

Step 3: 3(*R,S*)-(Methylsulphinyl)aniline:

A solution of N-*tert*-butyloxycarbonyl-3(*R,S*)-(methylsulphinyl)aniline (480mg, 1.88mmol) in a mixture of dichloromethane (15ml) and trifluoroacetic acid (4ml) was allowed to stand at room temperature for 30 min. Solvents were removed under vacuum and the residue azeotroped with methanol (10ml) then purified by flash chromatography on silica gel, using a gradient elution (ethyl acetate:ethanol, 98:2 to 95:5) to give the title compound (280mg, 96%) as a pale yellow solid. $^1H$ NMR (250MHz, $CDCl_3$+D6-DMSO) δ 2.70 (3H, s), 6.77 (1H, ddd, J = 7.8, 2.0 and 0.9Hz), 6.86 (1H, ddd, J = 7.8, 1.5 and 0.9Hz), 7.00 (1H, t, J = 2.0Hz), 7.25 (1H, t, J = 7.8Hz). MS (CI, $NH_3$) 154 (M-1).

Step 4: N-[3(*R,S*)-5-Cyclohexyl-2,3-dihydro-1-methyl-2-oxo-1H-1,4-benzodiazepin-3-yl] N'-[3(*R,S*)-(methylsulphinyl)phenyl]urea:

The title compound was prepared in 32% yield from 5-cyclohexyl-1,3-dihydro-1-methyl-3(*R,S*)-[(4-nitrophenyloxycarbonyl)amino]-2H-1,4-benzodiazepin-2-one and 3(*R,S*)-(methylsulphinyl)aniline using the same method as that described in Example 11, Step 4. The crude product was purified by flash chromatography on silica gel, using dichloromethane:methanol (94:6) as the eluant and crystallised from ethyl acetate. mp 160°C. $^1H$ NMR (360MHz, D6-DMSO) δ 0.92 (1H, m), 1.06-1.66 (7H, m), 1.78 (1H, m), 1.90 (1H, m), 2.68 (3H, s), 2.93 (1H, m), 3.32 (3H, s), 5.07 (1H, d, J = 8.2Hz), 7.17 (1H, brd, J = 6.6Hz), 7.33-7.44 (4H, m), 7.55 (1H, d, J =

7.6Hz), 7.64 (1H, t, J = 7.0Hz), 7.75 (1H, d, J = 8.2Hz), 7.77 (1H, s), 9.30 (1H, s). MS (CI, $NH_3$) 453 ($M^+$).

EXAMPLE 43 N-[3(*R,S*)-5-Cyclohexyl-2,3-dihydro-1-methyl-2-oxo- 1H- 1,4-benzodiazepin-3-yl] N′-[3-(5-hydroxy-4-pyron-2-yl)phenyl] urea

Step 1: 3-Acetoxy-6-(3-nitrophenyl)-4-pyrone:

To a stirred solution of hexamethyldisilazane (10.8ml, 0.05mol) in anhydrous tetrahydrofuran (140ml), at -78°C, was added n-butyllithium (31.9ml of a 1.6M solution in hexane, 0.05mol) dropwise. After 20 min a solution of 1-methoxy-2-acetoxybuten-3-one (8.0g, 0.05mol) in anhydrous tetrahydrofuran (60ml) was added dropwise. After a further 20 min a solution of 3-nitrobenzoyl chloride (4.82g, 0.026mol) in anhydrous tetrahydrofuran (40ml) was added dropwise. The cooling bath was removed and the reaction mixture warmed to -15°C over 30 min. The mixture was then quenched using 2M HCl (50ml) and stirred for 1h. The organic layer was separated and the aqueous phase extracted with ether (2 x 100ml). The combined organic layers were washed with brine (200ml), dried ($Na_2SO_4$) and evaporated *in vacuo* to give an orange gum.

This gum was dissolved in toluene (100ml), pyridinium *para*-toluene sulphonate (1g) was added and the mixture heated to reflux for 1h. The solvent was removed *in vacuo*, the residue dissolved in chloroform (300ml) and washed with 10% sodium bicarbonate solution (2 x 100ml) and water (100ml). The organic phase was separated, dried ($Na_2SO_4$) and evaporated *in vacuo* to afford a brown residue. The residue was chromatographed on silica gel, eluting with ethyl acetate to afford a beige solid, which was recrystallised from ethyl acetate to give the title compound (2.00g, 28%) as a cream solid. mp 170-171°C. $^1$H NMR (360MHz, $CDCl_3$) δ 2.37 (3H, s), 7.00 (1H, s), 7.73 (1H, dd, J = 8.0 and 8.0Hz), 8.05-8.08 (2H, m), 8.39 (1H, dd, J = 8 and 1Hz), 8.66 (1H, dd, J = 1 and 1Hz).

Step 2: 3-Acetoxy-6-(3-aminophenyl)-4-pyrone:

3-Acetoxy-6-(3-nitrophenyl)-4-pyrone(1.80g, 6.5mmol) was dissolved in methanol (50ml) and hydrogenated at 10 psi for 1h, using 10% palladium on carbon catalyst (0.2g, 11% ($^w/_w$)). The reaction mixture was then filtered through "hyflo" and the filtrate evaporated *in vacuo*. The residue was recrystallised from ethyl acetate:hexane (2:1) to afford the desired product (1.20g, 75%) as a beige solid. mp 127-129°C. $^1$H NMR (360MHz, $CDCl_3$) δ 2.35 (3H, s), 3.85 (2H, brs), 6.81 (1H, dd, J = 8.0 and 1.0Hz), 6.84 (1H, s), 7.02 (1H, dd, J = 1.0 and 1.0Hz), 7.11 (1H, brd, J = 8.0Hz), 7.25 (1H, dd, J = 8.0 and 8.0Hz), 7.98 (1H, s).

Step 3: N-[3(*R,S*)-5-Cyclohexyl-2,3-dihydro-1-methyl-2-oxo-1H-1,4-benzodiazepin-3-yl] N′-[3-(5-acetoxy-4-pyron-2-yl)phenyl] urea:

To a stirred and cooled (4°C) solution of 3-acetoxy-6-(3-aminophenyl)-4-pyrone (335mg, 1.37mmol) in anhydrous tetrahydrofuran (8ml), was added triphosgene (133mg, 0.45mmol) followed by triethylamine (0.19ml, 1.37mmol). The ice bath was removed and the reaction mixture stirred at room temperature for 20 min. A solution of 3(*R,S*)-amino-5-cyclohexyl-1,3-dihydro-1-methyl-2H-1,4-benzodiazepin-2-one (310mg, 1.14mmol) in anhydrous tetrahydrofuran (8ml) was added and the reaction mixture stirred at room temperature for 1h. After this time water (20ml) and ethyl acetate (30ml) were added, the organic layer separated and the aqueous phase extracted with ethyl acetate (30ml). The combined organic layers were washed with water (30ml) and brine (30ml) then dried ($Na_2SO_4$). The filtrate was evaporated *in vacuo* and the resultant orange solid recrystallised from ethyl acetate/ether to give the title compound (520mg, 84%) as a pale yellow solid. mp 183-187°C. $^1$H NMR (360MHz, $CDCl_3$) δ 1.08-2.04 (10H, m), 2.34 (3H,s), 2.81 (1H, dd, J = 11 and 11Hz), 3.45 (3H, s), 5.39 (1H, d, J = 7.5Hz), 6.81 (1H, s), 7.03 (1H, d, J = 7.5Hz), 7.25-7.60 (8H,m), 7.87 (1H,s), 7.93 (1H,s).

Step 4: N-[3(*R,S*)-5-Cyclohexyl-2,3-dihydro-1-methyl-2-oxo-1H-1,4-benzodiazepin-3-yl] N′-[3-(5-hydroxy-4-pyron-2yl)phenyl] urea:

To a stirred suspension of N-[3(*R,S*)-5-cyclohexyl-2,3-dihydro-1-methyl-2-oxo-1H-1,4-benzodiazepin-3-yl] N′-[3-(5-acetoxy-4-pyron-2-yl)phenyl] urea (390 mg, 0.72mmol) in methanol (60ml) was added potassium carbonate (150mg, 1.08mmol). The reaction mixture was stirred at room temperature for 1h. Citric acid (226mg, 1.1mmol) was added and the solvent evaporated *in vacuo*. The residue was partitioned between water (15ml) and ethyl acetate (30ml), and the organic layer separated. The aqueous phase was re-extracted with ethyl acetate (30ml) and the combined organic layers dried ($Na_2SO_4$) and evaporated *in vacuo*. The resultant pale yellow solid was recrystallised from ethyl acetate/ether to afford the title compound (250mg, 69%) as a beige solid.

mp 207-210°C. $^1$H NMR (360MHz, D6-DMSO) δ 0.88-1.93 (10H, m), 2.94 (1H, dd, J = 13 and 13Hz), 3.33 (3H, s), 5.08 (1H, d, J = 8Hz), 6.90 (1H, s), 7.34-7.45 (5H, m), 7.55 (1H, d, J = 7Hz), 7.64 (1H, dd, J = 7 and 7Hz), 7.75 (1H, d, J = 7Hz), 7.99 (1H, s), 8.13 (1H, s), 9.23 (2H, brs).

EXAMPLE 44 N-[3(*R,S*)-5-Cyclohexyl-2,3-dihydro-1-methyl-2-oxo-1H-1,4-benzodiazepin-3-yl] N′-(3,4-dihydro-2,2-dioxo-4-oxo-1H-2,3-benzothiazin-6-yl] urea

Step 1: Methyl 2-methyl-5-nitrobenzoate:

To methanol (150ml) at 0°C under nitrogen was added dropwise thionyl chloride (4.0ml, 54.8mmol) over 2 min. The mixture was stirred at 0°C for 15 min, before adding a solution of 2-methyl-5-nitrobenzoic acid (4.99g, 27.5mmol) in methanol (50ml) by cannula over 5 min. The mixture was then allowed to warm to room temperature before heating at 50°C for 18h under nitrogen. The solvents were evaporated *in vacuo* and the residue stirred with water (100ml) and dichloromethane (100ml). The aqueous layer was basified with potassium carbonate, separated and re-extracted with more dichloromethane (100ml). The two organic extracts were combined, dried ($MgSO_4$), and evaporated *in vacuo* to leave the title compound (5.24g, 98%) as a white solid. $^1$H NMr (360MHz, $CDCl_3$) δ 2.72 (3H, s), 3.96 (3H, s), 7.44 (1H, d, J = 8.3Hz), 8.24 (1H, dd, J = 8.4 and 2.6Hz), 8.78 (1H, d, J = 2.6Hz).

Step 2: Sodium 2-carbomethoxy-4-nitrobenzyl sulphonate:

A mixture of methyl 2-methyl-5-nitrobenzoate (2.51g, 12.9mmol) and benzoyl peroxide (containing approx. 25% water) (164mg, 0.51mmol) in carbon tetrachloride (50ml) was purged with nitrogen before removing 12ml of the solvent by distillation. N-Bromosuccinimide (2.30g, 12.9mmol) was then added in small portions to the refluxing mixture under irradiation (60W) and the mixture heated at reflux for 2.5h under nitrogen. The succinimide was removed by filtration and the filtrate evaporated *in vacuo* to give crude methyl 2-bromomethyl-5-nitrobenzoate (3.67g) as a yellow oil. To this was added sodium sulphite (2.43g, 19.3mmol) and water (10ml) and the mixture heated at 90°C for 3h. The resulting solution was allowed to cool to room temperature and the solid formed was collected, washed with cold water (2 x 5ml), then diethyl ether (3 x 10ml), and dried under high vacuum in the presence of phosphorus pentoxide. Two more crops were similarly collected to afford the title product (1.46g, 38%) as a white solid. mp 290-297°C. $^1$H NMR (360MHz, D6-DMSO) δ 3.84 (3H, s), 4.34 (2H, s), 7.68 (1H, d, J = 8.5Hz), 8.30 (1H, dd, J = 8.5 and 2.6Hz), 8.41 (1H, d, J = 2.5Hz). MS (FAB) 274 (M-1).

Step 3: Methyl 2-(aminosulphonylmethyl)-5-nitrobenzoate:

A mixture of sodium 2-carbomethoxy-4-nitrobenzylsulphonate (0.68g, 2.29mmol) and phosphorous pentachloride (0.74g, 3.55mmol) was heated at 100°C for 70 min, before removing the phosphorous oxychloride *in vacuo*. The residue was stirred with dichloromethane (5ml) at 40°C, then filtered, washing the solid well with more dichloromethane. The combined filtrates were taken and ammonia gas was bubbled through the solution for 5 min. The mixture was immediately filtered and the filtrate left to stand for 3 days. After this time a white crystalline solid was collected. Concentration of the filtrate afforded a second crop to give the title product (438mg, 70%) as a white solid. mp 167-170°C. $^1$H NMR (360MHz, D6-DMSO) δ 3.90 (3H, s), 4.94 (2H, s), 7.00 (2H, brs), 7.77 (1H, d, J = 8.5Hz), 8.46 (1H, dd, J = 8.5 and 2.5Hz), 8.56 (1H, d, J = 2.5Hz). MS (CI, $NH_3$) 292 ($M+NH_4^+$).

Step 4: 3,4-Dihydro-2,2-dioxo-6-nitro-4-oxo-1H-2,3-benzothiazine:

To a solution of methyl 2-(aminosulphonylmethyl)-5-nitrobenzoate (314mg, 1.14mmol) in dimethylformamide (25ml) was added sodium hydride (60% dispersion in oil, 45mg, 1.13mmol) and the resulting red mixture stirred at room temperature for 3h. The mixture was then partitioned between 2N HCl (100ml) and ethyl acetate (100ml). The aqueous layer was re-extracted with ethyl acetate (2 x 100ml), and the combined organic extracts were dried ($MgSO_4$) and evaporated *in vacuo*. The residue was purified by flash chromatography on silica gel, eluting with 10-30% methanol/dichloromethane, to afford the title compound (275mg, 100%) as a white solid. $^1$H NMR (360MHz, D6-DMSO) δ 4.28 (2H, s), 7.59 (1H, d, J = 8.3Hz), 8.25 (1H, dd, J = 8.3 and 2.6Hz), 8.64 (1H, d, J = 2.6Hz).

Step 5: 6-Amino-3,4-dihydro-2,2-dioxo-4-oxo-1H-2,3-benzothiazine:

A mixture of 3,4-dihydro-2,2-dioxo-6-nitro-4-oxo-1H-2,3-benzothiazine (267mg, 1.1mmol) and 10% palladium on carbon (26mg, 10% ($^{w}/_{w}$)) in methanol (50ml) was hydrogenated at 50 psi for 30 min. The mixture was then filtered, the solid washed with methanol and the filtrates evaporated *in vacuo* to give the title compound (239mg, 100%) as a yellow solid. $^1$H NMR (360MHz, D6-DMSO) δ 3.87 (2H, s), 5.11 (2H, brs), 6.59 (1H, dd, J = 8.0 and 2.5Hz), 6.88 (1H, d, J = 8.0Hz), 7.15 (1H, d, J = 2.5Hz). MS (FAB) 211 (M-1).

Step 6: N-[3(*R,S*)-5-Cyclohexyl-2,3-dihydro-1-methyl-2-oxo-1H-1,4-benzodiazepin-3-yl] N'-[3,4-dihydro-2,2-dioxo-4-oxo-1H-2,3-benzothiazin-6-yl] urea:

To a solution of 5-cyclohexyl-1,3-dihydro-1-methyl-3(*R,S*)-[(4-nitrophenyloxycarbonyl)amino]-2H-1,4-benzodiazepin-2-one (221mg, 0.51mmol) in anhydrous dimethylformamide (3ml) under nitrogen was added triethylamine (70μl, 0.51mmol). The solution was stirred for 5 min before adding a solution of 6-amino-3,4-dihydro-2,2-dioxo-4-oxo-1H-2,3-benzothiazine (107mg, 0.51mmol) and triethylamine (70μl, 0.51mmol) in anhydrous dimethylformamide (3ml) and acetonitrile (10ml). The resulting solution was heated at 50°C for 10h, before removing the solvents *in vacuo* and partitioning the residue between 20% aqueous acetic acid (5ml) and ethyl acetate (20ml). The aqueous phase was re-extracted with more ethyl acetate (2 x 20ml) and the combined organic extracts dried ($Na_2SO_4$) and evaporated *in vacuo*. The resulting yellow oil was stirred with diethyl ether (10ml) to give a pale yellow solid. This was purified by bash chromatography on silica gel, eluting with 5-20% methanol/dichloromethane, to afford the title compound (95mg, 37%) as a pale yellow solid, which was recrystallised from isopropanoldichloromethane. mp > 300°C. $^1$H NMR (360MHz, D6-DMSO) δ 0.93 (1H, m), 1.12-1.64 (7H, m), 1.76 (1H, m), 1.89 (1H, m), 2.93 (1H, m), 3.29 (3H, s), 3.96 (2H, s), 5.08 (1H, d, J = 8.4Hz), 7.09 (1H, d, J = 8.3Hz), 7.24 (1H, d, J = 8.5Hz), 7.38 (1H, t, J = 7.9Hz), 7.49 (1H, dd, J = 8.2 and 2.4Hz), 7.55 (1H, d, J = 8.3Hz), 7.63 (1H,t, J = 7.9Hz), 7.74-7.76 (2H, m), 9.04 (1H, s). MS (FAB) 508 (M-1).

EXAMPLE 45A Tablets containing 1-25mg of compound

| | Amount mg | | |
|---|---|---|---|
| Compound of formula (I) | 1.0 | 2.0 | 25.0 |
| Microcrystalline cellulose | 20.0 | 20.0 | 20.0 |
| Modified food corn starch | 20.0 | 20.0 | 20.0 |
| Lactose | 58.5 | 57.5 | 34.5 |
| Magnesium Stearate | 0.5 | 0.5 | 0.5 |

EXAMPLE 45B Tablets containing 26-100mg of compound

| | Amount mg | | |
|---|---|---|---|
| Compound of formula (I) | 26.0 | 50.0 | 100.0 |
| Microcrystalline cellulose | 80.0 | 80.0 | 80.0 |
| Modified food corn starch | 80.0 | 80.0 | 80.0 |
| Lactose | 213.5 | 189.5 | 139.5 |
| Magnesium Stearate | 0.5 | 0.5 | 0.5 |

The compound of formula (I), cellulose, lactose and a portion of the corn starch are mixed and granulated with 10% corn starch paste. The resulting granulation is sieved, dried and blended with the remainder of the corn starch and the magnesium stearate. The resulting granulation is then compressed into tablets containing 1.0mg, 2.0mg, 25.0mg, 26.0mg, 50.0mg and 100mg of the active compound per tablet.

EXAMPLE 46 Parenteral injection

| | Amount mg |
|---|---|
| Compound of formula (I) | 1 to 100mg |
| Citric Acid Monohydrate | 0.75mg |
| Sodium Phosphate | 4.5mg |
| Sodium Chloride | 9mg |
| Water for injection | to 1ml |

The sodium phosphate, citric acid monohydrate and sodium chloride are dissolved in a portion of the water. The compound of formula (I) is dissolved or suspended in the solution and made up to volume.

EXAMPLE 47 Topical formulation

| | Amount mg |
|---|---|
| Compound of formula (I) | 1-10g |
| Emulsifying Wax | 30g |
| Liquid paraffin | 20g |
| White Soft Paraffin | to 100g |

The white soft paraffin is heated until molten. The liquid paraffin and emulsifying wax are incorporated and stirred until dissolved. The compound of formula (I) is added and stirring continued until dispersed. The mixture is then cooled until solid.

BIOLOGICAL ACTIVITY

1. CCK Receptor Binding (Pancreas)

CCK-8 sulphated was radiolabelled with $^{125}$I-Bolton Hunter reagent (2000 Ci/mmole). Receptor binding was performed according to Chang and Lotti (Proc. Natl. Acad. Sci. 83, 4923-4926, 1986) with minor modifications.

Male Sprague-Dawley rats (150-200g) were sacrificed by decapitation. The whole pancreas was dissected free of fat tissue and was homogenized in 25 volumes of ice-cold 10 mM N-2-hydroxyethyl-piperazine-N′-2-ethane sulphonic acid (HEPES) buffer with 0.1% soya bean trypsin inhibitor (pH 7.4 at 25°C) with a Kinematica Polytron. The homogenates were centrifuged at 47,800 g for 10 min. Pellets were resuspended in 10 volumes of binding assay buffer (20mM (HEPES)), 1mM ethylene glycol-bis-(β-aminoethylether-N,N′-tetraacetic acid) (EGTA), 5mM $MgCl_2$, 150 mM NaCl, bacitracin 0.25 mg/ml, soya bean trypsin inhibitor 0.1 mg/ml, and bovine serum albumin 2 mg/ml pH 6.5 at 25°C) using a Teflon (trademark) homogenizer, 15 strokes at 500 rpm. The homogenate was further diluted in binding assay buffer to give a final concentration of 0.5 mg original wet weight/1 ml buffer. For the binding assay, 50 μl of buffer (for total binding) or unlabelled CCK-8 sulphated to give a final concentration of 1 μM (for nonspecific binding) or the compounds of Formula I (for determination of inhibition of $^{125}$I-CCK-8 binding) and 50 μl of 500 pM $^{125}$I-CCK-8 (i.e. 50 pM final concentration) were added to 400 μl of the membrane suspensions in microfuge tubes. All assays were run in duplicate. The reaction mixtures were incubated at 25°C for 2 hours and the reaction terminated by rapid filtration (Brandell 24 well cell harvester) over Whatman GF/C filters, washing 3 x 4 mls with ice-cold 100 Mm NaCl. The radioactivity on the filters was counted with a LKB gamma counter.

2. CCK Receptor Binding (Brain)

CCK-8 sulphated was radiolabelled and the binding was performed according to the description for the pancreas method with minor modifications.

Male Hartley guinea pigs (300-500g) were sacrificed by decapitation and the cortex was removed and homogenized in 25 mL ice-cold 0.32 M sucrose. The homogenates were centrifuged at 1000 g for 10 minutes

and the resulting supernatant was recentrifuged at 20,000 g for 20 minutes. The $P_2$ pellet was resuspended in binding assay buffer (20mM HEPES, 5 mM $MgCl_2$, 0.25 mg/ml bacitracin, 1 mM EGTA pH 6.5 at 25°C), using a Teflon (trademark) homogenizer (5 strokes at 500 rpm) to give a final concentration of 10 mg original wet weight/1.2 ml buffer. For the binding assay, 50 μl of buffer (for total binding) or unlabelled CCK-8 sulphated to give a final concentration of 1 μM ( for nonspecific binding) or the compounds of Formula I (for determination of inhibition of $^{125}$I-CCK-8 binding) and 50 μl of 500 pM $^{125}$I-CCK-8 (i.e. final concentration of 50 pM) were added to 400 μl of the membrane suspensions in microfuge tubes. All assays were run in duplicate. The reaction mixtures were incubated at 25°C for 2 hours and then the reaction was terminated by rapid filtration (Brandell 24 well cell harvester) on Whatman GF/C filters with 3 x 5 ml washes of cold 100 mM NaCl. The radioactivity on the filters was counted with a LKB gamma counter.

3. Gastrin Antagonism

Gastrin antagonist activity of compounds of Formula I was determined using the following assay.

A.Gastrin Receptor Binding in Guinea Pig Gastric Glands

Preparation of guinea pig gastric mucosal glands

Guinea pig gastric mucosal glands were prepared by the procedure of Chang et. al., Science, 230, 177-179 (1985) with slight modifications. Gastric mucosa from guinea pigs (300-500 g body weight, male Hartley) were isolated by scraping with a glass slide after washing stomachs in ice-cold, aerated buffer consisting of the following: 130 mM NaCl, 12 mM $NaHCO_3$, 3 mM $NaH_2PO_4$, 3 mM $Na_2HPO_4$, 3 mM $K_2HPO_4$, 2 mM $MgSO_4$, 1 mM $CaCl_2$, 5 mM glucose and 4 mM L-glutamine, 50 mM HEPES, 0.25 mg/ml bacitracin, 0.10 mg/ml soya bean trypsin inhibitor, 0.1 mg/ml bovine serum albumin, at pH 6.5, and then incubated in a 37°C shaking water bath for 40 minutes in buffer containing 1 mg/ml collagenase and bubbled with 95% $O_2$ and 5% $CO_2$. The tissues were passed twice through a 5 ml syringe to liberate the gastric glands, and then filtered through Nitex (trademark) #202 gauge nylon mesh. The filtered glands were centrifuged at 272 g for 5 minutes and washed twice by resuspension in 25 ml buffer and centrifugation.

B. Binding studies

The washed guinea pig gastric glands prepared as above were resuspended in 25 ml of standard buffer. For binding studies, to 250 μl of gastric glands, 30 μl of buffer (for total binding) or gastrin (3 μM final concentration, for nonspecific binding) or test compound and 20 μl of $^{125}$I-gastrin (NEN, 2200 Ci/mmole, 0.1 nM final concentration) were added. All assays were run in triplicate. The tubes were aerated with 95% $O_2$ and 5% $CO_2$ and capped. The reaction mixtures, after incubation at 25°C for 30 minutes in a shaking water bath were rapidly filtered (Brandell 24 well cell harvester) over Whatman G/F B filters presoaked in assay buffer and immediately washed further with 3 x 4 ml of 100 mM ice cold NaCl. The radioactivity on the filters was measured using a LKB gamma counter.

In Vitro Results Effects of the Compounds of Formula I on $^{125}$I-CCK-8 receptor binding

The preferred compounds of Formula I are those which produced dose-dependent inhibition of specific $^{125}$I-CCK-8 binding as defined as the difference between total and non-specific (i.e. in the presence of 1 μM CCK) binding.

Drug displacement studies were performed with at least 10 concentrations of compounds of Formula I and the $IC_{50}$ values were determined by regression analysis $IC_{50}$ refers to the concentration of the compound required to inhibit 50% of specific binding of $^{125}$I-CCK-8.

The data in Table I were obtained for compounds of Formula I.

TABLE I

CCK RECEPTOR BINDING RESULTS

$IC_{50}$(nM)

| Compound of Ex # | $^{125}$I-CCK Pancreas | $^{125}$I-CCK Brain | $^{125}$I-Gastrin Gastric Glands |
|---|---|---|---|
| 1 | 1.5 | 2.2 | 0.83 |
| 2 | ~27% at 3μM | 0.42 | NT |
| 3 | 800 | 0.3 | NT |
| 4 | 11 | 0.09 | 0.44 |
| 5 | 140 | 0.71 | NT |
| 6 | 1100 | 0.96 | NT |
| 7 (Peak A) | 1700 | 0.47 | 0.92 |
| 7 (Peak B) | 0.36 | 100 | NT |
| 8 | 56 | 1.6 | NT |
| 9 | NT | NT | NT |
| 10 | 3.1 | 1.4 | NT |
| 11 | 118 | 0.32 | NT |
| 12 | 12.4 | 0.29 | 3.24 |
| 13 | 738 | 0.24 | 27 |
| 14 | 11.1 | 0.58 | NT |
| 15 | 19.8 | 0.4 | 1.06 |
| 16 | 2.0 | 0.58 | 0.45 |
| 17 | 137 | 0.9 | 10.5 |
| 18 | 13.8 | 0.09 | NT |
| 19 | 570 | 12 | NT |
| 20 | 953 | 1.4 | 1.77 |
| 21 | 9.7 | 1.2 | NT |
| 22 | 70 | 6.8 | NT |
| 23 | 5 | 0.96 | NT |
| 24 (Peak A) | 1180 | 0.78 | 0.7 |
| 24 (Peak B) | 5 | 18.9 | NT |
| 25 | 220 | 1.9 | NT |
| 26 | 60 | 2.3 | NT |

| Compound of Ex # | $^{125}$I-CCK Pancreas | $^{125}$I-CCK Brain | $^{125}$I-Gastrin Gastric Glands |
|---|---|---|---|
| 27 | 19% at 3μM | 0.38 | 1.34 |
| 28 | 8.3 | 4 | NT |
| 29 | 22 | 6.3 | NT |
| 30 | 170 | 3.2 | NT |
| 31 | 6.2 | 0.22 | NT |
| 32 | 610 | 1.3 | NT |
| 33 | 1600 | 0.27 | NT |
| 34 | 1700 | 2.3 | NT |
| 35 | 10.2 | 50.8 | NT |
| 36 | 1300 | 1.2 | NT |
| 37 | 960 | 0.19 | NT |
| 38 | 300 | 6.8 | NT |
| 39 | 270 | 0.56 | 1.18 |
| 40 | 310 | 3.8 | NT |
| 41 | NT | NT | NT |
| 42 | 18 | 15 | NT |
| 43 | 17.8 | 2.1 | NT |
| 44 | 100 | 3.4 | NT |

N.T. = not tested.

EXAMPLE 48 N-[3(*R,S*)-5-Cyclohexyl-2,3-dihydro-1-methyl-2-oxo-1H-1,4-benzodiazepin-3-yl]N'-[3-(2-(imidazol-3-yl)ethyl)phenyl] urea

Step 1: 1-(1,1-Dimethylethyloxycarbonyl)-2-((3-nitrophenyl)ethen-2-yl)imidazole:

A suspension of 3-nitrobenzyl bromide (10g, 46mmol) in acetonitrile (100ml) was added to a solution of triphenylphosphine (13.3g, 51mmol) in acetonitrile (150ml). The resulting suspension was heated at reflux (15 min), cooled and 15.5g of (3-nitrobenzyl)triphenylphosphonium bromide was obtained by filtration. A mixture of this compound (10g, 21mmol) and imidazole-2-carboxaldehyde (2g, 21mmol) was heated at reflux in ethanol (200ml) and a solution of sodium ethoxide in ethanol (sodium (480mg, 21mmol; ethanol (100ml)) was added dropwise over 2h. After a further 3h at reflux the solution was cooled, filtered and the solvent evaporated. The residue was taken up in 2M HCl (50ml). The aqueous phase was washed with diethyl ether (3 x 50ml), basified with 1N sodium hydroxide solution and the product extracted into diethyl ether (3 x 50ml). The organic phase was dried ($NaSO_4$) and evaporated to afford 4g of a crude *cis/trans* mixture of 2-((3-nitrophenyl)ethen-2-yl)imidazole as a yellow solid. This material (1.75g, 8.14mmol) was dissolved in a mixture of dichloromethane (40ml) and acetonitrile (40ml) and di-t-butyl dicarbonate (2.13g, 9.8mmol) was added. After 48h at room temperature the solvent was evaporated and the residue chromatographed on silica gel with ethyl acetate:petrol (bp 60-80°) (1:1) as eluant to afford 1.7g of a *cis/trans* mixture of the titled compound as a yellow oil. $^1$H NMR (250MHz, $CDCl_3$) δ 1.64 and 1.70 (9H, 2s), 6.72-8.50 (8H).

Step 2: 1-(1,1-Dimethylethyloxycarbonyl)-2-((3-aminophenyl)ethan-2-yl)imidazole:

A solution of 1-(1,1-dimethylethyloxycarbonyl)-2-((3-nitrophenyl)ethen-2-yl)imidazole (500mg, 1.59mmol)

in ethanol (50ml) was hydrogenated at 30 psi in the presence of 10% palladium-on-carbon (50mg) for 1h. The catalyst was then removed by filtration and the solvent evaporated to afford the titled compound as a viscous oil (340mg, 75% yield). $^1H$ NMR (250MHz, $CDCl_3$) δ 1.60 (9H, s), 2.92-3.04 (2H, m), 3.22-3.36 (2H, m), 3.70 (2H, brs), 6.42-6.68 (3H, m), 6.87 (1H, d, J = 2.5Hz), 7.05 (1H, t, J = 8Hz), 7.32 (1H, d, J = 2.5Hz).

Step 3: N-(3(*R,S*)-5-Cyclohexyl-2,3-dihydro-1-methyl-2-oxo-1H-1,4-benzodiazepin-3-yl] N'-[3-(2-(imidazol-2-yl)ethyl)phenyl] urea:

To a stirred solution of 5-cyclohexyl-1,3-dihydro-1-methyl-3(*R,S*)[(4-nitrophenyloxycarbonyl)amino]-2H-1,4-benzodiazepin2-one (250mg, 0.57mmol) in dimethylformamide (5ml) was added triethylamine (80μl, 0.57mmol). After 5 min a solution of 1-(1,1-dimethylethyloxycarbonyl)-2-((3-aminophenyl)ethan-2-yl)imidazole (165mg, 0.573mmol) in dimethylformamide (5ml) was added and the solution heated at 50°C for 4.5h. After this time the solution was cooled to ambient temperature and evaporated. The residue was partitioned between sodium bicarbonate solution (50ml) and ethyl acetate (50ml). The organic phase was washed with sodium bicarbonate solution (3 x 50ml), dried ($Na_2SO_4$) and evaporated to afford a gummy solid which was dissolved in dichloromethane (5ml) to which was added trifluoroacetic acid (0.5ml). The resulting solution was stirred at room temperature for 18h after which time the volatiles were removed by evaporation. The gummy residue was partitioned between dichloromethane (50ml) and sodium bicarbonate solution (20ml). The insolubles were collected by filtration, washed with water and ether and recrystallised from ethanol to afford the titled compound as a colourless solid, mp, 202-204°C. $^1H$ NMR (360MHz, D6-DMSO+TFA) δ 0.80-2.00 (10H, m), 2.95-3.05 (3H, m), 3.16 (2H, t, J = 7Hz), 3.33 (3H, s), 5.10 (1H, s), 6.68-6.75 (1H, m), 7.10-7.20 (2H, m), 7.26-7.44 (3H, m), 7.50-7.58 (3H, m), 7.66 (1H, t, J = 7Hz), 7.78 (1H, d, J = 8Hz), 9.0 (1H, s), 14.0 (1H, brs).

## Claims

1. A compound of formula (I), or a salt or prodrug thereof:

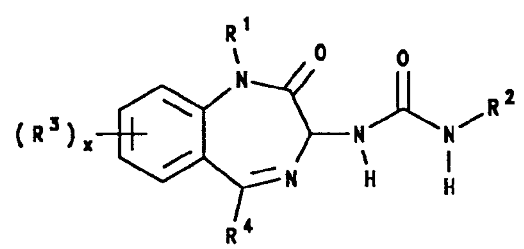

(I)

wherein:

$R^1$ represents $(CH_2)_q$imidazolyl, $(CH_2)_q$tetrazolyl, $(CH_2)_q$triazolyl; $C_{1-6}$alkyl optionally substituted by one or more groups selected from halo, hydroxy and $NR^6R^7$; $C_{3-7}$cycloalkyl; cyclopropylmethyl; $CH_2CO_2R^5$; $CH_2CONR^6R^7$; or $CH_2CH(OH)$-W-$(CH_2)_2NR^6R^7$ where $R^5$ represents $C_{1-4}$alkyl, $R^6$ and $R^7$ each independently represents a hydrogen atom or a $C_{1-4}$alkyl group, or taken together with the nitrogen atom to which they are attached form a pyrrolidinyl, or piperidinyl ring, q is 1, 2 or 3, and W is S or NH;

$R^2$ represents:

(i) a phenyl group optionally substituted by one or more substituents selected from $C_{1-6}$alkyl optionally substituted by hydroxy, $CONR^aR^b$, $CO_2R^a$ or $OCONR^aR^b$; $C_{2-6}$alkenyl optionally substituted by $CO_2R^a$; $C_{2-6}$alkynyl; halo; optionally protected hydroxy; $NHR^8$; $NHPO(OC_{1-4}alkyl)$; $(CH_2)_n$tetrazolyl optionally substituted in the tetrazolyl ring by $C_{1-4}$alkyl; $(CH_2)_n$imidazolyl; CONH-tetrazolyl; CONH-triazolyl; diazolinone; triazolinone optionally substituted by methyl; tetrazolinone; oxathiadiazolone; 5-hydroxy-4-pyrone; $CONH_2$; $CONHCOR^9$; $SO(C_{1-6}alkyl)$; $SO_2(C_{1-6}alkyl)$; $CONHCO_2R^9$; $CONHCONHR^9$; $C(NH_2)NOH$; $COC_{1-4}alkyl$; $CONHSO_2R^9$; $SO_2NH_2$; $NHSO_2NH_2$; $SO_2NHCO_2R^9$; $SO_2NHCONHR^9$; $SO_2NHSO_2R^9$; $SO_2NHPO(OR^aR^b)$; $SO_2NHR^{10}$; cyano; $B(OH)_2$; $CO_2H$; $CH_2OCH_2O(CH_2)_2OCH_3$, where $R^a$ and $R^b$ each independently represent H or $C_{1-6}$alkyl, n is 0, 1 or 2, $R^8$ represents H or $COC_{1-6}$alkyl, $R^9$ represents $C_{1-6}$alkyl, optionally substituted aryl, 2,2-difluorocyclopropane or trifluoromethyl, and $R^{10}$ represents a nitrogen containing heterocycle;

(ii) a group

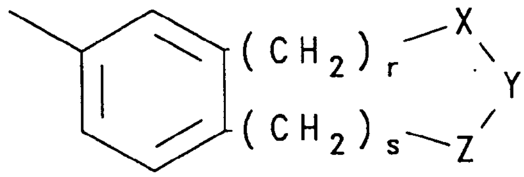

wherein X and Z each independently represent $CH_2$, O, $SO_2$, C=O, NH or N; and

Y represents $CH_2$, C=O, $NR^a$ or N, where $R^a$ is as above defined;

r and s each independently represent 0 or 1;

and the dotted line represents an optional double bond;

provided that: X and Z can only be the same when they are O, and when one of X and Z is O, the other of X and Z must be O;

when X or Z is N, Y is also N and the ring contains one unit of unsaturation;

when X or Z is $SO_2$, Y is NH and the other of X and Z is C=O;

when X or Z is $SO_2$, Y is NH and the other of X and Z is C=O, whichever of r and s is adjacent to $SO_2$ may be 1; otherwise r and s are the same and O;

(iii) a pyridyl group substituted by $C_{1-4}$alkoxy or halo;

$R^3$ represents $C_{1-6}$ alkyl or halo;

$R^4$ represents $C_{3-7}$ cycloalkyl;

x is 0, 1, 2 or 3.

**2.** A compound as claimed in claim 1 of formula (Ia),

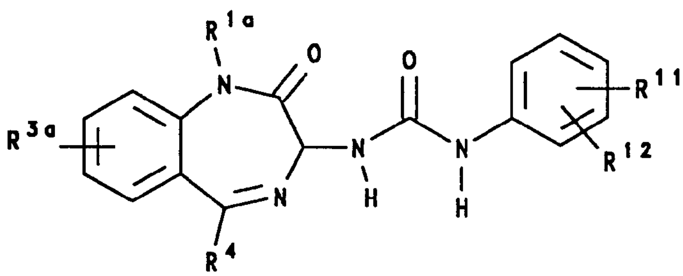

(Ia)

wherein:

$R^{1a}$ represents $C_{1-6}$ alkyl;

$R^{3a}$ represents hydrogen;

$R^4$ represents $C_{3-7}$ cycloalkyl;

$R^{11}$ represents hydrogen or $C_{1-6}$ alkyl;

$R^{12}$ represents $C_{1-6}$ alkyl; halo; $(CH_2)_n$-tetrazolyl optionally substituted in the tetrazole ring by $C_{1-4}$alkyl; $(CH_2)_n$-imidazolyl; 5-hydroxy-4-pyrone; $SO(C_{1-6}alkyl)$; $CONHSO_2R^{9a}$; $SO_2NHCOR^{9a}$ (where $R^{9a}$ is $C_{1-6}$ alkyl, optionally substituted aryl or trifluoromethyl), $SO_2NHR^{10a}$ (where $R^{10a}$ is a nitrogen containing heterocycle); or a group

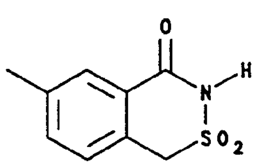

or

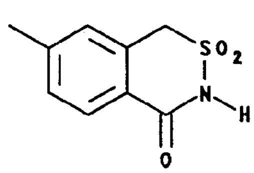

or a salt or prodrug thereof.

3. A compound as claimed in claim 2 wherein $R^{11}$ is H and $R^{12}$ is 3-tetrazol-5-yl.

4. A compound as claimed in claim 2 wherein $R^{12}$ is $CONHSO_2R^{9a}$ or $SO_2NHCOR^{9a}$.

5. A compound as claimed in claim 1 or claim 2 wherein $R^4$ is cyclobutyl, cyclopentyl or cyclohexyl.

6. A compound as claimed in claim 1 wherein $R^1$ is $C_{1-6}$alkyl, $C_{3-7}$cycloalkyl, cyclopropylmethyl, $(CH_2)_m$-imidazolyl (where m is 1 or 2), $CH_2CO_2R^6$ (where $R^6$ is $C_{1-4}$alkyl) or a group $CH_2CONR^7R^8$; $R^2$ is a phenyl group optionally substituted by one substituent selected from $C_{1-6}$ alkyl, halo, $(CH_2)_n$-tetrazolyl optionally substituted in the tetrazole ring by $C_{1-4}$alkyl, $(CH_2)_n$-imidazolyl, $CONHSO_2R^{9a}$, $SO_2NHCOR^{9b}$ (where $R^{9b}$ is $C_{1-6}$alkyl, optionally substituted aryl or trifluoromethyl), $SO_2NHR^{10}$ and $(CH_2)_nCO_2H$; and x is 0 or 1.

7. A compound as claimed in claim 6 wherein $R^2$ is a phenyl group optionally substituted by one substituent selected from $C_{1-6}$alkyl, halo, $(CH_2)_n$-tetrazolyl, $(CH_2)_n$-imidazolyl, $CONHSO_2R^{9a}$, $SO_2NHCOR^{9b}$ and $(CH_2)_nCO_2H$.

8. A compound as claimed in claim 7 wherein $R^2$ is a phenyl group substituted by one substituent selected from $C_{1-6}$alkyl, halo, $(CH_2)_n$-tetrazolyl, $(CH_2)_n$-imidazolyl and $(CH_2)_nCO_2H$.

9. A compound as claimed in claim 1 selected from:
N-[3(R,S)-5-cyclohexyl-2,3-dihydro-1-methyl-2-oxo-1H-1,4-benzodiazepin-3-yl]N'-[methylphenyl]urea;
N-[3(R,S)-5-cyclohexyl-2,3-dihydro-1-(2-methylpropyl)-2-oxo-1H-1,4-benzodiazepin-3-yl]N'-[3-tetrazol-5-ylphenyl]urea;
N-[3(R,S)-5-cyclopentyl-2,3-dihydro-1-(2-methylpropyl)-2-oxo-1H-1,4-benzodiazepin-3-yl]N'-[3-tetrazol-5-ylphenyl] urea;
N-[3(R,S)-5-cyclohexyl-2,3-dihydro-1-methyl-2-oxo-1H-1,4-benzodiazepin-3-yl]N'-[3-tetrazol-5-ylphenyl] urea;
N-[3(R,S)-5-cyclopentyl-2,3-dihydro-1-ethyl-2-oxo-1H-1,4-benzodiazepin-3-yl]N'-[3-tetrazol-5-ylphenyl] urea;
N-[3(R,S)-5-cyclopentyl-2,3-dihydro-1-propyl-2-oxo-1H-1,4-benzodiazepin-3-yl]N'-[3-tetrazol-5-ylphenyl] urea;
N-[3(R,S)-5-cyclopentyl-2,3-dihydro-1-methyl-2-oxo-1H-1,4-benzodiazepin-3-yl]N'-[3-tetrazol-5-ylphenyl] urea;
N-[3(R,S)-5-cyclohexyl-2,3-dihydro-1-methyl-2-oxo-1H-1,4-benzodiazepin-3-yl]N'-[3-ethynylphenyl]urea;
N-[3(R,S)-5-cyclohexyl-2,3-dihydro-1-methyl-2-oxo-1H-1,4-benzodiazepin-3-yl]N'-[3-carboxyphenyl]urea;
N-[3(R,S)-5-cyclohexyl-2,3-dihydro-1-methyl-2-oxo-1H-1,4-benzodiazepin-3-yl]N'-[3-(isopropylcarbonylamino sulphonyl)phenyl]urea;
N-[3(R,S)-5-cyclohexyl-2,3-dihydro-1-methyl-2-oxo-1H-1,4-benzodiazepin-3-yl]N'-[3-(isopropylsulphonylamino carbonyl)phenyl]urea;
N-[3(R,S)-5-cyclohexyl-2,3-dihydro-1-methyl-2-oxo-1H-1,4-benzodiazepin-3-yl]N'-[3-(phenylcarbonylaminosulphonyl) phenyl]urea;
N-[3(R,S)-5-cyclohexyl-2,3-dihydro-1-methyl-2-oxo-1H-1,4-benzodiazepin-3-yl]N'-[3-(phenylsulphonylaminocarbonyl) phenyl]urea;
N-[3(R,S)-5-cyclohexyl-2,3-dihydro-1-methyl-2-oxo-1H-1,4-benzodiazepin-3-yl]N'-[3-(methylcarbonylaminosulphonyl) phenyl]urea;
N-[3(R,S)-5-cyclohexyl-2,3-dihydro-1-methyl-2-oxo-1H-1,4-benzodiazepin-3-yl]N'-[3-(methylsulphonylaminocarbonyl) phenyl]urea;
N-[3(R,S)-5-cyclohexyl-2,3-dihydro-1-methyl-2-oxo-1H-1,4-benzodiazepin-3-yl]N'-[3-(trifluoromethylcarbonylamino sulphonyl)phenyl]urea;
N-[3(R,S)-5-cyclohexyl-2,3-dihydro-1-methyl-2-oxo-1H-1,4-benzodiazepin-3-yl]N'-[3-(trifluoromethylsulphonylamino carbonyl)phenyl]urea;
N-[3(R,S)-5-cyclohexyl-2,3-dihydro-1-(2-methylpropyl)-2-oxo-1H-1,4-benzodiazepin-3-yl]N'-[3-(methylsulphonylamino carbonyl)phenyl]urea;
N-[3(R,S)-5-cyclopentyl-2,3-dihydro-1-ethyl-2-oxo-1H-1,4-benzodiazepin-3-yl]N'-[3-(methylcarbonylaminosulphonyl) phenyl]urea;
N-[3(R,S)-5-cyclohexyl-2,3-dihydro-1-methyl-2-oxo-1H-1,4-benzodiazepin-3-yl]N'-[3-(1,3,4-thiadiazol-2-ylamino sulphonyl)phenyl]urea;
N-[3(R,S)-5-cyclohexyl-2,3-dihydro-1-methyl-2-oxo-1H-1,4-benzodiazepin-3-yl]N′-[3-(pyrazinylaminosulphonyl) phenyl]urea;
N-[3(R,S)-5-cyclohexyl-2,3-dihydro-1-methyl-2-oxo-1H-1,4-benzodiazepin-3-yl]N′-[3-(ethylsulphonylaminocarbonyl) phenyl]urea;
N-[3(R)-5-cyclohexyl-2,3-dihydro-1-methyl-2-oxo-1H-1,4-benzodiazepin-3-yl]N′-[3-(isopropylsulphonylamino carbonyl)phenyl]urea;
N-[3(S)-5-cyclohexyl-2,3-dihydro-1-methyl-2-oxo-1H-1,4-benzodiazepin-3-yl]N′-[3-(isopropylsulphonylamino carbonyl)phenyl]urea;
N-[3(R,S)-5-cyclohexyl-2,3-dihydro-1-propyl-2-oxo-1H-1,4-benzodiazepin-3-yl]N′-[3-(methylsulphonylaminocarbonyl) phenyl]urea;
N-[3(R,S)-5-cyclohexyl-2,3-dihydro-1-methyl-2-oxo-1H-1,4-benzodiazepin-3-yl]N′-[3-(thiazol-2-ylaminosulphonyl) phenyl]urea;
(-)-N-[3-(R)-5-cyclohexyl-2,3-dihydro-1-methyl-2-oxo-1H-1,4-benzodiazepin-3-yl]N′-[3-(phenylsulphonyl aminocarbonyl)phenyl]urea;
N-[3(R,S)-5-cyclohexyl-2,3-dihydro-1-methyl-2-oxo-1H-1,4-benzodiazepin-3-yl]N′-[3-(2-methyltetrazol-5-yl) phenyl] urea;
N-[3(R,S)-5-cyclopentyl-2,3-dihydro-1-ethyl-2-oxo-1H-1,4-benzodiazepin-3-yl]N′-[3-(methylsulphonylaminocarbonyl) phenyl]urea;
N-[3(R,S)-5-cyclopentyl-2,3-dihydro-1-ethyl-2-oxo-1H-1,4-benzodiazepin-3-yl]N′-[3-(isopropylsulphonylamino carbonyl)phenyl]urea;
N-[3(R,S)-5-cyclohexyl-2,3-dihydro-1-methyl-2-oxo-1H-1,4-benzodiazepin-3-yl]N′-[3-(1,1-dimethylethylsulphonylamino carbonyl)phenyl]urea;
N-[3(R,S)-5-cyclohexyl-2,3-dihydro-1-methyl-2-oxo-1H-1,4-benzodiazepin-3-yl]N′-[3-(1,1-dimethylethylcarbonylamino sulphonyl)phenyl]urea;
N-[3(R)-5-cyclohexyl-2,3-dihydro-1-methyl-2-oxo-1H-1,4-benzodiazepin-3-yl]N′-[3-(2-methylphenylsulphonylamino carbonyl)phenyl]urea;
N-[3(R)-5-cyclohexyl-2,3-dihydro-1-methyl-2-oxo-1H-1,4-benzodiazepin-3-yl]N′-[4-methyl-3-(methylsulphonylamino carbonyl)phenyl]urea;
N-[3(S)-5-cyclohexyl-2,3-dihydro-1-methyl-2-oxo-1H-1,4-benzodiazepin-3-yl]N′-[3-(phenylsulphonylamino carbonyl)phenyl]urea;
N-[3(R,S)-5-cyclopentyl-2,3-dihydro-1-propyl-2-oxo-1H-1,4-benzodiazepin-3-yl]N′-[3-(isopropylsulphonylamino carbonyl)phenyl]urea;
N-[3(R,S)-5-cyclopentyl-2,3-dihydro-1-propyl-2-oxo-1H-1,4-benzodiazepin-3-yl]N′-[3-(1,1-dimethylethylsulphonyl aminocarbonyl)phenyl]urea;
N-[3(R,S)-5-cyclopentyl-2,3-dihydro-1-methyl-2-oxo-1H-1,4-benzodiazepin-3-yl]N′-[3-(isopropylsulphonylamino carbonyl)phenyl]urea;
N-[3(R,S)-5-cyclopentyl-2,3-dihydro-1-methyl-2-oxo-1H-1,4-benzodiazepin-3-yl]N′-[3-(1,1-dimethylethylsulphonyl aminocarbonyl)phenyl]urea;
N-[3(R,S)-5-cyclopentyl-2,3-dihydro-1-methyl-2-oxo-1H-1,4-benzodiazepin-3-yl]N′-[3-(phenylsulphonylamino carbonyl)phenyl]urea;
N-[3(R,S)-5-cyclobutyl-2,3-dihydro-1-methyl-2-oxo-1H-1,4-benzodiazepin-3-yl]N′-[3-methylphenyl]urea;
N-[3(R,S)-5-cyclohexyl-2,3-dihydro-1-methyl-2-oxo-1H-1,4-benzodiazepin-3-yl]N′-[3(R,S)-methylsulphinyl) phenyl] urea;
N-[3(R,S)-5-cyclohexyl-2,3-dihydro-1-methyl-2-oxo-1H-1,4-benzodiazepin-3-yl]N′-[3-(5-hydroxy-4-pyron-2-yl)phenyl] urea;
N-[3(R,S)-5-cyclohexyl-2,3-dihydro-1-methyl-2-oxo-1H-1,4-benzodiazepin-3-yl]N′-[3,4-dihydro-2,2-dioxo-4-oxo-1H-2,3-benzothiazin-6-yl]urea;
N-[3(R,S)-5-cyclohexyl-2,3-dihydro-1-methyl-2-oxo-1H-1,4-benzodiazepin-3-yl]N′-[3-(2-(imidazol-3-yl)ethyl) phenyl]urea;
and salts and prodrugs thereof.

**10.** A pharmaceutical composition comprising a compound as claimed in any preceding claim in association with a pharmaceutically acceptable carrier or excipient.

**11.** The use of a compound as claimed in any of claims 1 to 9 for the manufacture of a medicament for the treatment of disorders involving CCK and/or gastrin.

**12.** A compound as claimed in any of claims 1 to 9 for use in therapy.

**13.** A process for the preparation of a compound as claimed in claim 1 which process comprises:
(A) reacting a compound of formula (II)

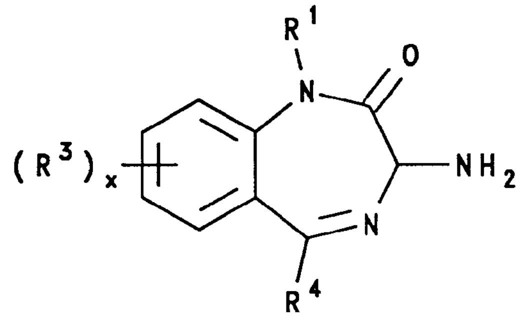

(II)

wherein $R^1$, $R^3$, $R^4$ and x are as defined for formula (I); with an isocyanate of formula $R^2$-N=C=O; or
(B) reacting a compound of formula (IV)

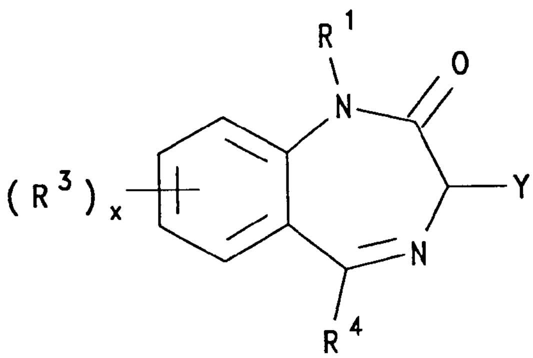

(IV)

wherein $R^1$ $R^3$, $R^4$ and x are as defined for formula (I) and Y represents an activated carbamate, with an amine of formula $R^2NH_2$ in the presence of a base.

**14.** An intermediate of formula (A):

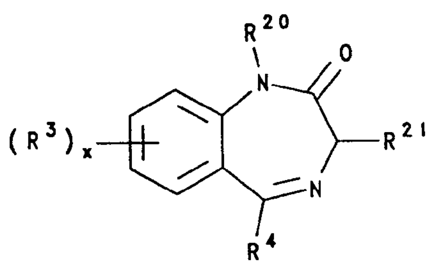

(A)

wherein $R^3$, $R^4$ and x are as defined for formula (I); $R^{20}$ is hydrogen or $R^1$ as defined for formula (I); and $R^{21}$ is $NH_2$, NHZ' (where Z' is a protecting group), or an activated carbamate.

European Patent Office

# EUROPEAN SEARCH REPORT

Application Number

EP 92 30 4264

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | EP-A-0 284 256 (MERCK & CO. INC.)<br>* the whole document, particularly pages 85-88<br>--- | 1-14 | C07D243/14<br>C07D403/12<br>C07D405/12<br>C07D417/12<br>A61K31/55 |
| X | EP-A-0 387 618 (KALI-CHEMIE PHARMA GMBH) | 14 | |
| X | * pages 16, 17, example 4E * | 14 | |
| Y | * the whole document, particularly pages 16, 17, example 4 *<br>----- | 1-14 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 14 AUGUST 1992 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)